# EUROPEAN PATENT SPECIFICATION

(11) **EP 2 542 675 B1**
(45) Date of publication and mention of the grant of the patent: **17.12.2014**
(21) Application number: 11712340.6
(22) Date of filing: 02.03.2011
(51) Int. Cl.: C12N 9/40

(54) **STABILIZED ALPHA-GALACTOSIDASE AND USES THEREOF**
STABILISIERTE ALPHA-GALAKTOSIDASE UND IHRE VERWENDUNG
ALPHA-GALACTOSIDASE STABILISÉE ET SES APPLICATIONS

(30) Priority: 20.01.2011 US 434503 P; 20.01.2011 US 434499 P; 17.11.2010 WO PCT/IL2010/000956; 02.03.2010 US 309487 P
(43) Date of publication of application: 09.01.2013
(73) Proprietor: Protalix Ltd., 2010000 Carmiel (IL)
(72) Inventor: SHULMAN, Avidor, 2017500 Doar-Na Misgav (IL); RUDERFER, Ilya, 21811 Carmiel (IL); BEN-MOSHE, Tehila, 20181 Doar-Na Misgav (IL); SHEKHTER, Talia, 53452 Givataim (IL); AZULAY, Yaniv, 24315 Akko (IL); SHAALTIEL, Yoseph, Timrat 3657600 (IL); KIZHNER, Tali, 2017000 Doar-Na Misgav (IL)
(74) Representative: Vossius & Partner
(86) International application number: PCT/IL2011/000209
(87) International publication number: WO 2011/107990

(56) References cited:
- WO-A2-2006/108052
- WO-A2-2009/024977
- US-A- 5 580 757

### SEQUENCE LISTING

<110> Protalix Ltd. Shulman, Avidor Ruderfer, Ilya Ben-Moshe, Tehila Shekhter, Talia Avulay, Yaniv Shaaltiel, Yoseph Kizhner, Tali
<120> STABILIZED ALPHA-GALACTOSIDASE AND USES THEREOF
<130> 50863
<150> US 61/309,487
   <151> 2010-03-02
<150> US 61/434,499
   <151> 2011-01-20
<150> PCT/IL2010/000956
   <151> 2010-11-17
<150> US 61/434,503
   <151> 2011-01-20
<160> 14
<170> PatentIn version 3.5
<210> 1
   <211> 405
   <212> PRT
   <213> Artificial sequence
<220>
   <223> Plant recombinant human alpha-GAL (prh-alpha-GAL)
<400> 1
   Glu Phe Leu Asp Asn Gly Leu Ala Arg Thr Pro Thr Met Gly Trp Leu
   1               5                   10                  15
   His Trp Glu Arg Phe Met Cys Asn Leu Asp Cys Gln Glu Glu Pro Asp
               20                  25                  30
   Ser Cys Ile Ser Glu Lys Leu Phe Met Glu Met Ala Glu Leu Met Val
           35                  40                  45
   Ser Glu Gly Trp Lys Asp Ala Gly Tyr Glu Tyr Leu Cys Ile Asp Asp
       50                  55                  60
   Cys Trp Met Ala Pro Gln Arg Asp Ser Glu Gly Arg Leu Gln Ala Asp
   65                  70                  75                  80
   Pro Gln Arg Phe Pro His Gly Ile Arg Gln Leu Ala Asn Tyr Val His
                   85                  90                  95
   Ser Lys Gly Leu Lys Leu Gly Ile Tyr Ala Asp Val Gly Asn Lys Thr
               100                 105                 110
   Cys Ala Gly Phe Pro Gly Ser Phe Gly Tyr Tyr Asp Ile Asp Ala Gln
           115                 120                 125
   Thr Phe Ala Asp Trp Gly Val Asp Leu Leu Lys Phe Asp Gly Cys Tyr
   130 135 140
   Cys Asp Ser Leu Glu Asn Leu Ala Asp Gly Tyr Lys His Met Ser Leu
   145 150 155 160
   Ala Leu Asn Arg Thr Gly Arg Ser Ile Val Tyr Ser Cys Glu Trp Pro
   165 170 175
   Leu Tyr Met Trp Pro Phe Gln Lys Pro Asn Tyr Thr Glu Ile Arg Gln
   180 185 190
   Tyr Cys Asn His Trp Arg Asn Phe Ala Asp Ile Asp Asp Ser Trp Lys
   195 200 205
   Ser Ile Lys Ser Ile Leu Asp Trp Thr Ser Phe Asn Gln Glu Arg Ile
   210 215 220
   Val Asp Val Ala Gly Pro Gly Gly Trp Asn Asp Pro Asp Met Leu Val
   225 230 235 240
   Ile Gly Asn Phe Gly Leu Ser Trp Asn Gln Gln Val Thr Gln Met Ala
   245 250 255
   Leu Trp Ala Ile Met Ala Ala Pro Leu Phe Met Ser Asn Asp Leu Arg
   260 265 270
   His Ile Ser Pro Gln Ala Lys Ala Leu Leu Gln Asp Lys Asp Val Ile
   275 280 285
   Ala Ile Asn Gln Asp Pro Leu Gly Lys Gln Gly Tyr Gln Leu Arg Gln
   290 295 300
   Gly Asp Asn Phe Glu Val Trp Glu Arg Pro Leu Ser Gly Leu Ala Trp
   305 310 315 320
   Ala Val Ala Met Ile Asn Arg Gln Glu Ile Gly Gly Pro Arg Ser Tyr
   325 330 335
   Thr Ile Ala Val Ala Ser Leu Gly Lys Gly Val Ala Cys Asn Pro Ala
   340 345 350
   Cys Phe Ile Thr Gln Leu Leu Pro Val Lys Arg Lys Leu Gly Phe Tyr
   355 360 365
   Glu Trp Thr Ser Arg Leu Arg Ser His Ile Asn Pro Thr Gly Thr Val
   370 375 380
   Leu Leu Gln Leu Glu Asn Thr Met Gln Met Ser Leu Lys Asp Leu Ser
   385 390 395 400
   Glu Lys Asp Glu Leu
   405
<210> 2
   <211> 404
   <212> PRT
   <213> Artificial sequence
<220>
   <223> Plant recombinant human alpha-GAL (prh-alpha-GAL)
<400> 2
   Leu Asp Asn Gly Leu Ala Arg Thr Pro Thr Met Gly Trp Leu His Trp
   1 5 10 15
   Glu Arg Phe Met Cys Asn Leu Asp Cys Gln Glu Glu Pro Asp Ser Cys
   20 25 30
   Ile Ser Glu Lys Leu Phe Met Glu Met Ala Glu Leu Met Val Ser Glu
   35 40 45
   Gly Trp Lys Asp Ala Gly Tyr Glu Tyr Leu Cys Ile Asp Asp Cys Trp
   50 55 60
   Met Ala Pro Gln Arg Asp Ser Glu Gly Arg Leu Gln Ala Asp Pro Gln
   65 70 75 80
   Arg Phe Pro His Gly Ile Arg Gln Leu Ala Asn Tyr Val His Ser Lys
   85 90 95
   Gly Leu Lys Leu Gly Ile Tyr Ala Asp Val Gly Asn Lys Thr Cys Ala
   100 105 110
   Gly Phe Pro Gly Ser Phe Gly Tyr Tyr Asp Ile Asp Ala Gln Thr Phe
   115 120 125
   Ala Asp Trp Gly Val Asp Leu Leu Lys Phe Asp Gly Cys Tyr Cys Asp
   130 135 140
   Ser Leu Glu Asn Leu Ala Asp Gly Tyr Lys His Met Ser Leu Ala Leu
   145 150 155 160
   Asn Arg Thr Gly Arg Ser Ile Val Tyr Ser Cys Glu Trp Pro Leu Tyr
   165 170 175
   Met Trp Pro Phe Gln Lys Pro Asn Tyr Thr Glu Ile Arg Gln Tyr Cys
   180 185 190
   Asn His Trp Arg Asn Phe Ala Asp Ile Asp Asp Ser Trp Lys Ser Ile
   195 200 205
   Lys Ser Ile Leu Asp Trp Thr Ser Phe Asn Gln Glu Arg Ile Val Asp
   210 215 220
   Val Ala Gly Pro Gly Gly Trp Asn Asp Pro Asp Met Leu Val Ile Gly
   225 230 235 240
   Asn Phe Gly Leu Ser Trp Asn Gln Gln Val Thr Gln Met Ala Leu Trp
   245 250 255
   Ala Ile Met Ala Ala Pro Leu Phe Met Ser Asn Asp Leu Arg His Ile
   260 265 270
   Ser Pro Gln Ala Lys Ala Leu Leu Gln Asp Lys Asp Val Ile Ala Ile
   275 280 285
   Asn Gln Asp Pro Leu Gly Lys Gln Gly Tyr Gln Leu Arg Gln Gly Asp
   290 295 300
   Asn Phe Glu Val Trp Glu Arg Pro Leu Ser Gly Leu Ala Trp Ala Val
   305 310 315 320
   Ala Met Ile Asn Arg Gln Glu Ile Gly Gly Pro Arg Ser Tyr Thr Ile
   325 330 335
   Ala Val Ala Ser Leu Gly Lys Gly Val Ala Cys Asn Pro Ala Cys Phe
   340 345 350
   Ile Thr Gln Leu Leu Pro Val Lys Arg Lys Leu Gly Phe Tyr Glu Trp
   355 360 365
   Thr Ser Arg Leu Arg Ser His Ile Asn Pro Thr Gly Thr Val Leu Leu
   370 375 380
   Gln Leu Glu Asn Thr Met Gln Met Ser Leu Lys Asp Leu Leu Ser Glu
   385 390 395 400
   Lys Asp Glu Leu
<210> 3
   <211> 405
   <212> PRT
   <213> Artificial sequence
<220>
   <223> Plant recombinant human alpha-GAL (prh-alpha-GAL)
<400> 3
   Gly Leu Asp Asn Gly Leu Ala Arg Thr Pro Thr Met Gly Trp Leu His
   1 5 10 15
   Trp Glu Arg Phe Met Cys Asn Leu Asp Cys Gln Glu Glu Pro Asp Ser
   20 25 30
   Cys Ile Ser Glu Lys Leu Phe Met Glu Met Ala Glu Leu Met Val Ser
   35 40 45
   Glu Gly Trp Lys Asp Ala Gly Tyr Glu Tyr Leu Cys Ile Asp Asp Cys
   50 55 60
   Trp Met Ala Pro Gln Arg Asp Ser Glu Gly Arg Leu Gln Ala Asp Pro
   65 70 75 80
   Gln Arg Phe Pro His Gly Ile Arg Gln Leu Ala Asn Tyr Val His Ser
   85 90 95
   Lys Gly Leu Lys Leu Gly Ile Tyr Ala Asp Val Gly Asn Lys Thr Cys
   100 105 110
   Ala Gly Phe Pro Gly Ser Phe Gly Tyr Tyr Asp Ile Asp Ala Gln Thr
   115 120 125
   Phe Ala Asp Trp Gly Val Asp Leu Leu Lys Phe Asp Gly Cys Tyr Cys
   130 135 140
   Asp Ser Leu Glu Asn Leu Ala Asp Gly Tyr Lys His Met Ser Leu Ala
   145 150 155 160
   Leu Asn Arg Thr Gly Arg Ser Ile Val Tyr Ser Cys Glu Trp Pro Leu
   165 170 175
   Tyr Met Trp Pro Phe Gln Lys Pro Asn Tyr Thr Glu Ile Arg Gln Tyr
   180 185 190
   Cys Asn His Trp Arg Asn Phe Ala Asp Ile Asp Asp Ser Trp Lys Ser
   195 200 205
   Ile Lys Ser Ile Leu Asp Trp Thr Ser Phe Asn Gln Glu Arg Ile Val
   210 215 220
   Asp Val Ala Gly Pro Gly Gly Trp Asn Asp Pro Asp Met Leu Val Ile
   225 230 235 240
   Gly Asn Phe Gly Leu Ser Trp Asn Gln Gln Val Thr Gln Met Ala Leu
   245 250 255
   Trp Ala Ile Met Ala Ala Pro Leu Phe Met Ser Asn Asp Leu Arg His
   260 265 270
   Ile Ser Pro Gln Ala Lys Ala Leu Leu Gln Asp Lys Asp Val Ile Ala
   275 280 285
   Ile Asn Gln Asp Pro Leu Gly Lys Gln Gly Tyr Gln Leu Arg Gln Gly
   290 295 300
   Asp Asn Phe Glu Val Trp Glu Arg Pro Leu Ser Gly Leu Ala Trp Ala
   305 310 315 320
   Val Ala Met Ile Asn Arg Gln Glu Ile Gly Gly Pro Arg Ser Tyr Thr
   325 330 335
   Ile Ala Val Ala Ser Leu Gly Lys Gly Val Ala Cys Asn Pro Ala Cys
   340 345 350
   Phe Ile Thr Gln Leu Leu Pro Val Lys Arg Lys Leu Gly Phe Tyr Glu
   355 360 365
   Trp Thr Ser Arg Leu Arg Ser His Ile Asn Pro Thr Gly Thr Val Leu
   370 375 380
   Leu Gln Leu Glu Asn Thr Met Gln Met Ser Leu Lys Asp Leu Leu Ser
   385 390 395 400
   Glu Lys Asp Glu Leu
   405
<210> 4
   <211> 753
   <212> PRT
   <213> cucumis melo
<400> 4
   Met Thr Val Gly Ala Gly Ile Thr Ile Ser Asp Ala Asn Leu Thr Val
   1               5                   10                  15
   Leu Gly Asn Arg Val Leu Ser Asp Val His Asn Asn Ile Thr Leu Thr
               20                  25                  30
   Ala Ala Pro Gly Gly Gly Val Met Asn Gly Ala Phe Ile Gly Val Gln
           35                  40                  45
   Ser Asp Gln Ile Gly Ser Arg Arg Val Phe Pro Ile Gly Lys Leu Ile
       50                  55                  60
   Gly Leu Arg Phe Leu Cys Ala Phe Arg Phe Lys Leu Trp Trp Met Thr
   65                  70                  75                  80
   Gln Arg Met Gly Cys Ser Gly Gln Glu Val Pro Phe Glu Thr Gln Phe
                   85                  90                  95
   Leu Val Val Glu Thr Arg Asp Gly Ser Asn Ile Ala Gly Asn Gly Glu
               100                 105                 110
   Glu Gly Asp Ala Val Tyr Thr Val Phe Leu Pro Ile Leu Glu Gly Asp
           115                 120                 125
   Phe Arg Ala Val Leu Gln Gly Asn Asp Asn Asn Glu Ile Glu Ile Cys
       130                 135                 140
   Leu Glu Ser Gly Asp Pro Ser Val Asp Gly Phe Glu Gly Ser His Leu
   145                 150                 155                 160
   Val Phe Val Gly Ala Gly Ser Asp Pro Phe Glu Thr Ile Thr Tyr Ala
                   165                 170                 175
   Val Lys Ser Val Glu Lys His Leu Gln Thr Phe Ala His Arg Glu Arg
               180                 185                 190
   Lys Lys Met Pro Asp Ile Leu Asn Trp Phe Gly Trp Cys Thr Trp Asp
           195                 200                 205
   Ala Phe Tyr Thr Asp Val Thr Ser Asp Gly Val Lys Lys Gly Leu Glu
       210                 215                 220
   Ser Phe Glu Asn Gly Gly Ile Pro Pro Lys Phe Val Ile Ile Asp Asp
   225                 230                 235                 240
   Gly Trp Gln Ser Val Ala Lys Asp Ala Thr Ser Ala Asp Cys Lys Ala
                   245                 250                 255
   Asp Asn Thr Ala Asn Phe Ala Asn Arg Leu Thr His Ile Lys Glu Asn
               260                 265                 270
   Tyr Lys Phe Gln Lys Asp Gly Lys Glu Gly Glu Arg Ile Glu Asn Pro
           275                 280                 285
   Ala Leu Gly Leu Gln His Ile Val Ser Tyr Met Lys Glu Lys His Ala
       290                 295                 300
   Thr Lys Tyr Val Tyr Val Trp His Ala Ile Thr Gly Tyr Trp Gly Gly
   305                 310                 315                 320
   Val Ser Ala Gly Val Lys Glu Met Glu Gln Tyr Glu Ser Lys Ile Ala
                   325                 330                 335
   Tyr Pro Val Ala Ser Pro Gly Val Glu Ser Asn Glu Pro Cys Asp Ala
               340                 345                 350
   Leu Asn Ser Ile Thr Lys Thr Gly Leu Gly Leu Val Asn Pro Glu Lys
           355                 360                 365
   Val Phe Asn Phe Tyr Asn Glu Gln His Ser Tyr Leu Ala Ser Ala Gly
       370                 375                 380
   Val Asp Gly Val Lys Val Asp Val Gln Asn Ile Leu Glu Thr Leu Gly
   385                 390                 395                 400
   Ala Gly His Gly Gly Arg Val Lys Leu Ala Arg Lys Tyr His Gln Ala
                   405                 410                 415
   Leu Glu Ala Ser Ile Ser Arg Asn Phe Gln Asp Asn Gly Ile Ile Ser
               420                 425                 430
   Cys Met Ser His Asn Thr Asp Gly Leu Tyr Ser Ser Lys Arg Asn Ala
           435                 440                 445
   Val Ile Arg Ala Ser Asp Asp Phe Trp Pro Arg Asp Pro Ala Ser His
       450                 455                 460
   Thr Ile His Ile Ala Ser Val Ala Tyr Asn Ser Leu Phe Leu Gly Glu
   465                 470                 475                 480
   Phe Met Gln Pro Asp Trp Asp Met Phe His Ser Leu His Pro Met Ala
                   485                 490                 495
   Glu Tyr His Gly Ala Ala Arg Ala Val Gly Gly Cys Ala Ile Tyr Val
               500                 505                 510
   Ser Asp Lys Pro Gly Gln His Asp Phe Asn Leu Leu Lys Lys Leu Val
           515                 520                 525
   Leu Pro Asp Gly Ser Ile Leu Arg Ala Lys Leu Pro Gly Arg Pro Thr
       530                 535                 540
   Lys Asp Cys Leu Phe Thr Asp Pro Ala Arg Asp Gly Lys Ser Leu Leu
   545                 550                 555                 560
   Lys Ile Trp Asn Leu Asn Asp Leu Ser Gly Val Val Gly Val Phe Asn
   565 570 575
   Cys Gln Gly Ala Gly Trp Cys Lys Val Gly Lys Lys Asn Leu Ile His
   580 585 590
   Asp Glu Asn Pro Asp Thr Ile Thr Gly Val Ile Arg Ala Lys Asp Val
   595 600 605
   Ser Tyr Leu Trp Lys Ile Ala Gly Glu Ser Trp Thr Gly Asp Ala Val
   610 615 620
   Ile Phe Ser His Leu Ala Gly Glu Val Val Tyr Leu Pro Gln Asp Ala
   625 630 635 640
   Ser Met Pro Ile Thr Leu Lys Pro Arg Glu Phe Asp Val Phe Thr Val
   645 650 655
   Val Pro Val Lys Glu Leu Val Asn Asp Ile Lys Phe Ala Pro Ile Gly
   660 665 670
   Leu Ile Lys Met Phe Asn Ser Gly Gly Ala Val Lys Glu Met Asn His
   675 680 685
   Gln Pro Gly Ser Ser Asn Val Ser Leu Lys Val Arg Gly Ser Gly Pro
   690 695 700
   Phe Gly Ala Tyr Ser Ser Ser Lys Pro Lys Arg Val Ala Val Asp Ser
   705 710 715 720
   Glu Glu Val Glu Phe Met Tyr Asp Glu Gly Gly Leu Ile Thr Ile Asp
   725 730 735
   Leu Lys Val Pro Glu Lys Glu Leu Tyr Leu Trp Asp Ile Arg Ile Glu
   740 745 750
   Leu
<210> 5
   <211> 772
   <212> PRT
   <213> Cucumis melo
<400> 5
   Met Thr Val Thr Pro Lys Ile Ser Val Asn Asp Gly Asn Leu Val Val
   1 5 10 15
   His Gly Lys Thr Ile Leu Thr Gly Val Pro Asp Asn Ile Val Leu Thr
   20 25 30
   Pro Gly Ser Gly Leu Gly Leu Val Ala Gly Ala Phe Ile Gly Ala Thr
   35 40 45
   Ala Ser Asn Ser Lys Ser Leu His Val Phe Pro Val Gly Val Leu Glu
   50 55 60
   Gly Thr Arg Phe Leu Cys Cys Phe Arg Phe Lys Leu Trp Trp Met Thr
   65 70 75 80
   Gln Arg Met Gly Thr Ser Gly Arg Asp Ile Pro Phe Glu Thr Gln Phe
   85 90 95
   Leu Leu Met Glu Ser Lys Gly Asn Asp Gly Glu Asp Pro Asp Asn Ser
   100 105 110
   Ser Thr Ile Tyr Thr Val Phe Leu Pro Leu Leu Glu Gly Gln Phe Arg
   115 120 125
   Ala Ala Leu Gln Gly Asn Glu Lys Asn Glu Met Glu Ile Cys Leu Glu
   130 135 140
   Ser Gly Asp Asn Thr Val Glu Thr Asn Gln Gly Leu Ser Leu Val Tyr
   145 150 155 160
   Met His Ala Gly Thr Asn Pro Phe Glu Val Ile Thr Gln Ala Val Lys
   165 170 175
   Ala Val Glu Lys His Thr Gln Thr Phe Leu His Arg Glu Lys Lys Lys
   180 185 190
   Leu Pro Ser Phe Leu Asp Trp Phe Gly Trp Cys Thr Trp Asp Ala Phe
   195 200 205
   Tyr Thr Asp Val Thr Ala Glu Gly Val Val Glu Gly Leu Lys Ser Leu
   210 215 220
   Ser Glu Gly Gly Ala Pro Pro Lys Phe Leu Ile Ile Asp Asp Gly Trp
   225 230 235 240
   Gln Gln Ile Glu Ala Lys Pro Lys Asp Ala Asp Cys Val Val Gln Glu
   245 250 255
   Gly Ala Gln Phe Ala Ser Arg Leu Ser Gly Ile Lys Glu Asn His Lys
   260 265 270
   Phe Gln Lys Asn Gly Asn Asn Tyr Asp Gln Val Pro Gly Leu Lys Val
   275 280 285
   Val Val Asp Asp Ala Lys Lys Gln His Lys Val Lys Phe Val Tyr Ala
   290 295 300
   Trp His Ala Leu Ala Gly Tyr Trp Gly Gly Val Lys Pro Ala Ser Pro
   305 310 315 320
   Gly Met Glu His Tyr Asp Ser Ala Leu Ala Tyr Pro Val Gln Ser Pro
   325 330 335
   Gly Met Leu Gly Asn Gln Pro Asp Ile Val Val Asp Ser Leu Ala Val
   340 345 350
   His Gly Ile Gly Leu Val His Pro Lys Lys Val Phe Asn Phe Tyr Asn
           355                 360                 365
   Glu Leu His Ser Tyr Leu Ala Ser Cys Gly Ile Asp Gly Val Lys Val
       370                 375                 380
   Asp Val Gln Asn Ile Ile Glu Thr Leu Gly Ala Gly His Gly Gly Arg
   385                 390                 395                 400
   Val Thr Leu Thr Arg Ser Tyr His Gln Ala Leu Glu Ala Ser Ile Ala
                   405                 410                 415
   Arg Asn Phe Ser Asp Asn Gly Cys Ile Ala Cys Met Cys His Asn Thr
               420                 425                 430
   Asp Ser Leu Tyr Ser Ala Lys Gln Thr Ala Val Val Arg Ala Ser Asp
           435                 440                 445
   Asp Tyr Tyr Pro Arg Asp Pro Ala Ser His Thr Ile His Ile Ser Ser
       450                 455                 460
   Val Ala Tyr Asn Ser Leu Phe Leu Gly Glu Phe Met Gln Pro Asp Trp
   465                 470                 475                 480
   Asp Met Phe His Ser Leu His Pro Thr Ala Glu Tyr His Gly Ala Ala
                   485                 490                 495
   Arg Ala Ile Gly Gly Cys Ala Ile Tyr Val Ser Asp Lys Pro Gly Asn
               500                 505                 510
   His Asn Phe Asp Leu Leu Lys Lys Leu Val Leu Pro Asp Gly Ser Val
           515                 520                 525
   Leu Arg Ala Gln Leu Pro Gly Arg Pro Thr Arg Asp Ser Leu Phe Asn
       530                 535                 540
   Asp Pro Ala Arg Asp Gly Thr Ser Leu Leu Lys Ile Trp Asn Met Asn
   545                 550                 555                 560
   Lys Cys Ser Gly Val Val Gly Val Phe Asn Cys Gln Gly Ala Gly Trp
                   565                 570                 575
   Cys Arg Ile Thr Lys Lys Thr Arg Ile His Asp Glu Ser Pro Gly Thr
               580                 585                 590
   Leu Thr Thr Ser Val Arg Ala Ala Asp Val Asp Ala Ile Ser Gln Val
           595                 600                 605
   Ala Gly Ala Asp Trp Lys Gly Asp Thr Ile Val Tyr Ala Tyr Arg Ser
       610                 615                 620
   Gly Asp Leu Ile Arg Leu Pro Lys Gly Ala Ser Val Pro Val Thr Leu
   625                 630                 635                 640
   Lys Val Leu Glu Tyr Asp Leu Leu His Ile Ser Pro Leu Lys Asp Ile
   645 650 655
   Ala Ser Asn Ile Ser Phe Ala Pro Ile Gly Leu Leu Asp Met Phe Asn
   660 665 670
   Thr Gly Gly Ala Val Glu Gln Val Asn Val Gln Val Val Glu Pro Ile
   675 680 685
   Pro Glu Phe Asp Gly Glu Val Ala Ser Glu Leu Thr Cys Ser Leu Pro
   690 695 700
   Asn Asp Arg Pro Pro Thr Ala Thr Ile Thr Met Lys Ala Arg Gly Cys
   705 710 715 720
   Arg Arg Phe Gly Leu Tyr Ser Ser Gln Arg Pro Leu Lys Cys Ser Val
   725 730 735
   Asp Lys Val Asp Val Asp Phe Val Tyr Asp Glu Val Thr Gly Leu Val
   740 745 750
   Thr Phe Glu Ile Pro Ile Pro Thr Glu Glu Met Tyr Arg Trp Asn Ile
   755 760 765
   Glu Ile Gln Val
   770
<210> 6
   <211> 767
   <212> PRT
   <213> Tetragonia tetragonloides
<400> 6
   Met Thr Ile Thr Pro Ser Ile Ser Val Ser Asn Gly Asn Leu Val Val
   1 5 10 15
   His Gly Lys Thr Ile Leu Thr Gly Val Pro Asp Asn Ile Ile Leu Thr
   20 25 30
   Pro Gly Ser Gly Ala Gly Leu Ala Ala Gly Ala Phe Ile Gly Ala Thr
   35 40 45
   Ala Asp Asp Ser Lys Cys Leu His Val Phe Pro Met Gly Thr Leu Glu
   50 55 60
   Gly Leu Arg Phe Met Cys Cys Leu Arg Phe Lys Leu Trp Trp Met Thr
   65 70 75 80
   Gln Arg Met Gly Lys Cys Gly Lys Asp Ile Pro Leu Glu Thr Gln Phe
   85 90 95
   Met Ile Val Glu Ser Lys Asp Asp Thr Val Glu Gly Glu Pro Asp Asp
   100 105 110
   Ser Pro Thr Ile Tyr Thr Val Phe Leu Pro Leu Leu Glu Gly Gln Phe
   115 120 125
   Arg Ala Val Leu Gln Gly Thr Glu Lys Asn Glu Ile Glu Ile Cys Leu
       130                 135                 140
   Glu Ser Gly Asp Thr Thr Val Gln Thr Ser Gln Gly Leu His Leu Val
   145                 150                 155                 160
   Tyr Met His Ala Gly Thr Asn Pro Tyr Glu Val Ile Asn Gln Ala Val
                   165                 170                 175
   Lys Ala Val Glu Lys His Met Gln Thr Phe Arg His Arg Glu Lys Lys
               180                 185                 190
   Arg Leu Pro Ser Phe Val Asp Trp Phe Gly Trp Cys Thr Trp Asp Ala
           195                 200                 205
   Phe Tyr Thr Asp Val Thr Ala Glu Gly Val Asp Glu Gly Leu Arg Ser
       210                 215                 220
   Leu Ser Glu Gly Gly Thr Pro Pro Arg Phe Leu Ile Ile Asp Asp Gly
   225                 230                 235                 240
   Trp Gln Gln Ile Gly Asn Glu Ile Val Lys Asp Glu Asn Cys Met Val
                   245                 250                 255
   Gln Glu Gly Ala Gln Phe Ala Asn Arg Leu Thr Gly Ile Lys Glu Asn
               260                 265                 270
   Ala Lys Phe Gln Lys Lys Lys Asn Gly Glu Asp Lys Asp Gln Val Pro
           275                 280                 285
   Gly Leu Lys His Val Val Glu Glu Ala Lys Gln Arg His Asn Val Lys
       290                 295                 300
   Ser Val Tyr Val Trp His Ala Leu Ala Gly Tyr Trp Gly Gly Val Lys
   305                 310                 315                 320
   Pro Ala Ala Ala Gly Met Glu His Tyr Asp Thr Ala Leu Ala Tyr Pro
                   325                 330                 335
   Val Gln Ser Pro Gly Val Leu Gly Asn Gln Pro Asp Val Val Met Asp
               340                 345                 350
   Ser Leu Ser Val His Gly Leu Gly Leu Val His Pro Lys Lys Val Phe
           355                 360                 365
   Asn Phe Tyr Asn Glu Leu His Ala Tyr Leu Ala Ala Cys Gly Val Asp
       370                 375                 380
   Gly Val Lys Val Asp Val Gln Asn Ile Ile Glu Thr Leu Gly Ala Gly
   385                 390                 395                 400
   His Gly Gly Arg Val Ser Leu Thr Arg Ala Tyr His Gln Ala Leu Glu
                   405                 410                 415
   Ala Ser Ile Ala Arg Asn Phe Pro Asp Asn Gly Cys Ile Ser Cys Met
               420                 425                 430
   Cys His Asn Thr Asp Gly Ile Tyr Ser Thr Lys Gln Thr Ala Val Val
   435 440 445
   Arg Ala Ser Asp Asp Phe Tyr Pro Arg Asp Pro Ala Ser His Thr Ile
   450 455 460
   His Ile Ser Ser Val Ala Tyr Asn Ser Leu Phe Leu Gly Glu Phe Met
   465 470 475 480
   Gln Pro Asp Trp Asp Met Phe His Ser Leu His Pro Ala Ala Asp Tyr
   485 490 495
   His Ala Ala Ala Arg Ala Val Gly Gly Cys Pro Ile Tyr Val Ser Asp
   500 505 510
   Lys Pro Gly Phe His Asn Phe Glu Leu Leu Lys Lys Leu Val Leu Pro
   515 520 525
   Asp Gly Ser Val Leu Arg Ala Arg Leu Pro Gly Arg Pro Thr Arg Asp
   530 535 540
   Cys Leu Phe Asn Asp Pro Ala Arg Asp Gly Thr Ser Leu Leu Lys Ile
   545 550 555 560
   Trp Asn Lys Asn Asn Cys Ser Gly Val Val Gly Val Phe Asn Cys Gln
   565 570 575
   Gly Ala Gly Trp Cys Lys Ile Glu Lys Lys Ile Arg Ile His Asp Thr
   580 585 590
   Ser Pro Gly Thr Leu Thr Gly Ser Val Arg Ala Thr Asp Val Asp Ser
   595 600 605
   Ile Ala Glu Val Ala Gly Gln Gly Trp Asn Gly Asp Val Val Val Tyr
   610 615 620
   Leu Tyr Arg Ala Gly Glu Leu Val Cys Leu Pro Lys Gly Ala Ser Leu
   625 630 635 640
   Pro Val Thr Leu Lys Val Arg Glu Tyr Glu Leu Phe His Phe Cys Pro
   645 650 655
   Ile Lys Glu Ile Thr Ser Asn Ile Ser Phe Ala Pro Ile Gly Leu Leu
   660 665 670
   Asp Met Phe Asn Gly Ser Gly Ala Val Asp Gln Phe Asp Val Gln Leu
   675 680 685
   Thr Ser Glu Asn Arg Thr Glu Leu Ser Asp Gly Glu Lys Arg Ser Pro
   690 695 700
   Ser Ala Ser Ile Gln Leu Lys Val Arg Gly Cys Gly Arg Phe Gly Ala
   705 710 715 720
   Tyr Ser Ser Gln Cys Pro Leu Lys Cys Thr Val Gly Gly Ala Asp Ser
   725 730 735
   Gly Phe Asn Tyr Asp Glu Glu Thr Cys Leu Leu Thr Leu Thr Leu Pro
   740 745 750
   Val Pro Gln Glu Glu Met Tyr Arg Trp Pro Val Glu Ile Gln Val
   755 760 765
<210> 7
   <211> 772
   <212> PRT
   <213> Cucumis sativus
<400> 7
   Met Thr Val Thr Pro Lys Ile Thr Val Asn Asp Gly Asn Leu Val Val
   1 5 10 15
   His Gly Lys Thr Ile Leu Thr Gly Val Pro Asp Asn Ile Val Leu Thr
   20 25 30
   Pro Gly Ser Gly Leu Gly Leu Val Ala Gly Ala Phe Ile Gly Ala Thr
   35 40 45
   Ala Ser Asn Ser Lys Ser Leu His Val Phe Pro Val Gly Val Leu Glu
   50 55 60
   Gly Thr Arg Phe Leu Cys Cys Phe Arg Phe Lys Leu Trp Trp Met Thr
   65 70 75 80
   Gln Arg Met Gly Thr Ser Gly Arg Asp Ile Pro Phe Glu Thr Gln Phe
   85 90 95
   Leu Leu Met Glu Ser Gln Gly Asn Asp Gly Glu Asp Pro Asp Asn Ser
   100 105 110
   Ser Thr Ile Tyr Thr Val Phe Leu Pro Leu Leu Glu Gly Gln Phe Arg
   115 120 125
   Ala Ala Leu Gln Gly Asn Glu Lys Asn Glu Met Glu Ile Cys Leu Glu
   130 135 140
   Ser Gly Asp Asn Thr Val Glu Thr Asn Gln Gly Leu Ser Leu Val Tyr
   145 150 155 160
   Met His Ala Gly Thr Asn Pro Phe Glu Val Ile Thr Gln Ala Val Lys
   165 170 175
   Ala Val Glu Lys His Thr Gln Thr Phe Leu His Arg Glu Lys Lys Lys
   180 185 190
   Leu Pro Ser Phe Leu Asp Trp Phe Gly Trp Cys Thr Trp Asp Ala Phe
   195 200 205
   Tyr Thr Asp Val Thr Ala Glu Gly Val Val Glu Gly Leu Gln Ser Leu
   210 215 220
   Ser Asp Gly Gly Ala Pro Pro Lys Phe Leu Ile Ile Asp Asp Gly Trp
   225 230 235 240
   Gln Gln Ile Glu Ala Lys Pro Lys Asp Ala Asp Cys Val Val Gln Glu
   245 250 255
   Gly Ala Gln Phe Ala Ser Arg Leu Ser Gly Ile Lys Glu Asn His Lys
   260 265 270
   Phe Gln Lys Asn Gly Asn Asn Tyr Asp Gln Val Pro Gly Leu Lys Val
   275 280 285
   Val Val Asp Asp Ala Lys Lys Gln His Lys Val Lys Phe Val Tyr Ala
   290 295 300
   Trp His Ala Leu Ala Gly Tyr Trp Gly Gly Val Lys Pro Ala Ser Pro
   305 310 315 320
   Gly Met Glu His Tyr Asp Ser Ala Leu Ala Tyr Pro Val Gln Ser Pro
   325 330 335
   Gly Met Leu Gly Asn Gln Pro Asp Ile Val Val Asp Ser Leu Ala Val
   340 345 350
   His Gly Ile Gly Leu Val His Pro Lys Lys Val Phe Asn Phe Tyr Asn
   355 360 365
   Glu Leu His Ser Tyr Leu Ala Ser Cys Gly Ile Asp Gly Val Lys Val
   370 375 380
   Asp Val Gln Asn Ile Ile Glu Thr Leu Gly Ala Gly His Gly Gly Arg
   385 390 395 400
   Val Thr Leu Thr Arg Ser Tyr His Gln Ala Leu Glu Ala Ser Ile Ala
   405 410 415
   Arg Asn Phe Ser Asp Asn Gly Cys Ile Ala Cys Met Cys His Asn Thr
   420 425 430
   Asp Ser Leu Tyr Ser Ala Lys Gln Thr Ala Val Val Arg Ala Ser Asp
   435 440 445
   Asp Tyr Tyr Pro Arg Asp Pro Ala Ser His Thr Ile His Ile Ser Ser
   450 455 460
   Val Ala Tyr Asn Ser Leu Phe Leu Gly Glu Phe Met Gln Pro Asp Trp
   465 470 475 480
   Asp Met Phe His Ser Leu His Pro Thr Ala Glu Tyr His Gly Ala Ala
   485 490 495
   Arg Ala Ile Gly Gly Cys Ala Ile Tyr Val Ser Asp Lys Pro Gly Asn
   500 505 510
   His Asn Phe Asp Leu Leu Lys Lys Leu Val Leu Pro Asp Gly Ser Val
   515 520 525
   Leu Arg Ala Gln Leu Pro Gly Arg Pro Thr Arg Asp Ser Leu Phe Asn
   530 535 540
   Asp Pro Ala Arg Asp Gly Thr Ser Leu Leu Lys Ile Trp Asn Met Asn
   545 550 555 560
   Lys Cys Ser Gly Val Val Gly Val Phe Asn Cys Gln Gly Ala Gly Trp
   565 570 575
   Cys Arg Ile Thr Lys Lys Thr Arg Ile His Asp Glu Ser Pro Gly Thr
   580 585 590
   Leu Thr Thr Ser Val Arg Ala Ala Asp Val Asp Ala Ile Ser Gln Val
   595 600 605
   Ala Gly Ala Asp Trp Lys Gly Asp Thr Ile Val Tyr Ala Tyr Arg Ser
   610 615 620
   Gly Asp Leu Thr Arg Leu Pro Lys Gly Ala Ser Val Pro Val Thr Leu
   625 630 635 640
   Lys Val Leu Glu Tyr Asp Leu Phe His Ile Ser Pro Leu Lys Asp Ile
   645 650 655
   Thr Ser Asn Ile Ser Phe Ala Pro Ile Gly Leu Val Asp Met Phe Asn
   660 665 670
   Ile Gly Gly Ala Val Glu Gln Val Asp Ile Gln Val Val Glu Pro Ile
   675 680 685
   Pro Glu Phe Asp Gly Glu Val Ala Ser Glu Leu Thr Cys Ser Leu Pro
   690 695 700
   Asp Asp Arg Pro Pro Thr Ala Thr Ile Thr Met Lys Ala Arg Gly Cys
   705 710 715 720
   Gly Arg Phe Gly Leu Tyr Ser Ser Gln Arg Pro Leu Lys Cys Ser Val
   725 730 735
   Asp Lys Val Gly Thr Asp Phe Val Tyr Asp Asp Val Thr Gly Leu Val
   740 745 750
   Thr Phe Glu Ile Pro Ile Pro Thr Glu Glu Met Tyr Arg Trp Asn Ile
   755 760 765
   Glu Ile Glu Val
   770
<210> 8
   <211> 211
   <212> PRT
<211> zea mays
<400> 8
   Pro Phe Glu Val Ile Thr Ser Ser Val Lys Ala Val Glu Arg His Leu
   1 5 10 15
   Gln Thr Phe Ser His Arg Glu Lys Lys Lys Met Pro Asp Ile Leu Asn
   20 25 30
   Trp Phe Gly Trp Cys Thr Trp Asp Ala Phe Tyr Thr Asn Val Thr Ala
   35 40 45
   Gln Gly Val Lys Gln Gly Leu Gln Ser Leu Glu Lys Gly Gly Val Ser
   50 55 60
   Pro Arg Phe Val Ile Ile Asp Asp Gly Trp Gln Ser Val Ala Met Asp
   65 70 75 80
   Pro Val Gly Ile Ala Cys Leu Ser Asp Asn Ser Ala Asn Phe Ala Asn
   85 90 95
   Arg Leu Thr His Ile Arg Glu Asn His Lys Phe Gln Lys Asn Gly Arg
   100 105 110
   Glu Gly His Arg Glu Asp Asp Pro Ala Lys Gly Leu Ala His Val Val
   115 120 125
   Asn Glu Ile Lys Gly Lys His Gln Leu Lys Tyr Val Tyr Val Trp His
   130 135 140
   Ala Ile Thr Gly Tyr Trp Gly Gly Val Arg Pro Gly Ala Ala Gly Met
   145 150 155 160
   Glu His Tyr Gly Ser Lys Met Gln Arg Pro Val Pro Ser Pro Gly Val
   165 170 175
   Pro Lys Asn Glu Arg Cys Glu Ala Leu Asp Ser Met Thr Ala Asn Gly
   180 185 190
   Leu Gly Leu Val Asn Leu Asp Arg Ala Phe Ser Phe Tyr Asp Glu Leu
   195 200 205
   His Ser Tyr
   210
<210> 9
   <211> 747
   <212> PRT
   <213> Zea mays
<400> 9
   Met Thr Val Ala Ser Ser Val Arg Leu Ala Gly Gly Asn Leu Thr Val
   1 5 10 15
   Cys Gly Arg Thr Val Leu Ser Gly Val Pro Asp Ala Val Val Ala Thr
   20 25 30
   Ser Ala Ala Thr Glu Gly Ala Val Asp Gly Ile Phe Leu Gly Ala Asp
   35 40 45
   Phe Ala Glu Pro Ala Ala Arg His Val Val Ser Leu Gly Asp Leu Arg
   50 55 60
   Asp Val Arg Phe Met Ala Cys Phe Arg Phe Lys Leu Trp Trp Met Ala
   65 70 75 80
   Gln Arg Met Gly Glu Lys Gly Ser Asp Val Pro Arg Glu Thr Gln Phe
   85 90 95
   Leu Leu Val Glu Ser Arg Gly Val Gly Asp Glu Asp Ala Ala Tyr Val
   100 105 110
   Val Phe Leu Pro Leu Val Glu Gly Ala Phe Arg Ala Ser Ile Gln Gly
   115 120 125
   Gly Ala Gly Asp Ala Leu Glu Leu Cys Val Glu Ser Gly Asp Asp Asp
   130 135 140
   Thr Arg Ala Ala Ser Phe Glu Arg Ser Leu Phe Val Gly Ala Ala Glu
   145 150 155 160
   Ser Asp Pro Phe Ala Ala Ile Ser Gly Ala Val Ala Ala Ala Lys Ser
   165 170 175
   Ala Leu Arg Thr Phe Arg Val Arg Ala Glu Lys Lys Leu Pro Gly Ile
   180 185 190
   Val Asp Tyr Phe Gly Trp Cys Thr Trp Asp Ala Phe Tyr Gln Asp Val
   195 200 205
   Thr Gln Glu Gly Val Glu Ala Gly Leu Arg Ser Leu Ile Ala Gly Gly
   210 215 220
   Ala Pro Pro Lys Phe Val Ile Ile Asp Asp Gly Trp Gln Ser Val Ala
   225 230 235 240
   Thr Asp Thr Asn Glu Ser Ala Gly Glu Asp Lys Pro Pro Leu Leu Ser
   245 250 255
   Arg Leu Thr Gly Ile Lys Glu Asn Ser Lys Phe Gln Asn Ala Asp Asp
   260 265 270
   Pro Ala Ala Gly Ile Lys Thr Val Val Arg Leu Ala Lys Glu Glu Tyr
   275 280 285
   Arg Leu Lys Tyr Val Tyr Val Trp His Ala Ile Thr Gly Tyr Trp Gly
   290 295 300
   Gly Val Arg Pro Gly Glu Glu His Tyr Arg Ser Ser Met Gln Phe Pro
   305 310 315 320
   Lys Val Ser Pro Gly Val Met Glu Asn Glu Pro Gly Met Lys Thr Asp
   325 330 335
   Val Leu Thr Val Gln Gly Leu Gly Leu Val His Pro Arg Ala Val Tyr
   340 345 350
   Arg Phe Tyr Asp Glu Leu His Ala Tyr Leu Ala Ala Ala Gly Val Asp
   355 360 365
   Gly Val Lys Val Asp Val Gln Cys Ile Leu Glu Thr Leu Gly Ala Gly
   370 375 380
   His Gly Gly Arg Val Gln Leu Thr Arg Gln Tyr His Gln Ala Leu Asp
   385 390 395 400
   Ala Ser Val Ala Lys Asn Phe Pro Glu Asn Gly Ile Ile Ala Cys Met
   405 410 415
   Ser His Asn Thr Asp Ala Leu Tyr Cys Ser Lys Gln Thr Ala Val Val
   420 425 430
   Arg Ala Ser Asp Asp Phe Cys Pro Arg Asp Pro Ala Ser His Thr Ile
   435 440 445
   His Ile Ala Ser Val Ala Tyr Asn Ser Val Phe Leu Gly Glu Phe Met
   450 455 460
   Leu Pro Asp Trp Asp Met Phe His Ser Leu His Gln Ala Gly Asp Tyr
   465 470 475 480
   His Gly Ser Ala Arg Ala Ile Ser Gly Gly Pro Val Tyr Val Ser Asp
   485 490 495
   Ala Pro Gly Lys His Asn Phe Glu Leu Leu Lys Lys Ile Val Leu Pro
   500 505 510
   Asp Gly Ser Ile Leu Arg Ala Arg Leu Pro Gly Arg Pro Thr Lys Asp
   515 520 525
   Cys Leu Phe Thr Asp Pro Ala Arg Asp Gly Val Ser Leu Leu Lys Ile
   530 535 540
   Trp Asn Met Asn Lys Phe Thr Gly Val Leu Gly Val Tyr Asn Cys Gln
   545 550 555 560
   Gly Ala Ala Trp Asn Ser Val Glu Lys Lys Asn Thr Phe His Gln Thr
   565 570 575
   Gly Thr Glu Ala Leu Thr Cys Gly Val Lys Gly Gly Asp Val His Leu
   580 585 590
   Ile Ser Glu Ala Ala Thr Asp Thr Glu Trp Asp Gly Asp Cys Ala Met
   595 600 605
   Tyr Arg His Ala Asp Gly Asp Leu Val Val Leu Pro His Asp Ala Ala
   610 615 620
   Leu Pro Val Ser Leu Lys Val Leu Glu His Asp Ile Leu Thr Val Ser
   625 630 635 640
   Pro Ile Lys Glu Leu Ala Pro Gly Phe Arg Phe Ala Pro Ile Gly Leu
   645 650 655
   Val Asp Met Phe Asn Ser Gly Gly Ala Val Glu Gly Leu Thr Tyr His
   660 665 670
   Leu Leu Gly Gly Asp Gly Ser Thr Leu Gly Ser Glu Ala Val Ala Leu
   675 680 685
   Ala Cys Met Glu Val Lys Gly Cys Gly Arg Phe Gly Ala Tyr Ser Ser
   690 695 700
   Val Arg Pro Arg Lys Ser Thr Leu Gly Ser Ala Gln Ile Glu Leu Lys
   705 710 715 720
   Tyr Asp Ser Ser Ser Gly Leu Leu Ile Leu Gln Leu Asp Ala Met Pro
   725 730 735
   Lys Glu Arg Val His Lys Ile Val Ile Glu Leu
   740 745
<210> 10
   <211> 753
   <212> PRT
   <213> Oryza sativa
<400> 10
   Met Thr Val Gly Ala Gly Val Ala Val Gln Asp Gly Gly Leu Val Ala
   1 5 10 15
   Leu Gly Ala Thr Val Leu Thr Glu Val Arg Asp Asn Val Leu Leu Thr
   20 25 30
   Pro Ala Ala Gly Ala Gly Met Thr Ser Gly Thr Phe Val Gly Val Arg
   35 40 45
   Ser Ala Thr Ala Gly Ser Arg Ser Val Phe Pro Val Gly Lys Leu Arg
   50 55 60
   Gly Leu Arg Phe Ile Cys Thr Phe Arg Phe Lys Met Trp Trp Met Thr
   65 70 75 80
   Gln Arg Met Gly Thr Ser Gly Arg Asp Ile Pro Phe Glu Thr Gln Phe
   85 90 95
   Leu Leu Val Glu Ala Ala Asp Ala Asp Gly Ser His Leu Ala Gly Asp
   100 105 110
   Gly Ala Ala Ala Val Tyr Thr Val Phe Leu Pro Ile Leu Glu Gly Pro
   115 120 125
   Phe Arg Ala Val Leu Gln Gly Asn Ser Asp Asp Glu Leu Glu Ile Cys
   130 135 140
   Leu Glu Ser Gly Asp Pro Ala Val Glu Ser Phe Glu Gly Thr His Leu
   145 150 155 160
   Val Phe Val Gly Ala Gly Ser Asp Pro Phe Glu Val Ile Thr Asn Ser
   165 170 175
   Val Lys Ala Val Glu Arg His Leu Gln Thr Phe Thr His Arg Glu Lys
   180 185 190
   Lys Lys Met Pro Asp Met Leu Asn Trp Phe Gly Trp Cys Thr Trp Asp
   195 200 205
   Ala Phe Tyr Thr Asp Val Thr Ser Glu Gly Val Met Glu Gly Leu Gln
   210 215 220
   Ser Leu Gly Lys Gly Gly Thr Gly Pro Lys Phe Val Ile Ile Asp Asp
   225 230 235 240
   Gly Trp Gln Ser Val Ser Met Asp Pro Ala Gly Ile Ala Ser Leu Ala
   245 250 255
   Asp Asn Ser Ala Asn Phe Ala Asn Arg Leu Thr His Ile Lys Glu Asn
   260 265 270
   His Lys Phe Gln Leu Asn Gly Arg Lys Gly His Arg Glu Glu Asn Pro
   275 280 285
   Ala Asn Gly Leu Ala His Ile Val Asn Glu Ile Lys Gly Lys His Gln
   290 295 300
   Leu Lys Tyr Val Tyr Val Trp His Ala Ile Thr Gly Tyr Trp Gly Gly
   305 310 315 320
   Val Arg Pro Gly Ala Asp Gly Met Glu His Tyr Glu Ser Lys Met Gln
   325 330 335
   Tyr Pro Val Ser Ser Pro Gly Val Gln Lys Asn Glu Pro Cys Asp Ala
   340 345 350
   Leu Asn Ser Ile Thr Thr Asn Gly Leu Gly Leu Val Asn Pro Asp Arg
   355 360 365
   Val Phe Ser Phe Tyr Asn Glu Leu His Ala Tyr Leu Ala Ser Ala Gly
   370 375 380
   Ile Asp Gly Val Lys Val Asp Val Gln Asn Ile Leu Glu Thr Leu Gly
   385 390 395 400
   Ala Gly His Gly Gly Arg Val Leu Leu Ala Arg Lys Tyr His Gln Ala
   405 410 415
   Leu Glu Ala Ser Ile Ala Arg Asn Phe Arg Asp Asn Gly Ile Ile Cys
   420 425 430
   Cys Met Ser His Asn Thr Asp Asn Leu Tyr Ser Ser Lys Arg Ser Ala
   435 440 445
   Val Val Arg Ala Ser Asp Asp Phe Trp Pro Arg Asp Pro Ala Ser His
   450 455 460
   Thr Ile His Ile Ala Ser Val Ala Tyr Asn Thr Val Phe Leu Gly Glu
   465 470 475 480
   Phe Met Gln Pro Asp Trp Asp Met Phe His Ser Val His Pro Met Ala
   485 490 495
   Glu Tyr His Ala Ala Ala Arg Ala Val Gly Gly Cys Ala Ile Tyr Val
   500 505 510
   Ser Asp Lys Pro Gly Asn His Asp Phe Asn Leu Leu Lys Lys Leu Val
   515 520 525
   Leu Pro Asp Gly Ser Ile Leu Arg Ala Lys Leu Pro Gly Arg Pro Thr
   530 535 540
   Arg Asp Cys Leu Phe Ser Asp Pro Ala Arg Asp Gly Lys Ser Ile Leu
   545 550 555 560
   Lys Ile Trp Asn Leu Asn Glu His Ser Gly Val Ile Gly Ala Phe Asn
   565 570 575
   Cys Gln Gly Ala Gly Trp Cys Arg Val Gly Lys Lys Asn Leu Val His
   580 585 590
   Asp Glu Gln Pro Ala Thr Val Thr Gly Val Ile Arg Ala Gln Asp Val
   595 600 605
   His His Leu Ala Thr Val Ala Ala Asp Gly Trp Asn Gly Asp Val Ile
   610 615 620
   Val Tyr Ser His Ile Gly Gly Glu Val Thr Cys Leu Pro Lys Asn Ala
   625 630 635 640
   Ser Leu Pro Val Thr Leu Lys Thr Arg Glu Tyr Glu Val Phe Thr Val
   645 650 655
   Val Pro Leu Lys Lys Leu Asp Asn Gly Val Ser Phe Ala Ala Val Gly
   660 665 670
   Leu Ile Gly Met Phe Asn Ser Gly Gly Ala Val Thr Ala Val Arg Tyr
   675 680 685
   Val Glu Asp Ala Gly Val Glu Val Arg Val Arg Gly Ser Gly Thr Val
   690 695 700
   Gly Ala Tyr Ser Ser Ala Lys Pro Ala Arg Val Val Val Asp Ser Glu
   705 710 715 720
   Ala Ala Glu Phe Ser Tyr Asp Asp Gly Cys Gly Leu Val Thr Phe Glu
   725 730 735
   Leu Ala Val Pro Glu Gln Glu Leu Tyr Ser Trp Thr Ile Ser Ile Glu
   740 745 750
   Tyr
<210> 11
   <211> 777
   <212> PRT
   <213> Pisum sativum
<400> 11
   Met Thr Val Thr Pro Lys Ile Ser Val Asn Asp Gly Asn Leu Val Val
   1               5                   10                  15
   His Gly Lys Thr Ile Leu Lys Gly Val Pro Glu Asn Val Val Leu Thr
               20                  25                  30
   Pro Gly Ser Gly Asn Gly Leu Leu Thr Gly Gly Ala Phe Ile Gly Ala
           35                  40                  45
   Thr Ala Ser Asn Ser Lys Ser Leu His Val Phe Pro Ile Gly Ile Leu
       50                  55                  60
   Glu Gly Leu Arg Phe Val Cys Cys Phe Arg Phe Lys Leu Trp Trp Met
   65                  70                  75                  80
   Thr Gln Arg Met Gly Thr Cys Gly Arg Asp Ile Pro Leu Glu Thr Gln
                   85                  90                  95
   Phe Met Leu Ile Glu Ser Lys Asp Ser Glu Gly Glu Glu Gly Asn Ser
               100                 105                 110
   Pro Val Ile Tyr Thr Val Leu Leu Pro Leu Leu Glu Gly Pro Phe Arg
           115                 120                 125
   Ser Val Leu Gln Gly Asn Glu Lys Ser Glu Ile Glu Ile Cys Phe Glu
       130                 135                 140
   Ser Gly Asp His Ala Val Glu Thr Asn Gln Gly Leu His Met Val Tyr
   145                 150                 155                 160
   Met His Ala Gly Thr Asn Pro Phe Glu Val Ile Asn Gln Ala Val Lys
                   165                 170                 175
   Ala Val Glu Lys His Met Gln Thr Phe His His Arg Glu Lys Lys Arg
               180                 185                 190
   Leu Pro Ser Phe Leu Asp Met Phe Gly Trp Cys Thr Trp Asp Ala Phe
           195                 200                 205
   Tyr Thr Asp Val Thr Ala Glu Gly Val Glu Gln Gly Leu Lys Ser Leu
       210                 215                 220
   Ser Glu Gly Gly Thr Pro Pro Arg Phe Leu Ile Ile Asp Asp Gly Trp
   225                 230                 235                 240
   Gln Gln Ile Glu Ser Lys Ala Lys Asp Pro Gly Cys Val Val Gln Glu
                   245                 250                 255
   Gly Ala Gln Phe Ala Thr Met Leu Thr Gly Ile Lys Glu Asn Ala Lys
               260                 265                 270
   Phe Gln Lys Asn Lys Asn Glu Glu His Ser Glu Pro Thr Ser Gly Leu
           275                 280                 285
   Lys His Leu Val Asp Gly Val Lys Lys His His Asn Val Lys Asn Val
       290                 295                 300
   Tyr Val Trp His Ala Leu Ala Gly Tyr Trp Gly Gly Val Lys Pro Ala
   305                 310                 315                 320
   Ala Thr Gly Met Glu His Tyr Asp Thr Ala Leu Ala Tyr Pro Val Gln
                   325                 330                 335
   Ser Pro Gly Val Leu Gly Asn Gln Pro Asp Ile Val Met Asp Ser Leu
               340                 345                 350
   Ser Val His Gly Leu Gly Leu Val His Pro Lys Lys Val Phe Asn Phe
           355                 360                 365
   Tyr Asn Glu Leu His Ala Tyr Leu Ala Ser Cys Gly Val Asp Gly Val
       370                 375                 380
   Lys Val Asp Val Gln Asn Ile Ile Glu Thr Leu Gly Ala Gly His Gly
   385                 390                 395                 400
   Gly Arg Val Ser Leu Thr Arg Ser Tyr His His Ala Leu Glu Ala Ser
                   405                 410                 415
   Ile Ala Arg Asn Phe Ser Asp Asn Gly Cys Ile Ala Cys Met Cys His
               420                 425                 430
   Asn Thr Asp Gly Leu Tyr Ser Ala Lys Gln Thr Ala Val Val Arg Ala
           435                 440                 445
   Ser Asp Asp Phe Tyr Pro Arg Asp Pro Ala Ser His Thr Ile His Ile
       450                 455                 460
   Ser Ser Val Ala Tyr Asn Ser Leu Phe Leu Gly Glu Phe Met Gln Pro
   465                 470                 475                 480
   Asp Trp Asp Met Phe His Ser Leu His Pro Ala Ala Glu Tyr His Ala
                   485                 490                 495
   Ala Ala Arg Ala Ile Gly Gly Cys Pro Ile Tyr Val Ser Asp Lys Pro
               500                 505                 510
   Gly Asn His Asn Phe Asp Leu Leu Lys Lys Leu Val Leu Ser Asp Gly
           515                 520                 525
   Ser Val Leu Arg Ala Gln Leu Pro Gly Arg Pro Thr Arg Asp Ser Leu
       530                 535                 540
   Phe Val Asp Pro Ala Arg Asp Arg Thr Ser Leu Leu Lys Ile Trp Asn
   545 550 555 560
   Met Asn Lys Cys Thr Gly Val Val Gly Val Phe Asn Cys Gln Gly Ala
   565 570 575
   Gly Trp Cys Lys Val Glu Lys Lys Thr Arg Ile His Asp Ile Ser Pro
   580 585 590
   Gly Thr Leu Thr Ser Ser Val Cys Ala Ser Asp Val Asp Leu Ile Thr
   595 600 605
   Gln Val Ala Gly Ala Glu Trp His Gly Glu Thr Ile Val Tyr Ala Tyr
   610 615 620
   Arg Ser Gly Glu Val Ile Arg Leu Pro Lys Gly Val Ser Ile Pro Val
   625 630 635 640
   Thr Leu Lys Val Leu Glu Phe Glu Leu Phe His Phe Cys Pro Ile Gln
   645 650 655
   Glu Ile Ser Ser Ser Ile Ser Phe Ala Thr Ile Gly Leu Met Asp Met
   660 665 670
   Phe Asn Thr Gly Gly Ala Val Glu Glu Val Glu Ile His Arg Glu Thr
   675 680 685
   Asp Asn Lys Gln Glu Leu Phe Glu Gly Glu Ala Val Ser Ser Glu Leu
   690 695 700
   Ile Thr Ser Leu Gly Pro Asn Arg Thr Thr Thr Ala Thr Ile Thr Leu
   705 710 715 720
   Lys Val Arg Gly Ser Gly Lys Phe Gly Val Tyr Ser Ser Gln Arg Pro
   725 730 735
   Ile Lys Cys Met Val Asp Gly Thr Glu Thr Asp Phe Asn Tyr Asp Ser
   740 745 750
   Glu Thr Gly Leu Thr Thr Phe Ile Ile Pro Val Pro Gln Glu Glu Leu
   755 760 765
   Tyr Lys Trp Leu Ile Glu Ile Gln Val
   770 775
<210> 12
   <211> 753
   <212> PRT
   <213> Cucumis satlvus
<400> 12
   Met Thr Val Gly Ala Gly Ile Thr Ile Ser Asp Ala Asn Leu Thr Val
   1 5 10 15
   Leu Gly Asn Arg Val Leu Ser Asp Val His Asn Asn Ile Thr Leu Thr
   20 25 30
   Ala Ala Pro Gly Gly Gly Val Met Asn Gly Ala Phe Ile Gly Val Gln
   35 40 45
   Ser Asp Gln Ile Gly Ser Arg Arg Val Phe Pro Ile Gly Lys Leu Ile
   50 55 60
   Gly Leu Arg Phe Leu Cys Ala Phe Arg Phe Lys Leu Trp Trp Met Thr
   65 70 75 80
   Gln Arg Met Gly Cys Ser Gly Gln Glu Ile Pro Phe Glu Thr Gln Phe
   85 90 95
   Leu Val Val Glu Thr Arg Asp Gly Ser Asn Ile Ala Gly Asn Gly Glu
   100 105 110
   Glu Gly Asp Ala Val Tyr Thr Val Phe Leu Pro Ile Leu Glu Gly Asp
   115 120 125
   Phe Arg Ala Val Leu Gln Gly Asn Asp Asn Asn Glu Leu Glu Ile Cys
   130 135 140
   Leu Glu Ser Gly Asp Pro Ser Val Asp Gly Phe Glu Gly Ser His Leu
   145 150 155 160
   Val Phe Val Gly Ala Gly Ser Asp Pro Phe Glu Thr Ile Thr Tyr Ala
   165 170 175
   Val Lys Ser Val Glu Lys His Leu Gln Thr Phe Ala His Arg Glu Arg
   180 185 190
   Lys Lys Met Pro Asp Ile Leu Asn Trp Phe Gly Trp Cys Thr Trp Asp
   195 200 205
   Ala Phe Tyr Thr Asp Val Thr Ser Asp Gly Val Lys Lys Gly Leu Glu
   210 215 220
   Ser Phe Glu Asn Gly Gly Ile Pro Pro Lys Phe Val Ile Ile Asp Asp
   225 230 235 240
   Gly Trp Gln Ser Val Ala Lys Asp Ala Ala Ser Thr Asp Cys Lys Ala
   245 250 255
   Asp Asn Thr Ala Asn Phe Ala Asn Arg Leu Thr His Ile Lys Glu Asn
   260 265 270
   Tyr Lys Phe Gln Lys Asp Gly Lys Glu Gly Glu Arg Ile Glu Asn Pro
   275 280 285
   Ala Leu Gly Leu Gln His Ile Val Ser Tyr Met Lys Glu Lys His Ala
   290 295 300
   Thr Lys Tyr Val Tyr Val Trp His Ala Ile Thr Gly Tyr Trp Gly Gly
   305 310 315 320
   Val Ser Ser Gly Val Lys Glu Met Glu Gln Tyr Glu Ser Lys Ile Ala
                   325                 330                 335
   Tyr Pro Val Ala Ser Pro Gly Val Glu Ser Asn Glu Pro Cys Asp Ala
               340                 345                 350
   Leu Asn Ser Ile Ser Lys Thr Gly Leu Gly Leu Val Asn Pro Glu Lys
           355                 360                 365
   Val Phe Asn Phe Tyr Asn Glu Gln His Ser Tyr Leu Ala Ser Ala Gly
       370                 375                 380
   Val Asp Gly Val Lys Val Asp Val Gln Asn Ile Leu Glu Thr Leu Gly
   385                 390                 395                 400
   Ala Gly His Gly Gly Arg Val Lys Leu Ala Arg Lys Tyr His Gln Ala
                   405                 410                 415
   Leu Glu Ala Ser Ile Ser Arg Asn Phe Gln Asp Asn Gly Ile Ile Ser
               420                 425                 430
   Cys Met Ser His Asn Thr Asp Gly Leu Tyr Ser Ser Lys Arg Asn Ala
           435                 440                 445
   Val Ile Arg Ala Ser Asp Asp Phe Trp Pro Arg Asp Pro Ala Ser His
       450                 455                 460
   Thr Ile His Ile Ala Ser Val Ala Tyr Asn Ser Leu Phe Leu Gly Glu
   465                 470                 475                 480
   Phe Met Gln Pro Asp Trp Asp Met Phe His Ser Leu His Pro Met Ala
                   485                 490                 495
   Glu Tyr His Gly Ala Ala Arg Ala Val Gly Gly Cys Ala Ile Tyr Val
               500                 505                 510
   Ser Asp Lys Pro Gly Gln His Asp Phe Asn Leu Leu Lys Lys Leu Val
           515                 520                 525
   Leu His Asp Gly Ser Ile Leu Arg Ala Lys Leu Pro Gly Arg Pro Thr
       530                 535                 540
   Lys Asp Cys Leu Phe Ala Asp Pro Ala Arg Asp Gly Lys Ser Leu Leu
   545                 550                 555                 560
   Lys Ile Trp Asn Met Asn Asp Leu Ser Gly Val Val Gly Val Phe Asn
                   565                 570                 575
   Cys Gln Gly Ala Gly Trp Cys Lys Val Gly Lys Lys Asn Leu Ile His
               580                 585                 590
   Asp Glu Asn Pro Asp Thr Ile Thr Gly Val Ile Arg Ala Lys Asp Val
           595                 600                 605
   Ser Tyr Leu Trp Lys Ile Ala Gly Glu Ser Trp Thr Gly Asp Ala Val
       610                 615                 620
   Ile Phe Ser His Leu Ala Gly Glu Val Val Tyr Leu Pro Gln Asp Ala
   625 630 635 640
   Ser Met Pro Ile Thr Leu Lys Ser Arg Glu Phe Asp Val Phe Thr Val
   645 650 655
   Val Pro Val Lys Glu Leu Ala Asn Asp Ile Lys Phe Ala Pro Ile Gly
   660 665 670
   Leu Met Lys Met Phe Asn Ser Gly Gly Ala Val Lys Glu Met Asn His
   675 680 685
   Gln Pro Gly Ser Ser Asn Val Ser Leu Lys Val Arg Gly Ser Gly Pro
   690 695 700
   Phe Gly Ala Tyr Ser Ser Ser Lys Pro Lys Arg Val Ala Val Asp Ser
   705 710 715 720
   Glu Glu Val Glu Phe Ile Tyr Asp Glu Gly Gly Leu Ile Thr Ile Asp
   725 730 735
   Leu Lys Val Pro Glu Lys Glu Leu Tyr Leu Trp Asp Ile Arg Ile Glu
   740 745 750
   Leu
<210> 13
   <211> 772
   <212> PRT
   <213> Cucumis melo
<400> 13
   Met Thr Val Thr Pro Lys Ile Ser Val Asn Asp Gly Asn Leu Val Val
   1 5 10 15
   His Gly Lys Thr Ile Leu Thr Gly Val Pro Asp Asn Ile Val Leu Thr
   20 25 30
   Pro Gly Ser Gly Leu Gly Leu Val Ala Gly Ala Phe Ile Gly Ala Thr
   35 40 45
   Ala Ser Asn Ser Lys Ser Leu His Val Phe Pro Val Gly Val Leu Glu
   50 55 60
   Gly Thr Arg Phe Leu Cys Cys Phe Arg Phe Lys Leu Trp Trp Met Thr
   65 70 75 80
   Gln Arg Met Gly Thr Ser Gly Arg Asp Ile Pro Phe Glu Thr Gln Phe
   85 90 95
   Leu Leu Met Glu Ser Lys Gly Asn Asp Gly Glu Asp Pro Asp Asn Ser
   100 105 110
   Ser Thr Ile Tyr Thr Val Phe Leu Pro Leu Leu Glu Gly Gln Phe Arg
   115 120 125
   Ala Ala Leu Gln Gly Asn Glu Lys Asn Glu Met Glu Ile Cys Leu Glu
   130 135 140
   Ser Gly Asp Asn Thr Val Glu Thr Asn Gln Gly Leu Ser Leu Val Tyr
   145 150 155 160
   Met His Ala Gly Thr Asn Pro Phe Glu Val Ile Thr Gln Ala Val Lys
   165 170 175
   Ala Val Glu Lys His Thr Gln Thr Phe Leu His Arg Glu Lys Lys Lys
   180 185 190
   Leu Pro Ser Phe Leu Asp Trp Phe Gly Trp Cys Thr Trp Asp Ala Phe
   195 200 205
   Tyr Thr Asp Ala Thr Ala Glu Gly Val Val Glu Gly Leu Lys Ser Leu
   210 215 220
   Ser Glu Gly Gly Ala Pro Pro Lys Phe Leu Ile Ile Asp Asp Gly Trp
   225 230 235 240
   Gln Gln Ile Glu Ala Lys Pro Lys Asp Ala Asp Cys Val Val Gln Glu
   245 250 255
   Gly Ala Gln Phe Ala Ser Arg Leu Ser Gly Ile Lys Glu Asn His Lys
   260 265 270
   Phe Gln Lys Asn Gly Asn Asn Tyr Asp Gln Val Pro Gly Leu Lys Val
   275 280 285
   Val Val Asp Asp Ala Lys Lys Gln His Lys Val Lys Phe Val Tyr Ala
   290 295 300
   Trp His Ala Leu Ala Gly Tyr Trp Gly Gly Val Lys Pro Ala Ser Pro
   305 310 315 320
   Gly Met Glu His Tyr Asp Ser Ala Leu Ala Tyr Pro Val Gln Ser Pro
   325 330 335
   Gly Met Leu Gly Asn Gln Pro Asp Ile Val Val Asp Ser Leu Ala Val
   340 345 350
   His Gly Ile Gly Leu Val His Pro Lys Lys Val Phe Asn Phe Tyr Asn
   355 360 365
   Glu Leu His Ser Tyr Leu Ala Ser Cys Gly Ile Asp Gly Val Lys Val
   370 375 380
   Asp Val Gln Asn Ile Ile Glu Thr Leu Gly Ala Gly His Gly Gly Arg
   385 390 395 400
   Val Thr Leu Thr Arg Ser Tyr His Gln Ala Leu Glu Ala Ser Ile Ala
   405 410 415
   Arg Asn Phe Ser Asp Asn Gly Cys Ile Ala Cys Met Cys His Asn Thr
   420 425 430
   Asp Ser Leu Tyr Ser Ala Lys Gln Thr Ala Val Val Arg Ala Ser Asp
   435 440 445
   Asp Tyr Tyr Pro Arg Asp Pro Thr Ser His Thr Ile His Ile Ser Ser
   450 455 460
   Val Ala Tyr Asn Ser Leu Phe Leu Gly Glu Phe Met Gln Pro Asp Trp
   465 470 475 480
   Asp Met Phe His Ser Leu His Pro Thr Ala Glu Tyr His Gly Ala Ala
   485 490 495
   Arg Ala Ile Gly Gly Cys Ala Ile Tyr Val Ser Asp Lys Pro Gly Asn
   500 505 510
   His Asn Phe Asp Leu Leu Lys Lys Leu Val Leu Pro Asp Gly Ser Val
   515 520 525
   Leu Arg Ala Gln Leu Pro Gly Arg Pro Thr Arg Asp Ser Leu Phe Asn
   530 535 540
   Asp Pro Ala Arg Asp Gly Ile Ser Leu Leu Lys Ile Trp Asn Met Asn
   545 550 555 560
   Lys Cys Ser Gly Val Val Gly Val Phe Asn Cys Gln Gly Ala Gly Trp
   565 570 575
   Cys Arg Ile Thr Lys Lys Thr Arg Ile His Asp Glu Ser Pro Gly Thr
   580 585 590
   Leu Thr Thr Ser Val Arg Ala Ala Asp Val Asp Ala Ile Ser Gln Val
   595 600 605
   Ala Gly Ala Asp Trp Lys Gly Asp Thr Ile Val Tyr Ala Tyr Arg Ser
   610 615 620
   Gly Asp Leu Ile Arg Leu Pro Lys Gly Ala Ser Val Pro Val Thr Leu
   625 630 635 640
   Lys Val Leu Glu Tyr Asp Leu Leu His Ile Ser Pro Leu Lys Asp Ile
   645 650 655
   Ala Ser Asn Ile Ser Phe Ala Pro Ile Gly Leu Leu Asp Met Phe Asn
   660 665 670
   Thr Gly Gly Ala Val Glu Gln Val Asn Val Gln Val Val Glu Pro Ile
   675 680 685
   Pro Glu Phe Asp Gly Glu Val Ala Ser Glu Leu Thr Cys Ser Leu Pro
   690 695 700
   Asn Asp Arg Pro Pro Thr Ala Thr Ile Thr Met Lys Ala Arg Gly Cys
   705 710 715 720
   Arg Arg Phe Gly Leu Tyr Ser Ser Gln Arg Pro Leu Lys Cys Ser Val
   725 730 735
   Asp Lys Val Asp Val Asp Phe Val Tyr Asp Glu Val Thr Gly Leu Val
   740 745 750
   Thr Phe Glu Ile Pro Ile Pro Thr Glu Glu Met Tyr Arg Trp Asp Ile
   755 760 765
   Glu Ile Gln Val
   770
<210> 14
   <211> 378
   <212> PRT
   <213> Coffea arabica
<400> 14
   Met Val Lys Ser Pro Gly Thr Glu Asp Tyr Thr Arg Arg Ser Leu Leu
   1 5 10 15
   Ala Asn Gly Leu Gly Leu Thr Pro Pro Met Gly Trp Asn Ser Trp Asn
   20 25 30
   His Phe Arg Cys Asn Leu Asp Glu Lys Leu Ile Arg Glu Thr Ala Asp
   35 40 45
   Ala Met Val Ser Lys Gly Leu Ala Ala Leu Gly Tyr Lys Tyr Ile Asn
   50 55 60
   Leu Asp Asp Cys Trp Ala Glu Leu Asn Arg Asp Ser Gln Gly Asn Leu
   65 70 75 80
   Val Pro Lys Gly Ser Thr Phe Pro Ser Gly Ile Lys Ala Leu Ala Asp
   85 90 95
   Tyr Val His Ser Lys Gly Leu Lys Leu Gly Ile Tyr Ser Asp Ala Gly
   100 105 110
   Thr Gln Thr Cys Ser Lys Thr Met Pro Gly Ser Leu Gly His Glu Glu
   115 120 125
   Gln Asp Ala Lys Thr Phe Ala Ser Trp Gly Val Asp Tyr Leu Lys Tyr
   130 135 140
   Asp Asn Cys Asn Asn Asn Asn Ile Ser Pro Lys Glu Arg Tyr Pro Ile
   145 150 155 160
   Met Ser Lys Ala Leu Leu Asn Ser Gly Arg Ser Ile Phe Phe Ser Leu
   165 170 175
   Cys Glu Trp Gly Glu Glu Asp Pro Ala Thr Trp Ala Lys Glu Val Gly
   180 185 190
   Asn Ser Trp Arg Thr Thr Gly Asp Ile Asp Asp Ser Trp Ser Ser Met
   195 200 205
   Thr Ser Arg Ala Asp Met Asn Asp Lys Trp Ala Ser Tyr Ala Gly Pro
   210 215 220
   Gly Gly Trp Asn Asp Pro Asp Met Leu Glu Val Gly Asn Gly Gly Met
   225 230 235 240
   Thr Thr Thr Glu Tyr Arg Ser His Phe Ser Ile Trp Ala Leu Ala Lys
   245 250 255
   Ala Pro Leu Leu Ile Gly Cys Asp Ile Arg Ser Met Asp Gly Ala Thr
   260 265 270
   Phe Gln Leu Leu Ser Asn Ala Glu Val Ile Ala Val Asn Gln Asp Lys
   275 280 285
   Leu Gly Val Gln Gly Asn Lys Val Lys Thr Tyr Gly Asp Leu Glu Val
   290 295 300
   Trp Ala Gly Pro Leu Ser Gly Lys Arg Val Ala Val Ala Leu Trp Asn
   305 310 315 320
   Arg Gly Ser Ser Thr Ala Thr Ile Thr Ala Tyr Trp Ser Asp Val Gly
   325 330 335
   Leu Pro Ser Thr Ala Val Val Asn Ala Arg Asp Leu Trp Ala His Ser
   340 345 350
   Thr Glu Lys Ser Val Lys Gly Gln Ile Ser Ala Ala Val Asp Ala His
   355 360 365
   Asp Ser Lys Met Tyr Val Leu Thr Pro Gln
   370 375

## Description

### FIELD AND BACKGROUND OF THE INVENTION

The present invention, in some embodiments thereof, relates to novel multimeric protein structures and, more particularly, but not exclusively, to multimeric protein structures of α-galactosidase and to uses thereof in treating Fabry disease.

The lysosomal enzyme α-galactosidase-A (α-GAL or α-Gal A; EC 3.2.1.22) catalyzes the removal of galactose from oligosaccharides, glycoproteins and glycolipids during the catabolism of macromolecules. Deficiencies in lysosomal enzymes lead to the accumulation of their substrates in the tissues, conditions known as lysosomal storage diseases. In humans, the absence of functional α-galactosidase-A leads to the accumulation of glycolipids containing terminal α-galactose residues (primarily globotriaosylceramide, which is also referred to as "ceramide trihexoside", "CTH" or "Gb₃") in the tissues, leading to Fabry disease. Fabry disease is an X-linked recessive disorder, first described in 1898, characterized by chronic pain, ocular opacities, liver and kidney impairment, skin lesions, vascular deterioration and/or cardiac deficiencies. Recombinant human α-galactosidase-A has the ability to restore enzyme function in patients, and enzyme replacement therapy (ERT) using α-GAL was approved in the United States in 2003 as a treatment for Fabry disease. α-GAL became the second recombinant protein approved for the treatment of a lysosomal storage disorder after β-glucosidase, a treatment for Gaucher disease.

Endogenous and recombinant α-GALs catalyze the hydrolysis of terminal galactosylated glycolipids in the lysosomes of cells of organs such as the liver, kidneys, spleen, heart, etc. This natural action site is characterized by its low pH, reaching as low as 4.5. Lysosomal enzymes, including α-GAL, are hence designed to exert their maximal activity at these low pH levels.

Current Fabry ERT treatments are based on mammalian-cell derived recombinant α-GAL which is considered to be a limited efficiency treatment. These treatments only decelerate the progress of the disease but are not able to stop its progress and do not offer a true and complete solution. Furthermore, in some cases, ERT with commercial recombinant α-GALs must be ceased due to development of an immunogenic response to the treatment and in some cases the treatment cannot be initiated in light of immunogenicity problems.

X-ray structure analysis reveals that human α-GAL is a homodimeric glycoprotein with each monomer composed of two domains, a (β/α)₈ domain containing the active site and a C-terminal domain containing eight antiparallel β strands on two sheets in a β sandwich [Garman & Garboczi, J Mol Biol 2004, 337:319-335]. The two monomers are arranged in a head-to-tail assembly and the dimerization is non-covalent. The two monomers pack with an interface that extends the 75 Å width of the dimer and buries 2200 Å² of surface area. In the dimer interface, 30 residues from each monomer contribute to the interface. The two active sites of the dimer are separated by approximately 50 Å.

The crystal structure of α-Gal was solved for a non-liganded protein as well as for a galactose-liganded protein. These two structures exhibit little change between the liganded and non-liganded structures. Nevertheless, the use of galactose instead of the natural substrate, globotriaosylceramide (Gb₃), the latter characterized by long lipidic chains able to interact with the hydrophobic domain of one monomer while the terminal galactose interacts with the active site of the second monomer, may not lead to evidence of active site cooperativity. Furthermore, biochemical evidence does suggest such cooperativity, exemplifying the importance of the homodimeric quaternary structure [Bishop & Desnick, J Biol Chem 1981, 256:1307-1316]. Thus, the kinetic properties of human α-Gal were studied and cooperativity between the monomers of the homodimeric enzyme, each with an interacting catalytic site, was shown. It was therefore suggested that enzymatic activity and stability may be dependent on dimerization.

WO 2009/024977, by the present assignee, teaches conjugates of a saccharide and a biomolecule, covalently linked therebetween via a non-hydrophobic linker, as well as medical uses utilizing such conjugates.

PCT International Patent Application No. PCT/IL2010/000956, by the present assignee, teaches methodologies which utilize α-galactosidase which exhibits a lysosomal activity at pH levels higher than lysosomal pH.

Additional background art include Bendele et al. [Toxicological Sciences 1998, 42:152-157], U.S. Patent Nos. 5,256804, 5,580757 and 5,766,897, International Patent Application PCT/NL2007/050684 (published as WO 2008/075957), and Seely & Richey [J Chromatography A 2001, 908:235-241].

### SUMMARY OF THE INVENTION

According to an aspect of some embodiments of the present invention there is provided a multimeric protein structure comprising at least two α-galactosidase monomers being covalently linked to one another via a linking moiety, the multimeric protein structure featuring a characteristic selected from the group consisting of:
(a) an α-galactosidase activity upon subjecting the multimeric protein structure to human plasma conditions for one hour, which is at least 10 % higher than an activity of native α-galactosidase upon subjecting the native α-galactosidase to human plasma conditions for one hour;
(b) an α-galactosidase activity which decreases upon subjecting the multimeric protein structure to human plasma conditions for one hour by a percentage which is at least 10 % less than the percentage by which an activity of the native α-galactosidase decreases upon subjecting the native α-galactosidase to human plasma conditions for one hour;
(c) an α-galactosidase activity which remains substantially unchanged upon subjecting the multimeric protein structure to human plasma conditions for one hour;
(d) an α-galactosidase activity, upon subjecting the multimeric protein structure to lysosomal conditions for one week, which is at least 10 % higher than an activity of native α-galactosidase upon subjecting the native α-galactosidase to lysosonial conditions for one week;
(e) an α-galactosidase activity which decreases upon subjecting the multimeric protein structure to lysosomal conditions for one day by a percentage which is at least 10 % less than the percentage by which an activity of the native α-galactosidase decreases upon subjecting the native α-galactosidase to lysosomal conditions for one day;
(f) an α-galactosidase activity which remains substantially unchanged upon subjecting the multimeric protein structure to lysosomal conditions for one day;
(g) an α-galactosidase activity, immediately upon subjecting the multimeric protein structure to lysosomal conditions, which is at least 10 % higher than an activity of native α-galactosidase immediately upon subjecting the native form of the protein to lysosomal conditions;
(h) an α-galactosidase activity, immediately upon subjecting the multimeric protein structure to an aqueous solution having a pH of 7 and a temperature of 37 °C, which is at least 10 % higher than an activity of native α-galactosidase immediately upon subjecting the native α-galactosidase to an aqueous solution having a pH of 7 and a temperature of 37 °C; and
(i) a circulating half-life in a physiological system which is higher by at least 20 % than the circulating half-life of the native α-galactosidase.

According to an aspect of some embodiments of the present invention there is provided a multimeric protein structure comprising at least two α-galactosidase monomers being covalently linked to one another via a linking moiety, wherein the linking moiety is not present in native α-galactosidase.

According to an aspect of some embodiments of the present invention there is provided a pharmaceutical composition comprising a multimeric protein structure as described herein and a pharmaceutically acceptable carrier.

According to an aspect of some embodiments of the present invention there is provided a method of treating Fabry disease, the method comprising administering to a subject in need thereof a therapeutically effective amount of a multimeric protein structure as described herein, thereby treating the Fabry disease.

According to an aspect of some embodiments of the present invention there is provided a process of preparing a multimeric protein structure as described herein, the process comprising reacting α-galactosidase with a cross-linking agent which comprises the linking moiety described herein and at least two reactive groups.

According to some embodiments of the invention, the linking moiety described herein is not present in native α-galactosidase.

According to some embodiments of the invention, the multimeric protein structure features a characteristic selected from the group consisting of:
(a) an α-galactosidase activity, upon subjecting the multimeric protein structure to human plasma conditions for one hour, which is at least 10 % higher than an activity of native α-galactosidase upon subjecting the native α-galactosidase to human plasma conditions for one hour;
(b) an α-galactosidase activity which decreases upon subjecting the multimeric protein structure to human plasma conditions for one hour by a percentage which is at least 10 % less than the percentage by which an activity of the native α-galactosidase decreases upon subjecting the native α-galactosidase to human plasma conditions for one hour;
(c) an α-galactosidase activity which remains substantially unchanged upon subjecting the multimeric protein structure to human plasma conditions for one hour;
(d) an α-galactosidase activity, upon subjecting the multimeric protein structure to lysosomal conditions for one week, which is at least 10 % higher than an activity of native α-galactosidase upon subjecting the native α-galactosidase to lysosomal conditions for one week;
(e) an α-galactosidase activity which decreases upon subjecting the multimeric protein structure to lysosomal conditions for one day by a percentage which is at least 10 % less than the percentage by which an activity of the native α-galactosidase decreases upon subjecting the native α-galactosidase to lysosomal conditions for one day;
(f) an α-galactosidase activity which remains substantially unchanged upon subjecting the multimeric protein structure to lysosomal conditions for one day;
(g) an α-galactosidase activity, immediately upon subjecting the multimeric protein structure to lysosomal conditions, which is at least 10 % higher than an activity of native α-galactosidase immediately upon subjecting the native α-galactosidase to lysosomal conditions;
(h) an α-galactosidase activity, immediately upon subjecting the multimeric protein structure to an aqueous solution having a pH of 7 and a temperature of 37 °C, which is at least 10 % higher than an activity of native α-galactosidase immediately upon subjecting the native α-galactosidase to an aqueous solution having a pH of 7 and a temperature of 37 °C; and
(i) a circulating half-life in a physiological system which is higher than a circulating half-life of the native α-galactosidase.

According to some embodiments of the invention, the α-galactosidase activity of the multimeric protein structure which remains substantially unchanged upon subjecting the multimeric protein structure to lysosomal conditions for one day, further remains substantially unchanged upon subjecting the multimeric protein structure to lysosomal conditions for one week.

According to some embodiments of the invention, the circulating half-life of the multimeric protein structure which is higher than a circulating half-life of the native α-galactosidase, is higher by at least 20 % than the circulating half-life of the native α-galactosidase.

According to some embodiments of the invention, the circulating half-life of the multimeric protein structure which is higher than a circulating half-life of the native α-galactosidase, is higher by at least 50 % than the circulating half-life of the native α-galactosidase.

According to some embodiments of the invention, the multimeric protein structure is characterized by an α-galactosidase activity in an organ upon administration of the multimeric protein structure to a vertebrate, the organ being selected from the group consisting of a spleen, a heart and a kidney.

According to some embodiments of the invention, the multimeric protein structure comprises two α-galactosidase monomers, the protein structure being a dimeric protein structure.

According to some embodiments of the invention, the α-galactosidase is a human α-galactosidase.

According to some embodiments of the invention, the α-galactosidase is a plant recombinant α-galactosidase.

According to some embodiments of the invention, the α-galactosidase has an amino acids sequence selected from the group consisting of SEQ ID NO:1, SEQ ID NO:2 and SEQ ID NO:3.

According to some embodiments of the invention, the α-galactosidase is an alkaline α-galactosidase.

According to some embodiments of the invention, the α-galactosidase is an acid α-galactosidase.

According to some embodiments of the invention, the linking moiety comprises a poly(alkylene glycol).

According to some embodiments of the invention, the poly(alkylene glycol) comprises at least two functional groups, each functional group forming a covalent bond with one of the α-galactosidase monomers.

According to some embodiments of the invention, the at least two functional groups are terminal groups of the poly(alkylene glycol).

According to some embodiments of the invention, the at least one linking moiety has a general formula:

-X₁-(CR₁R₂-CR₃R₄-Y)n=X₂-

wherein each of X₁ and X₂ is a functional group that forms a covalent bond with at least one α-galactosidase monomer;
Y is O, S or NR₅;
n is an integer from 1 to 200; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

According to some embodiments of the invention, at least one of the functional groups forms an amide bond with an α-galactosidase monomer.

According to some embodiments of the invention, n is an integer from 5 to 150.

According to some embodiments of the invention, n is an integer from 40 to 70.

According to some embodiments of the invention, the pharmaceutical composition further comprises a galactose.

According to some embodiments of the invention, the multimeric protein structure is for use as a medicament.

According to some embodiments of the invention, the medicament is for treating Fabry disease.

According to some embodiments of the invention, the multimeric protein structure is for use in treating Fabry disease.

According to some embodiments of the invention, the process comprises reacting dimeric α-galactosidase with the cross-linking agent.

According to some embodiments of the invention, the reactive groups comprise a leaving group.

According to some embodiments of the invention, the reactive group reacts with an amine group to form an amide bond.

According to some embodiments of the invention, each of the reactive groups is capable of forming a covalent bond between the linking moiety and at least one α-galactosidase monomer.

According to some embodiments of the invention, a molar ratio of the cross-linking agent to monomers of α-galactosidase is in a range of from 5:1 to 500:1.

According to some embodiments of the invention, the molar ratio is in a range of from 75:1 to 300:1.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

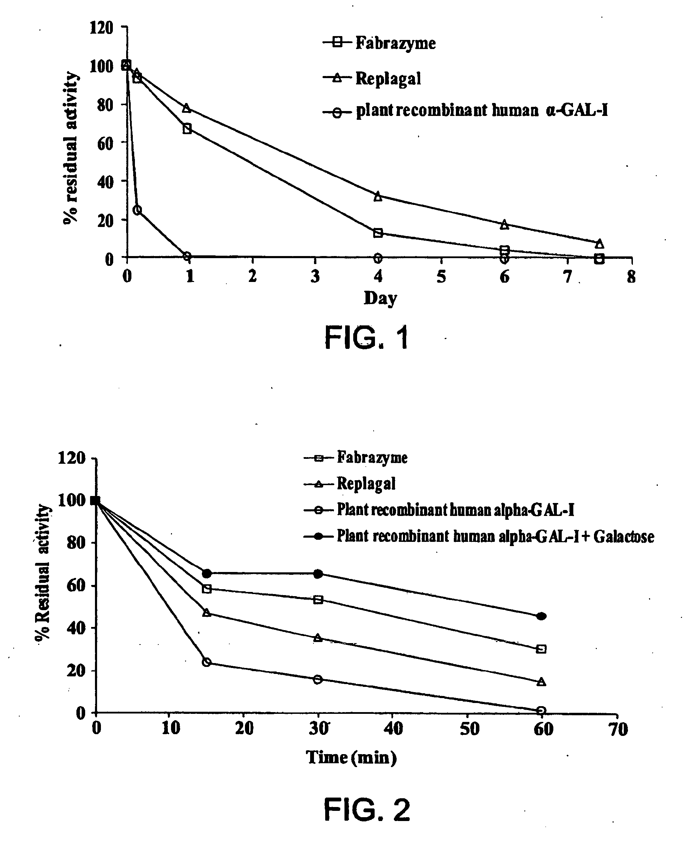

FIG. 1 is a graph showing the activity of Fabrazyme^{®} α-GAL, Replagal^{®} α-GAL and plant recombinant human α-GAL-I, as a function of incubation time under simulated lysosomal conditions (citrate phosphate buffer, pH 4.6, 37 °C);
FIG. 2 is a graph showing the activity of Fabrazyme^{®} α-GAL, Replagal^{®} α-GAL, plant recombinant human α-GAL-I, and plant recombinant α-GAL-I with galactose (100 mg/mL), as a function of incubation time under simulated physiological conditions (pH 7.4,37 °C);

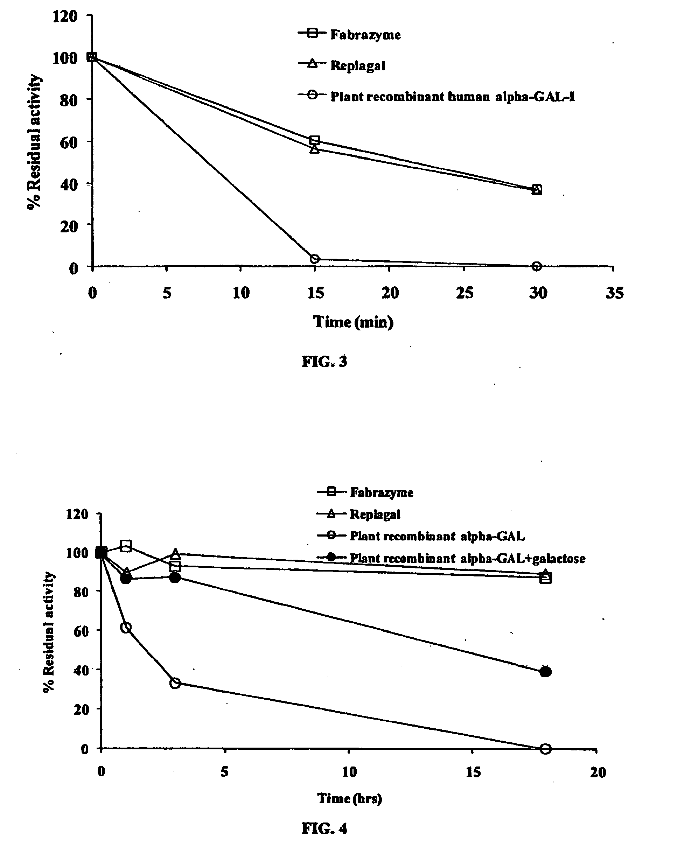

FIG. 3 is a graph showing the activity of Fabrazyme^{®} α-GAL, Replagal^{®} α-GAL and plant recombinant human α-GAL-I, as a function of incubation time in human plasma at 37 °C;
FIG. 4 is a graph showing the activity of Fabrazyme^{®} α-GAL, Replagal^{®} α-GAL, plant recombinant human α-GAL-I, and plant recombinant α-GAL-I with galactose (100 mg/mL), as a function of incubation time under simulated lysosomal conditions (citrate phosphate buffer, pH 4.6,37 °C);

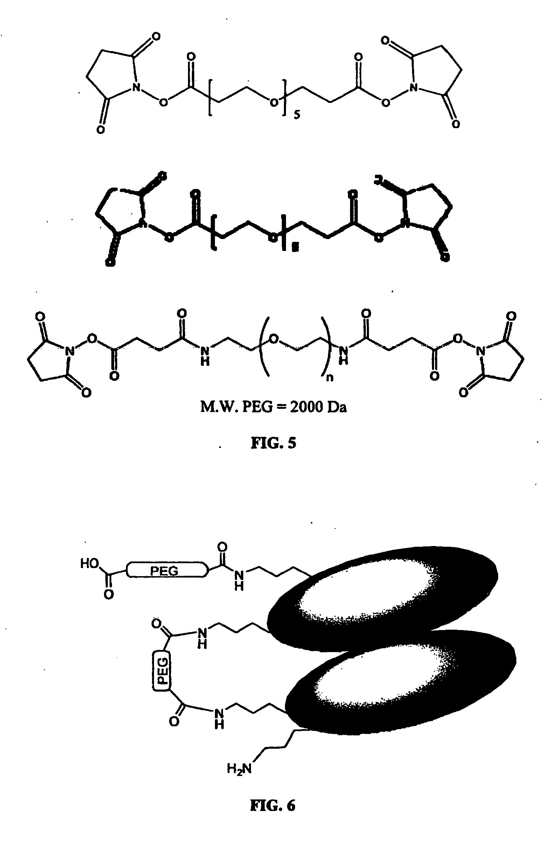

FIG. 5 is a scheme depicting the molecular structures of exemplary bis-*N-*hydroxysuccinimide-poly(ethylene glycol) (bis-NHS-PEG) cross-linking agents;
FIG. 6 is a scheme depicting a dimeric protein which has been reacted with bis-NHS-PEG cross-linking agents;

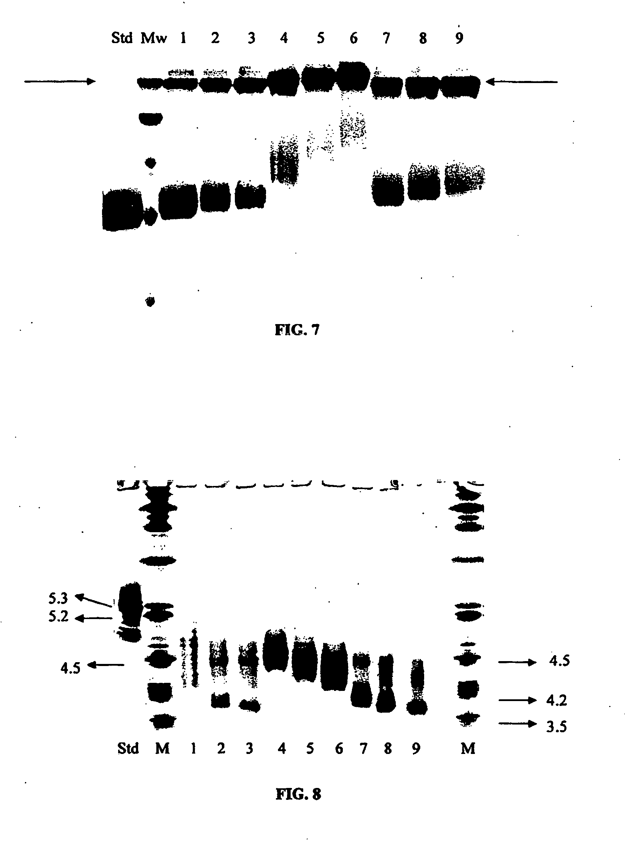

FIG. 7 presents a scan of an SDS-PAGE gel showing plant recombinant α-GAL-1 which was reacted with bis-NHS-PEG₅ (lanes 1-3), bis-NHS-PEG₈ (lanes 7-9), and bis-NHS-PEG₄₅ (lanes 4-6), at a molar ratio of 50:1 (lanes 1,4 and 7), 100:1 (lanes 2, 5 and 8) and 200:1 (lanes 3, 6 and 9) bis-NHS-PEG:α-GAL, as well as molecular weight markers (Mw) and non-reacted plant recombinant α-GAL-I standard (Std) (arrows show the band comprising an α-GAL dimer);
FIG. 8 presents a scan of an isoelectric focusing gel showing plant recombinant α-GAL-I which was reacted with bis-NHS-PEG₅ (lanes 1-3), bis-NHS-PEG₈ (lanes 7-9), and bis-NHS-PEG₄₅ (lanes 4-6), at a molar ratio of 50:1 (lanes 1, 4 and 7), 100:1 (lanes 2, 5 and 8) and 200:1 (lanes 3, 6 and 9) bis-NHS-PEG:α-GAL, as well as pH markers (M) and non-reacted plant recombinant α-GAL-I standard (Std) (arrows show pH values for various bands);

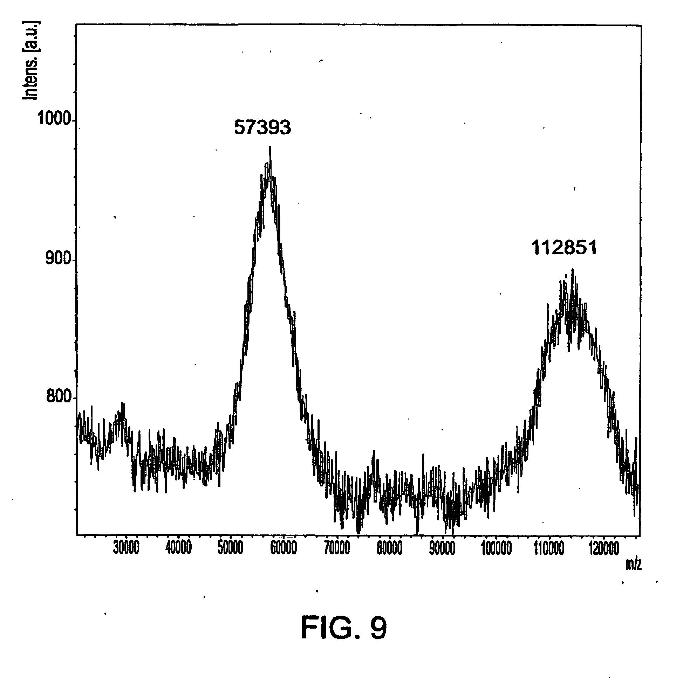

FIG. 9 is a MALDI-TOF mass spectroscopy spectrum of plant recombinant α-GAL-1 cross-linked by bis-NHS-PEG₄₅ (x-axis indicates m/z values, and m/z values of peaks are shown);

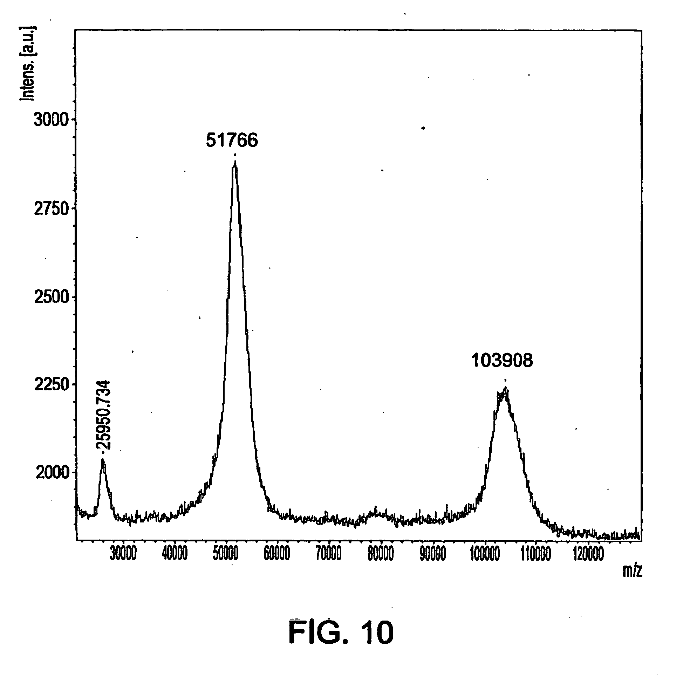

FIG. 10 is a MALDI-TOF mass spectroscopy spectrum of plant recombinant α-GAL-I cross-linked by bis-NHS-PEG₈ (x-axis indicates m/z values, and m/z values of peaks are shown);

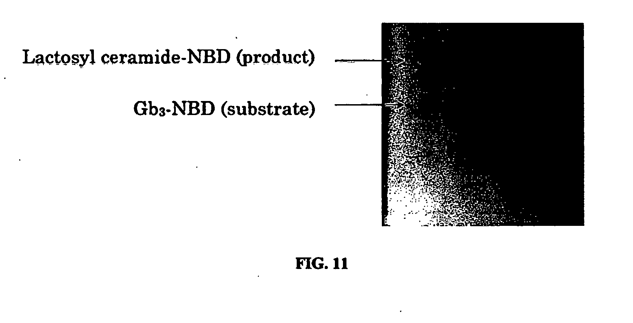

FIG. 11 presents a photograph showing the α-GAL substrate *N*-dodecanoyl-nitrobenzoxadiazole-ceramide trihexoside (Gb₃-NBD) and the α-GAL reaction product lactosyl ceramide-nitrobenzoxadiazole (lactosyl ceramide-NBD), as visualized by irradiation under UV light (365 nm) following high performance thin layer chromatography, following incubation of the substrate Gb₃-NBD with plant recombinant human α-GAL-I cross-linked with bis-NHS-PEG₄₅ (left lane), Replagal^{®} α-GAL (middle lane) and without α-GAL (right lane);

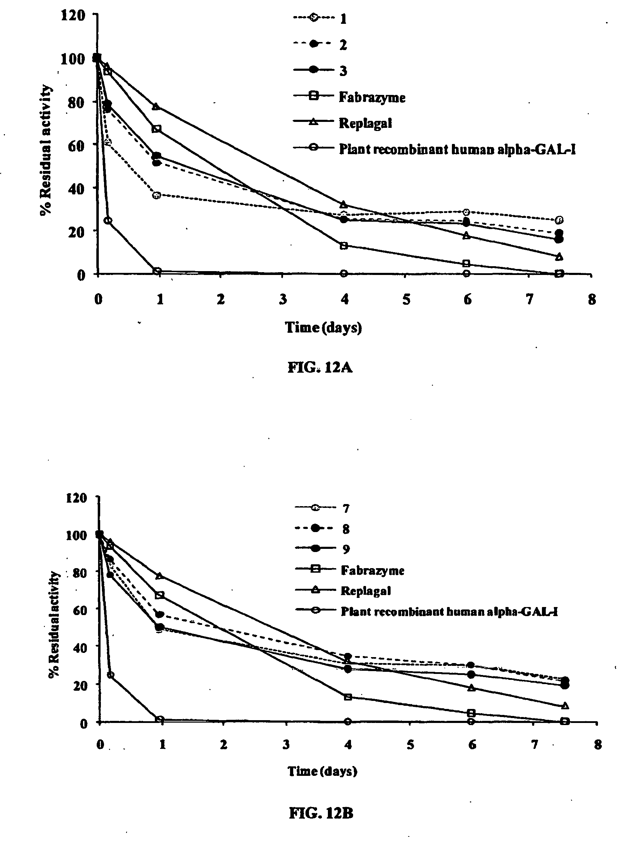

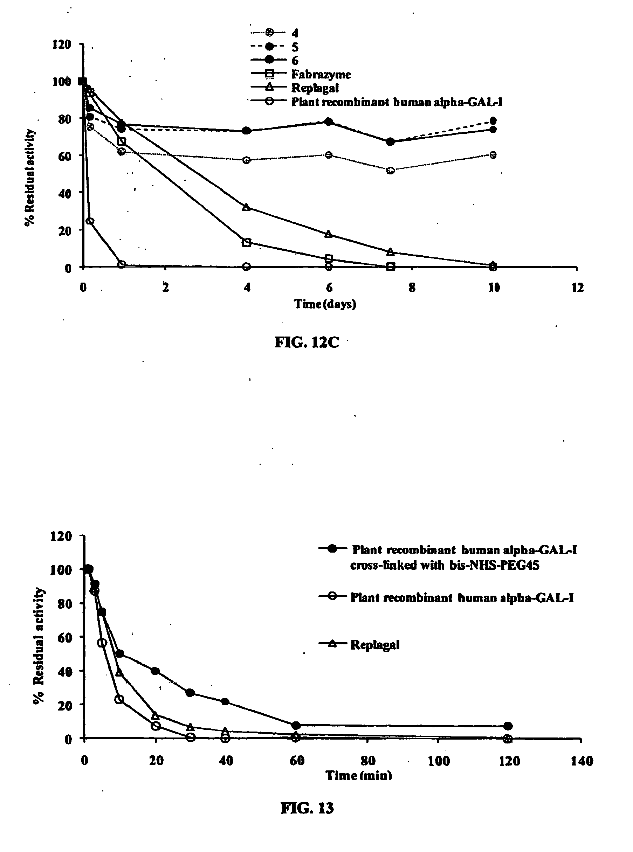

FIGs. 12A, 12B and 12C, are graphs showing the activity of Fabrazyme^{®} α-GAL, Replagal^{®} α-GAL, plant recombinant human α-GAL-I, and plant recombinant human α-GAL-I cross-linked with bis-NHS-PEG₅ (FIG. 12A), bis-NHS-PEG₈ (FIG. 12B) and bis-NHS-PEG₄₅ (FIG. 12C) at a molar ratio of 50:1 ("1" in FIG. 12A, "7" in FIG. 12B and "4" in FIG. 12C), 100:1 ("2" in FIG. 12A, "8" in FIG. 12B and "5" in FIG. 12C) and 200:1 ("3" in FIG. 12A, "9" in FIG. 12B and "6" in FIG. 12C) bis-NHS-PEG:α-GAL as a function of incubation time under simulated lysosomal conditions (citrate phosphate buffer, pH 4.6,37 °C);
FIG. 13 is a graph showing the pharmacokinetic profile of Replagal^{®} α-GAL, plant recombinant human α-GAL-I, and plant recombinant human α-GAL-I cross-linked with bis-NHS-PEG₄₅ in the plasma of Fabry mice; the residual activity of each α-GAL is presented as a percentage of the maximal residual activity of each α-GAL, as a function of time following injection of the α-GALs;

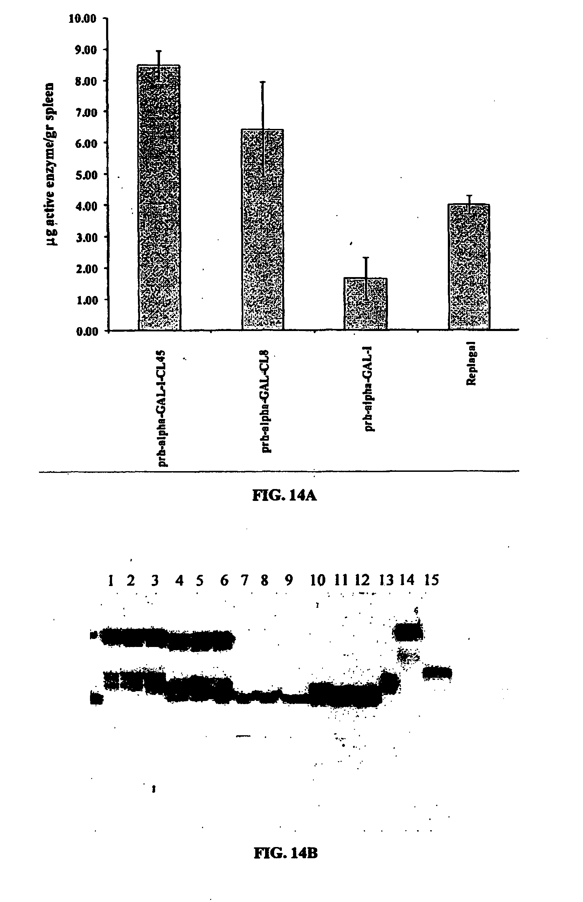

FIGs. 14A and 14B present a graph (FIG. 14A) showing the activity of Replagal^{®} α-GAL, plant recombinant human α-GAL-I (prh-alpha-GAL-I), and plant recombinant human α-GAL-I cross-linked with bis-NHS-PEG₈ (prh-alpha-GAL-I-CL8) or bis-NHS-PEG₄₅ (prh-alpha-GAL-1-CL45 ) in the spleens of Fabry mice 2 hours following injection of α-GAL, and a photograph of a Western blot (FIG. 14B) showing Replagal^{®} α-GAL (lanes 10-12 and 15), plant recombinant human α-GAL-I (lanes 7-9 and 13), and plant recombinant human α-GAL-I cross-linked with bis-NHS-PEG₈ (lanes 4-6) or bis-NHS-PEG₄₅ (lanes 1-3 and 14) in the spleens of Fabry mice following injection of α-GAL (lanes 1-12) or as a standard consisting of 50 ng α-GAL (lanes 13-15);

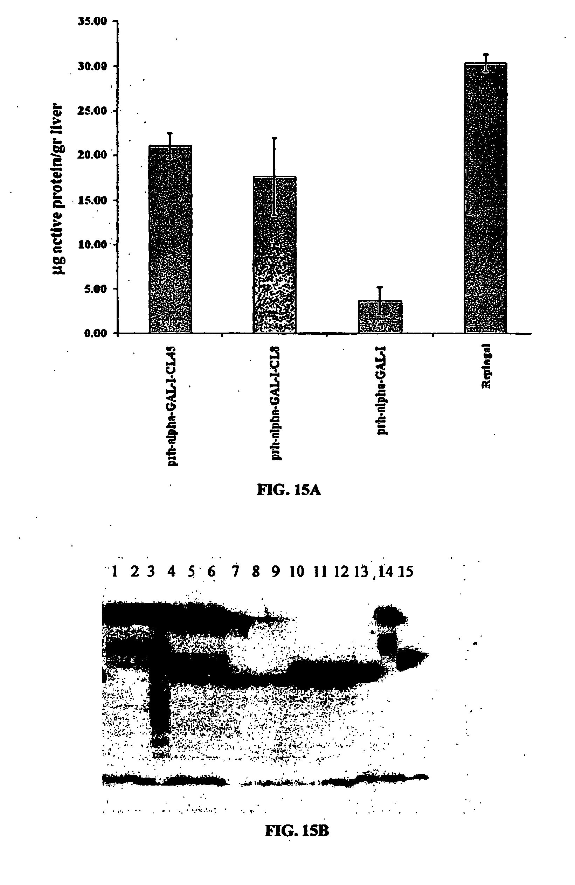

FIGs. 15A and 15B present a graph (FIG. 15A) showing the activity of Replagal^{®} α-GAL, plant recombinant human α-GAL-I (prh-alpha-GAL-I), and plant recombinant human α-GAL-I cross-linked with bis-NHS-PEG₈ (prh-alpha-GAL-I-CL8) or bis-NHS-PEG₄₅ (prh-alpha-GAL-I-CL45) in the livers of Fabry mice 2 hours following injection of α-GAL, and a photograph of a Western blot (FIG. 15B) showing Replagal^{®} α-GAL (lanes 10-12 and 15), plant recombinant human α-GAL-I (lanes 7-9 and 13), and plant recombinant human α-GAL-I cross-linked with bis-NHS-PEG₈ (lanes 4-6) or bis-NHS-PEG₄₅ (lanes 1-3 and 14) in the livers of Fabry mice following injection of α-GAL (lanes 1-12) or as a standard consisting of 50 ng α-GAL (lanes 13-15);

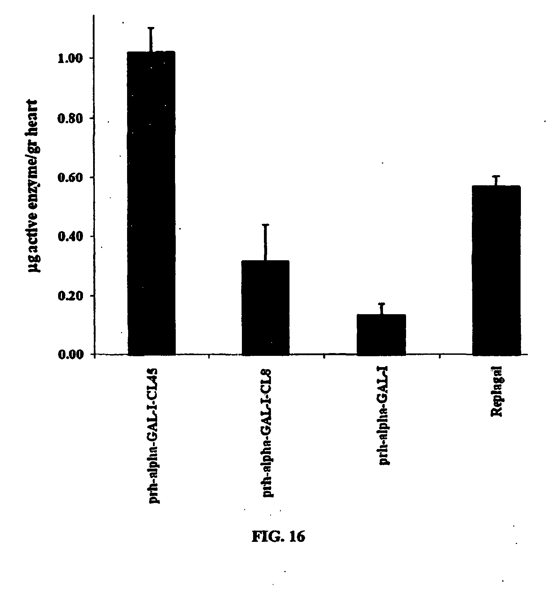

FIG. 16 is a graph showing the activity of Replagal^{®} α-GAL, plant recombinant human α-GAL-I (prh-alpha-GAL-I), and plant recombinant human α-GAL-I cross-linked with bis-NHS-PEG₈ (prh-alpha-GAL-I-) or bis-NHS-PEG₄₅ (prh-alpha-GAL-I-CL45) in the hearts of Fabry mice 2 hours following injection of α-GAL;

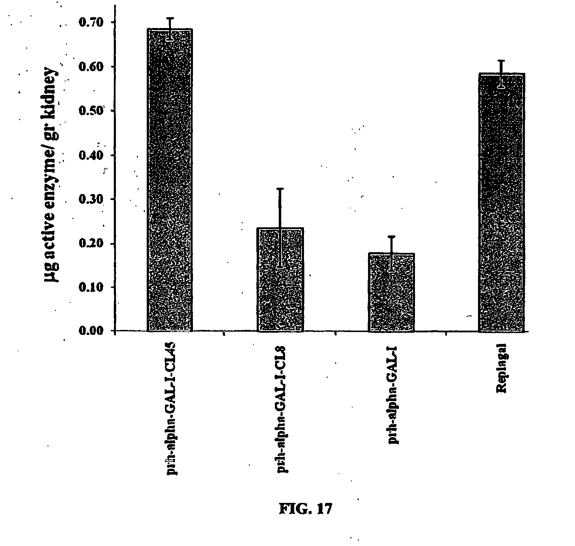

FIG. 17 is a graph showing the activity of Replagal^{®} α-GAL, plant recombinant human α-GAL-I (prh-alpha-GAL-I), and plant recombinant human α-GAL-I cross-linked with bis-NHS-PEG₈ (prh-alpha-GAL-I-CL8) or bis-NHS-PEG₄₅ (prh-alpha-GAL-I-CL45) in the kidneys of Fabry mice 2 hours following injection of α-GAL;

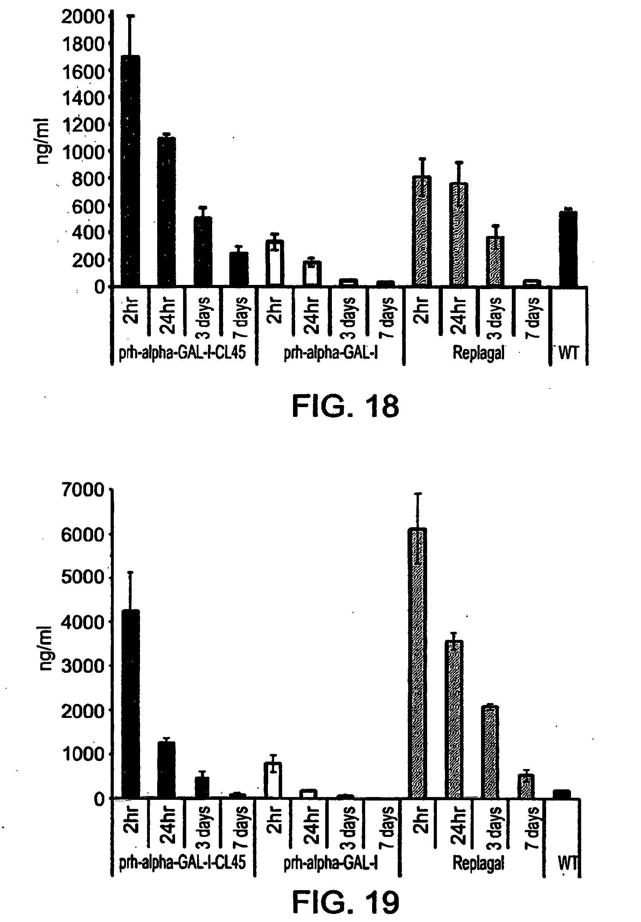

FIG. 18 is a graph showing the activity of Replagal^{®} α-GAL and plant recombinant human α-GAL-I (prh-alpha-GAL-I), and plant recombinant human α-GAL-I cross-linked with bis-NHS-PEG₄₅ (prh-alpha-GAL-I-CL45) in the spleens of Fabry mice 2 hours, 24 hours, 3 days and 7 days following injection of α-GAL (endogenous wild type α-GAL (WT) is shown as a standard);
FIG. 19 is a graph showing the activity of Replagal^{®} α-GAL and plant recombinant human α-GAL-I (prh-alpha-GAL-I), and plant recombinant human α-GAL I cross-linked with bis-NHS-PEG₄₅ (prh-alpha-GAL-I-CL45) in the livers of Fabry mice 2 hours, 24 hours, 3 days and 7 days following injection of α-GAL (endogenous wild type α-GAL (WT) is shown as a standard);

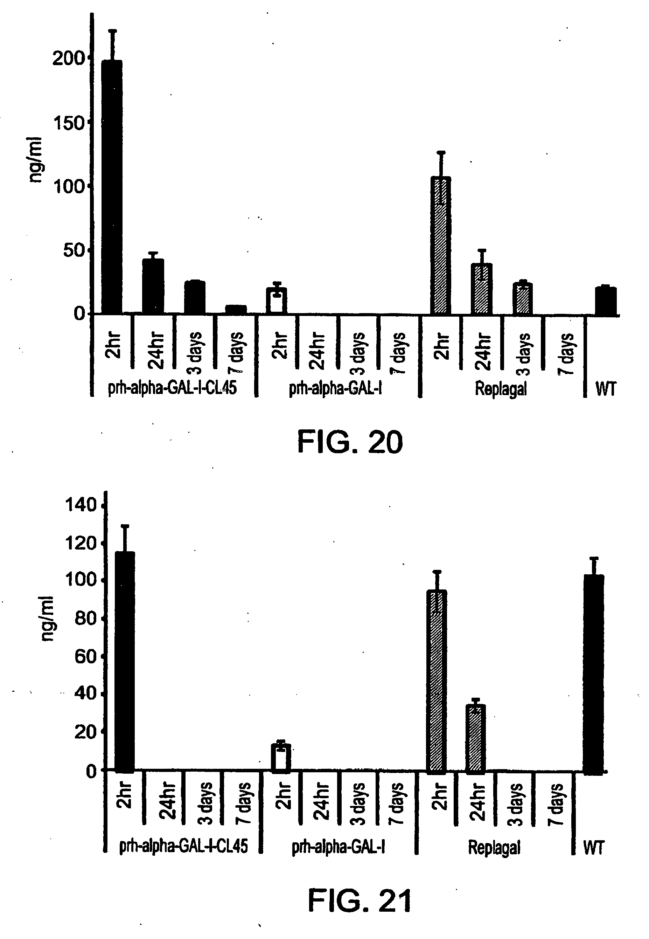

FIG. 20 is a graph showing the activity of Replagal^{®} α-GAL and plant recombinant human α-GAL-I (prh-alpha-GAL-I), and plant recombinant human α-GAL-I cross-linked with bis-NHS-PEG₄₅ (prh-alpha-GAL-I-CL45) in the hearts of Fabry mice 2 hours, 24 hours, 3 days and 7 days following injection of α-GAL (endogenous wild type α-GAL (WT) is shown as a standard);
FIG. 21 is a graph showing the activity of Replagal^{®} α-GAL and plant recombinant human α-GAL-I (prh-alpha-GAL-I), and plant recombinant human α-GAL-I cross-linked with bis-NHS-PEG₄₅ (prh-alpha-GAL-I-CL45) in the kidneys of Fabry mice 2 hours, 24 hours, 3 days and 7 days following injection of α-GAL (endogenous wild type α-GAL (WT) is shown as a standard);

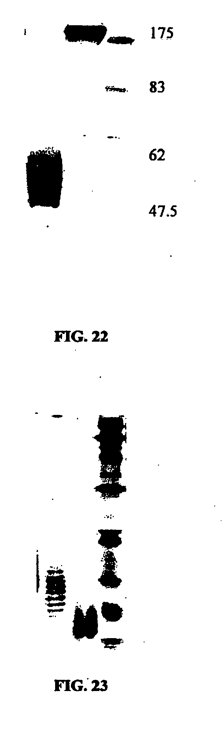

FIG. 22 presents a photograph of an image of an SDS-PAGE gel showing Replagal^{®} mammalian recombinant human α-GAL (left lane), and Replagal^{®} mammalian recombinant human α-GAL which was reacted with bis-NHS-PEG₄₅ (middle lane), as well as molecular weight markers (right lane; molecular weights of markers are indicated in KDa units);
FIG. 23 presents a photograph of an isoelectric focusing gel showing Replagal^{®} mammalian recombinant human α-GAL (left lane), and Replagal^{®} mammalian recombinant human α-GAL which was reacted with bis-NHS-PEG₄₅ (middle lane), as well as pH markers (right lane);

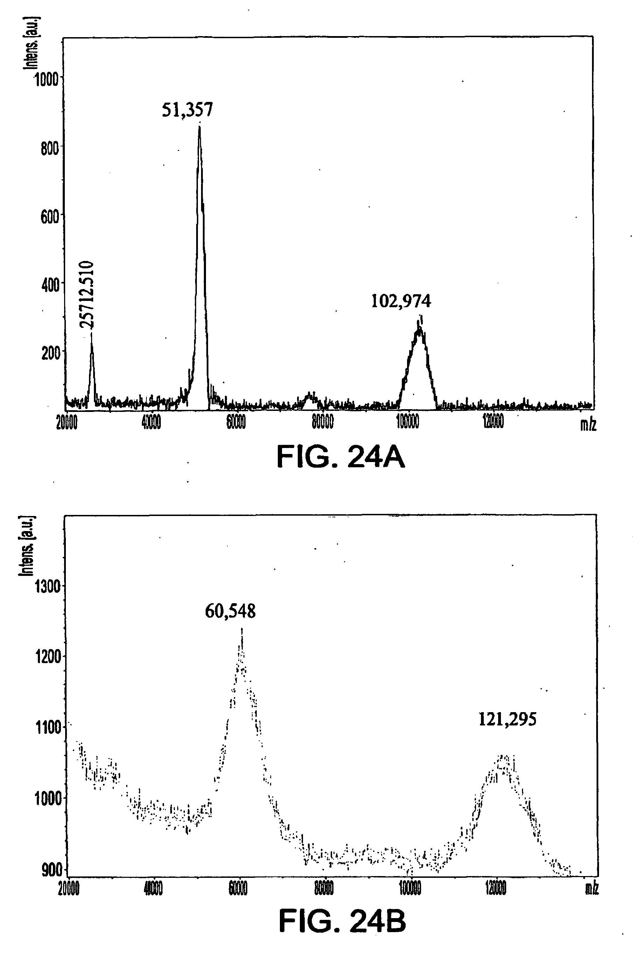

FIGs. 24A and 24B are MALDI-TOF mass spectroscopy spectra of Replagal^{®} mammalian recombinant human α-GAL (FIG. 24A), and Replagal^{®} mammalian recombinant human α-GAL cross-linked by bis-NHS-PEG₄₅ (x-axis indicates m/z values, and m/z values (in Da units) of peaks are shown);

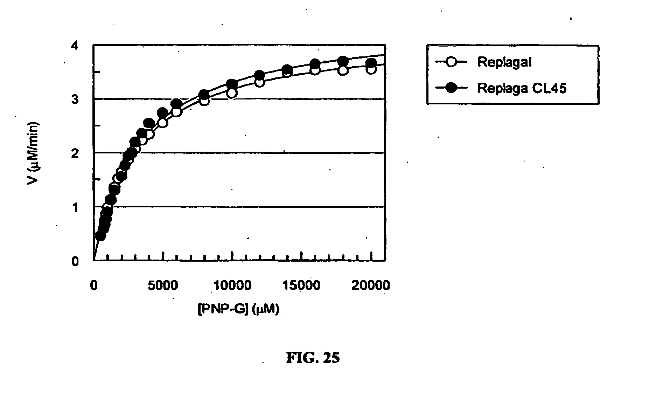

FIG. 25 is a Michaelis-Menten plot showing the velocity (V) of hydrolysis of *p-*nitrophenyl-α-*D*-galactopyranoside (*p*NP-G) by Replagal^{®} mammalian recombinant human α-GAL (Replagal) and Replagal^{®} mammalian recombinant human α-GAL cross-linked by bis-NHS-PEG₄₅ (Replagal CL45), as a function of *p*NP-G concentration;

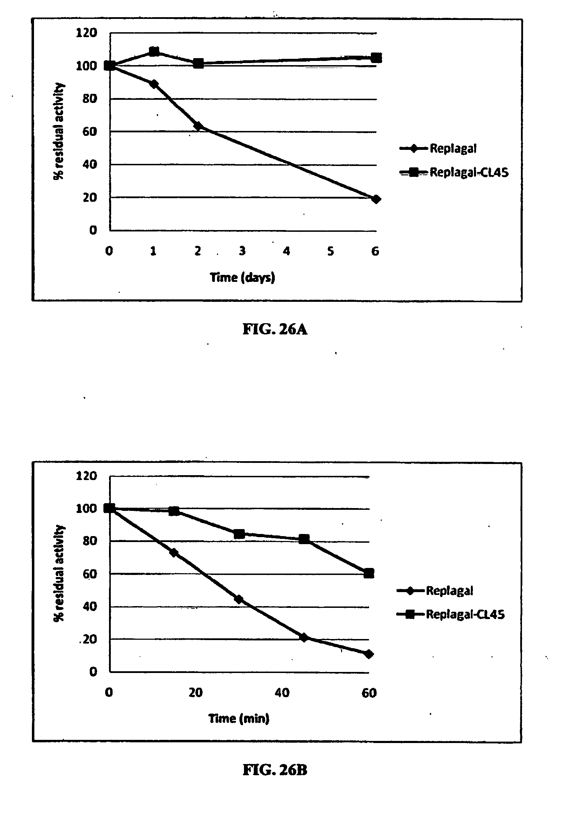

FIGs. 26A and 26B are graphs showing the activity of Replagal^{®} mammalian recombinant human α-GAL (Replagal) and Replagal® mammalian recombinant human α-GAL cross-linked by bis-NHS-PEG₄₅ (Replagal-CL45) as a function of incubation time under simulated lysosomal conditions (citrate phosphate buffer, pH 4.6, 37 °C) (FIG. 26A) or in human plasma at 37 °C (FIG. 26B);

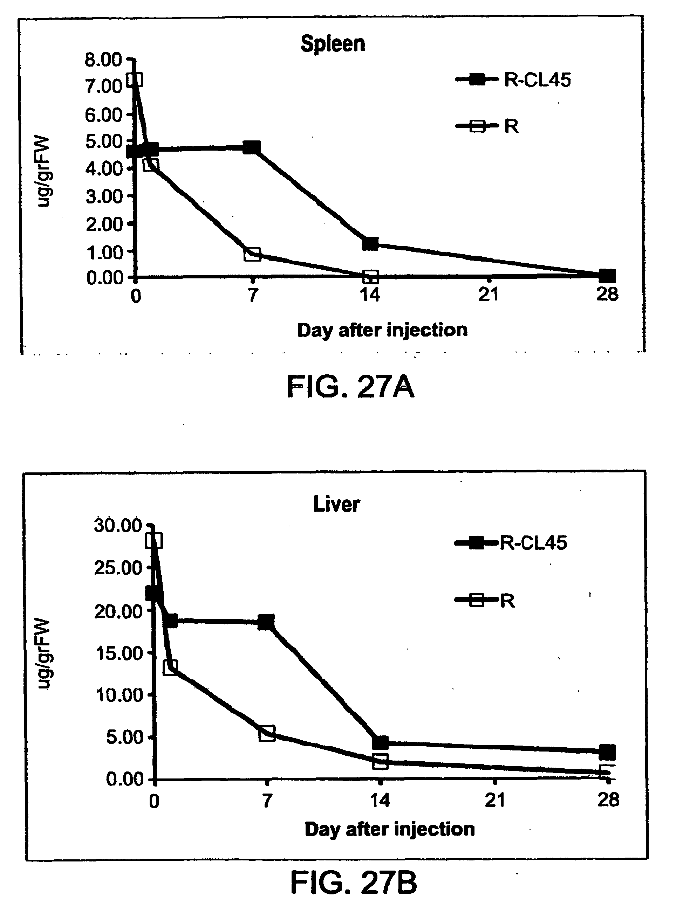

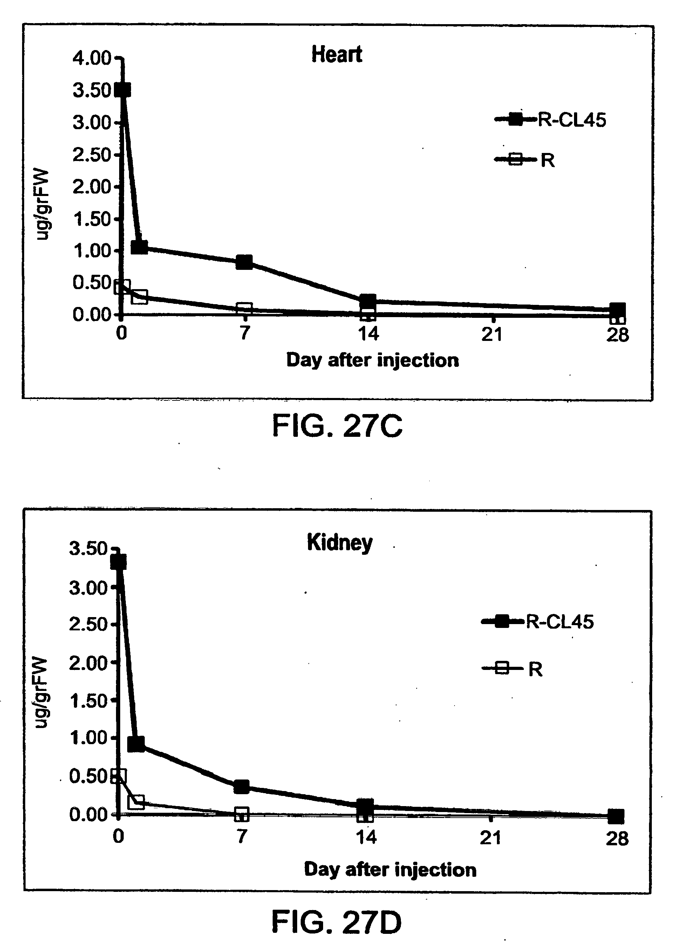

FIGs. 27A-27D are graphs showing the activity of Replagal^{®} α-GAL (R) and Replagal^{®} α-GAL cross-linked with bis-NHS-PEG₄₅ (R-CL45) in the spleens (FIG. 27A), livers (FIG. 27B), hearts (FIG. 27C) and kidneys (FIG. 27D) of Fabry mice 2 hours following injection of α-GAL;

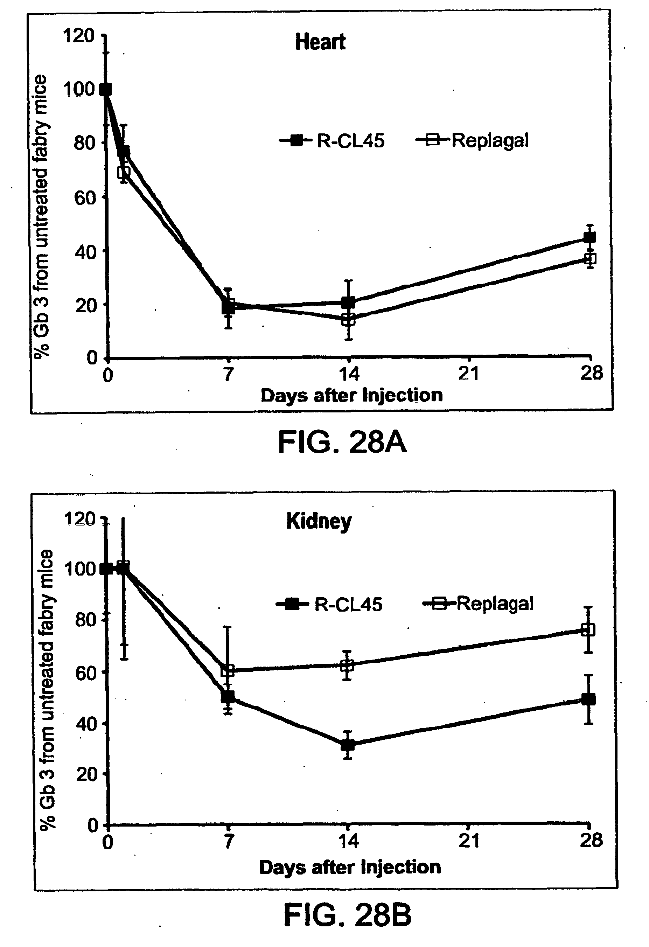

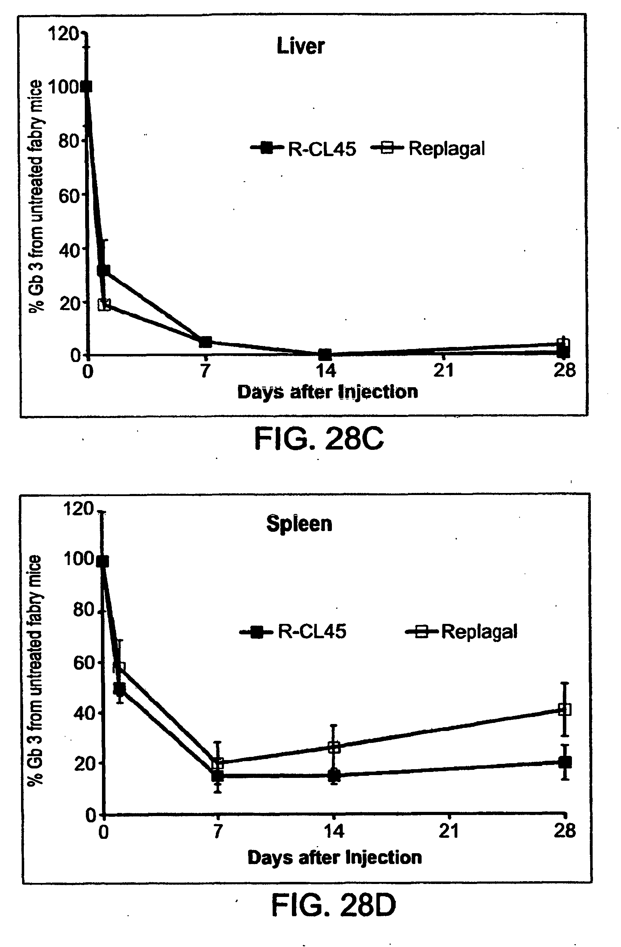

FIGs. 28A-28D are graphs showing Gb₃ levels in the hearts (FIG. 28A), kidneys (FIG. 28B), livers (FIG. 28C) and spleens (FIG. 28D) of Fabry mice, as a function of time following injection of Replagal^{®} α-GAL (R) or Replagal^{®} α-GAL cross-linked with bis-NHS-PEG₄₅ (R-CL45);

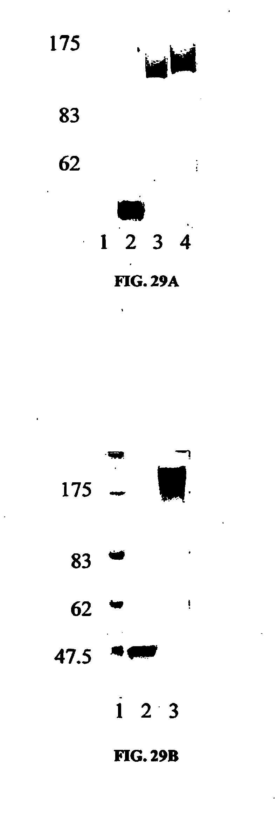

FIGs. 29A and 29B present scans of SDS-PAGE gels showing plant recombinant human α-GAL-II (FIGs. 29A and 29B, lane 2), and plant recombinant human α-GAL-II which was reacted with bis-NHS-PEG₂₁ (FIG. 29A, lane 3), bis-NHS-PEG₄₅ (FIG. 29A, lane 4) or bis-NHS-PEG₆₈ (FIG. 29B, lane 3), as well as molecular weight markers (FIGs. 29A and 29B, lane 1; molecular weights of markers are indicated in KDa units);

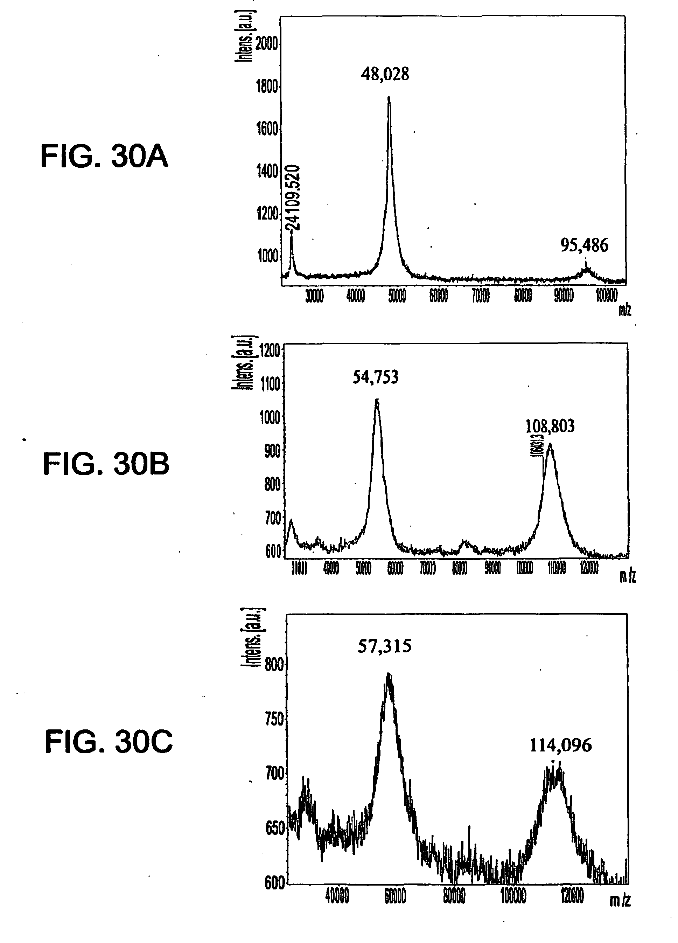

FIGs. 30A-30C are MALDI-TOF mass spectroscopy spectra of plant recombinant human α-GAL-II (FIG. 30A), and plant recombinant human α-GAL-II cross-linked by bis-NHS-PEG₂₁ (FIG. 30B) or bis-NHS-PEG₄₅ (FIG. 30C) (x-axis indicates m/z values, and m/z values (in Da units) of peaks are shown);

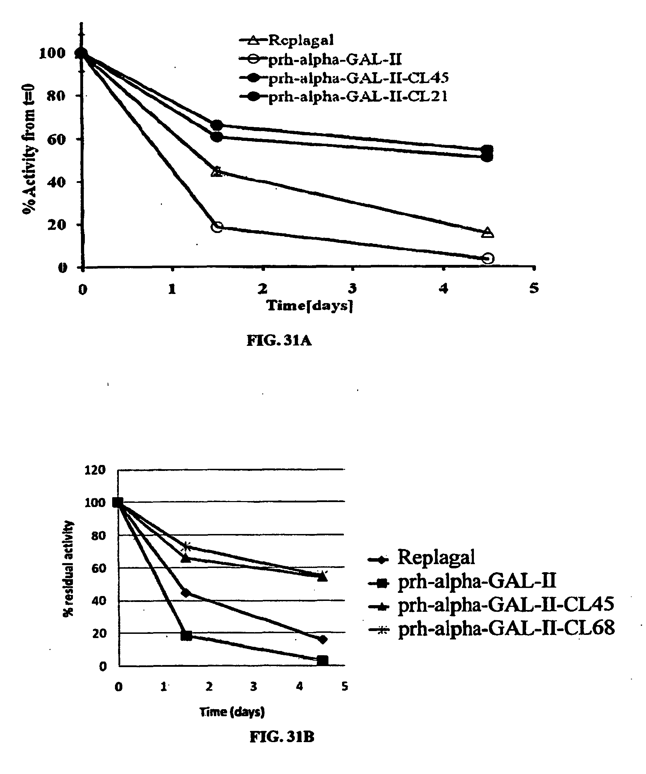

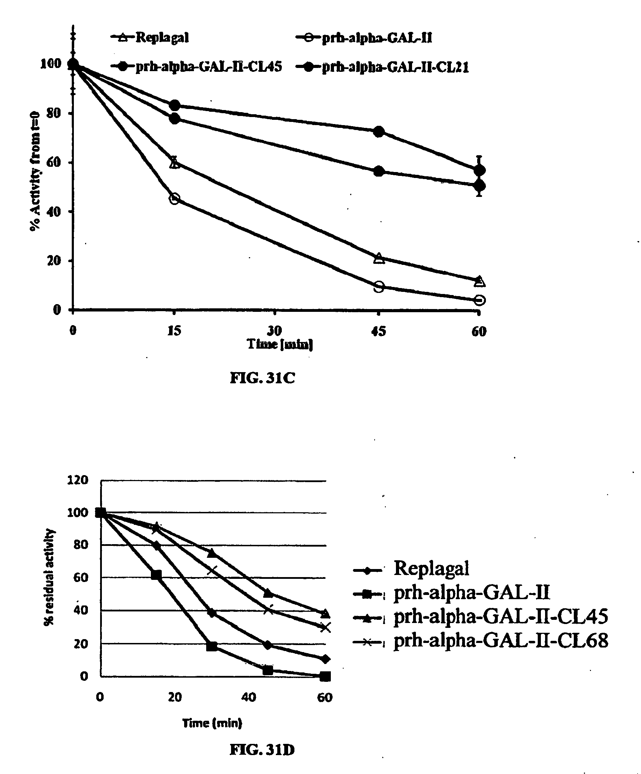

FIGs. 31A-31D are graphs showing the activity of Replagal^{®} mammalian recombinant human α-GAL (Replagal), plant recombinant human α-GAL-II (prh-alpha-GAL-II) and plant recombinant human α-GAL-II cross-linked by bis-NHS-PEG₂₁ (prh-alpha-GAL-II-CL21; FIGs. 31A and 31C), bis-NHS-PEG₄₅ (prh-alpha-GAL-II-CL45; FIGs. 31A-31D) or bis-NHS-PEG₆₈ (prh-alpha-GAL-II-CL68; FIGs. 31B and 31D) as a function of incubation time under simulated lysosomal conditions (citrate phosphate buffer, pH 4.6, 37 °C) (FIGs. 31A and 31B) of in human plasma at 37 °C (FIGs. 31C and 31D) (data shown in FIGs. 31C and 31D are from different experiments);

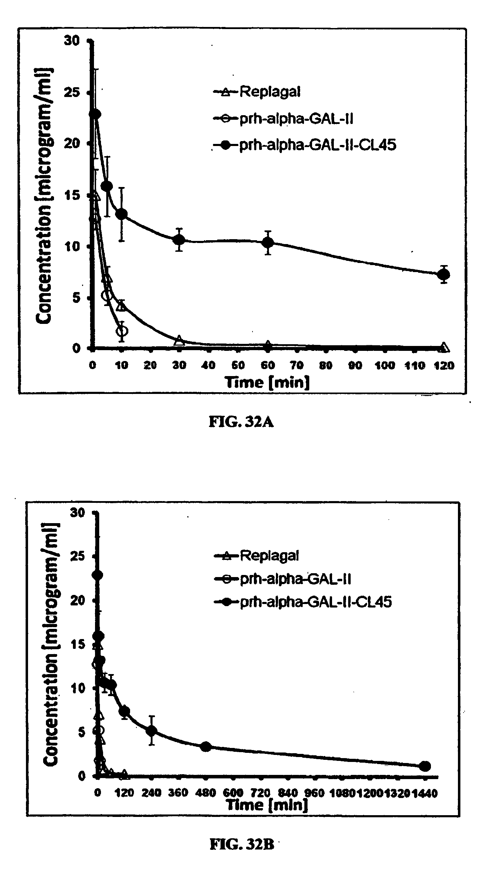

FIGs. 32A and 32B are graphs showing the pharmacokinetic profiles of Replagal^{®} α-GAL (Replagal), plant recombinant human α-GAL-II (prh-alpha-GAL-II), and plant recombinant human α-GAL-II cross-linked with bis-NHS-PEG₄₅ (prh-alpha-GAL-II-CL45) in the plasma of Fabry mice; concentration of each α-GAL is presented as a function of time following injection of α-GAL (FIGs. 32A and 32B present the same data at different time frames);

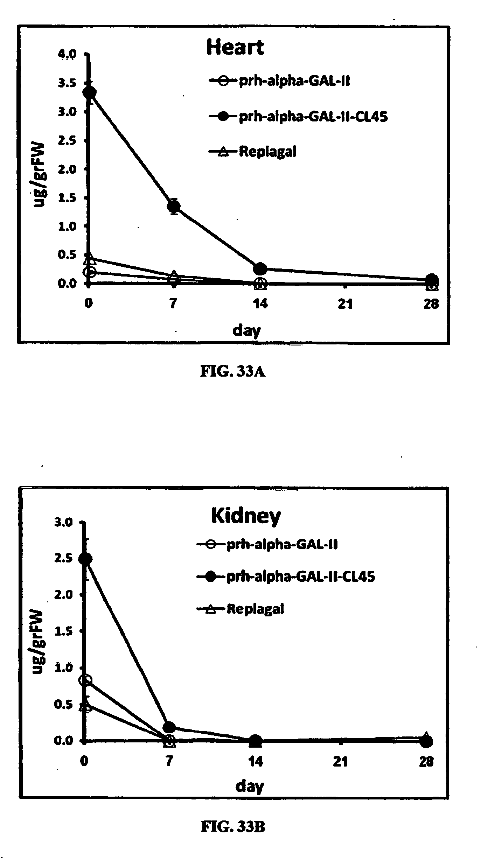

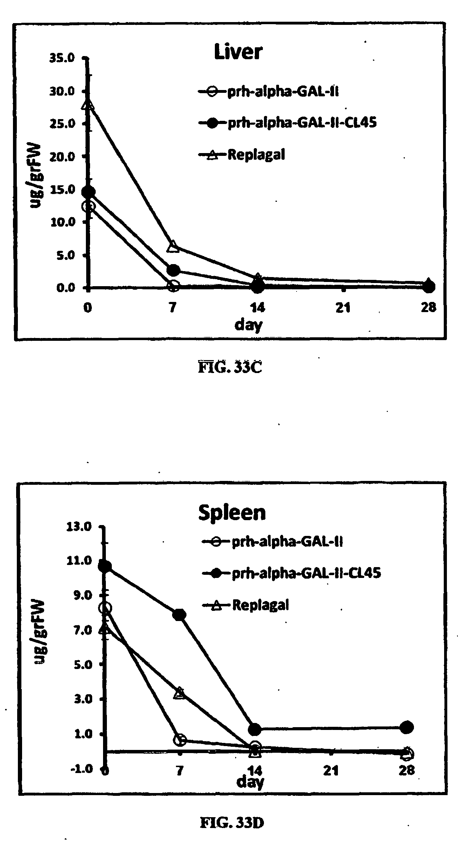

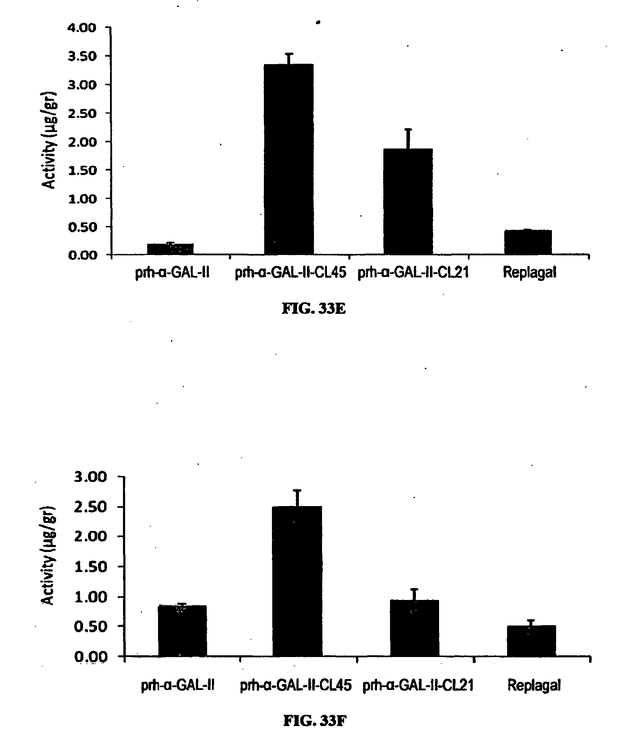

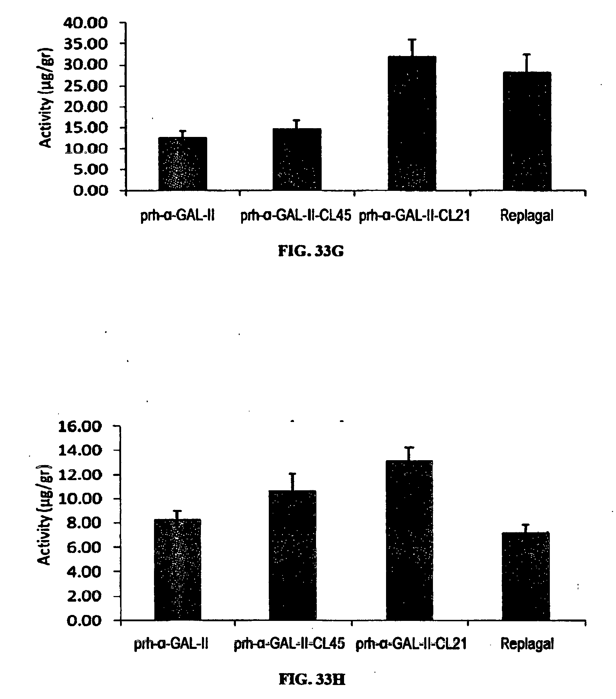

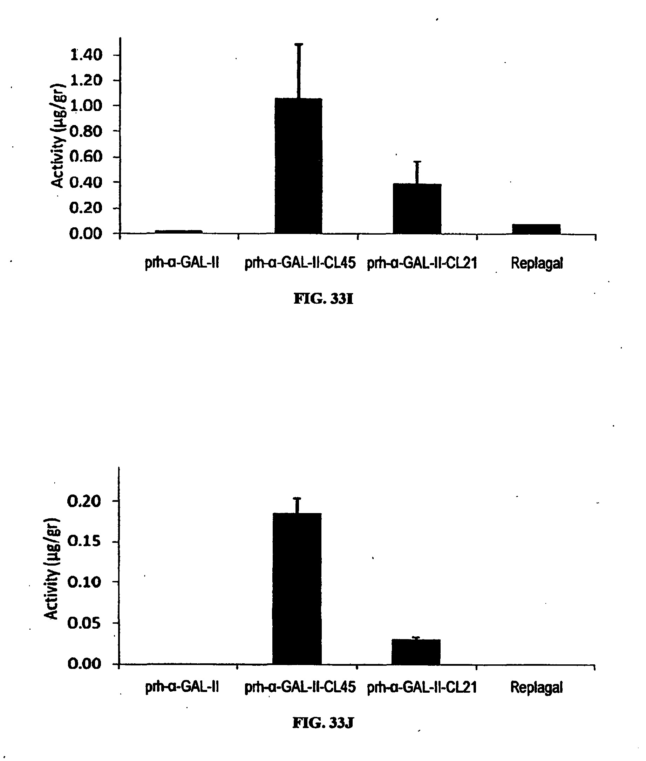

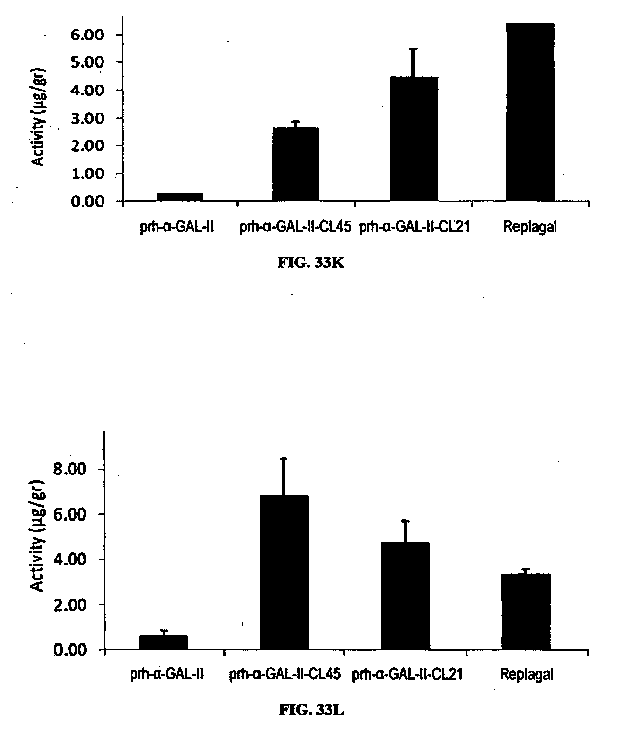

FIGs. 33A-33L are graphs showing the activity of Replagal^{®} α-GAL (Replagal), plant recombinant human α-GAL-II (prh-alpha-GAL-II) and plant recombinant human α-GAL-II cross-linked with bis-NHS-PEG₄₅ (prh-alpha-GAL-II-CL45; FIGs. 33A-33L) or bis-NHS-PEG₂₁ (prh-alpha-GAL-II-CL21; FIGs. 33E-33L) in the hearts (FIGs. 33A, 33E and 33I), kidneys (FIGs. 33B, 33F and 33J), livers (FIGs. 33C, 33G and 33K) and spleens (FIGs. 33D, 33H and 33L) of Fabry mice 2 hours (FIGs. 33A-33H), 7 days (FIGs. 33A-33D and 33I-33L), 14 days (FIGs. 33A-33D) and 28 days (FIGs. 33A-33D) following injection of α-GAL;

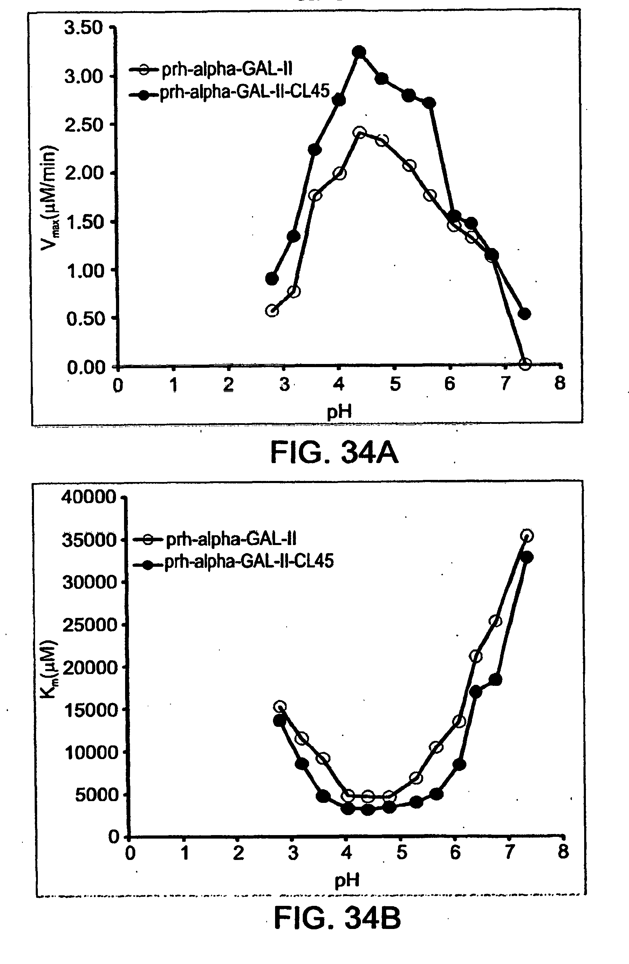

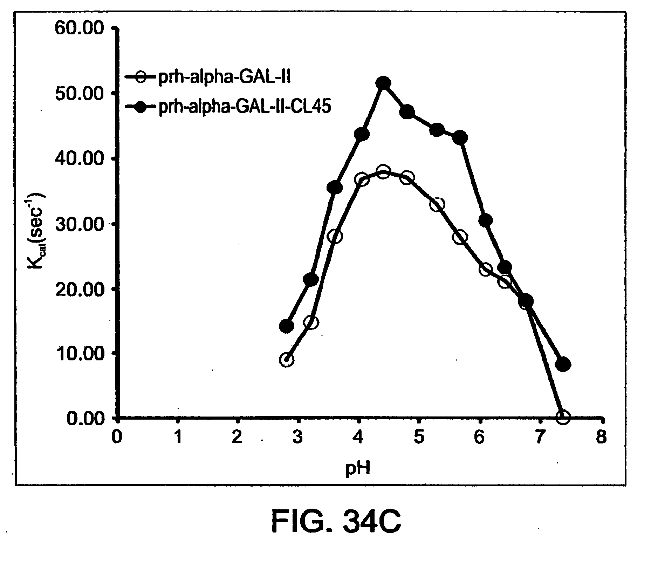

FIGs. 34A-34C are graphs showing the kinetic parameters *V*ₘₐₓ (FIG. 34A), *K*_{M} (FIG. 34B) and *k*_{cat} (FIG. 34C) for plant recombinant human α-GAL-II (prh-alpha-GAL-II) and plant recombinant human α-GAL-II cross-linked with bis-NHS-PEG₄₅ (prh-alpha-GAL-II-CL45), as a function of pH;

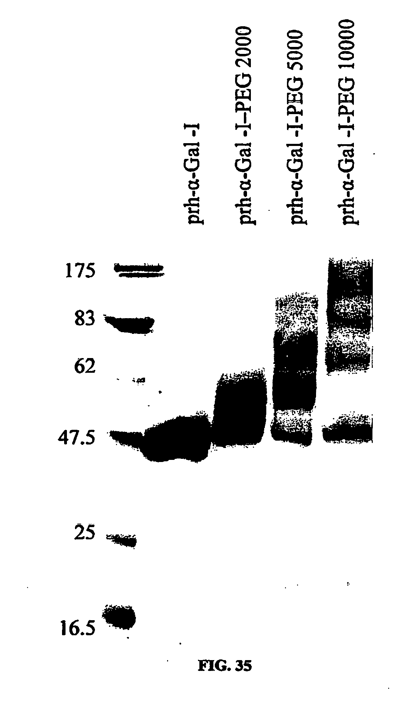

FIG. 35 presents a scan of an SDS-PAGE gel showing plant recombinant human α-GAL-I (prh-α-Gal-I), and plant recombinant human α-GAL-I which was reacted with methoxy-capped NHS-PEG having a molecular weight of 2 KDa (prh-α-Gal-I-PEG 2000), 5 KDa (prh-α-Gal-I-PEG 5000) or 10 KDa (prh-α-Gal-I-PEG 10000), as well as molecular weight markers (left lane; molecular weights of markers are indicated in KDa units);

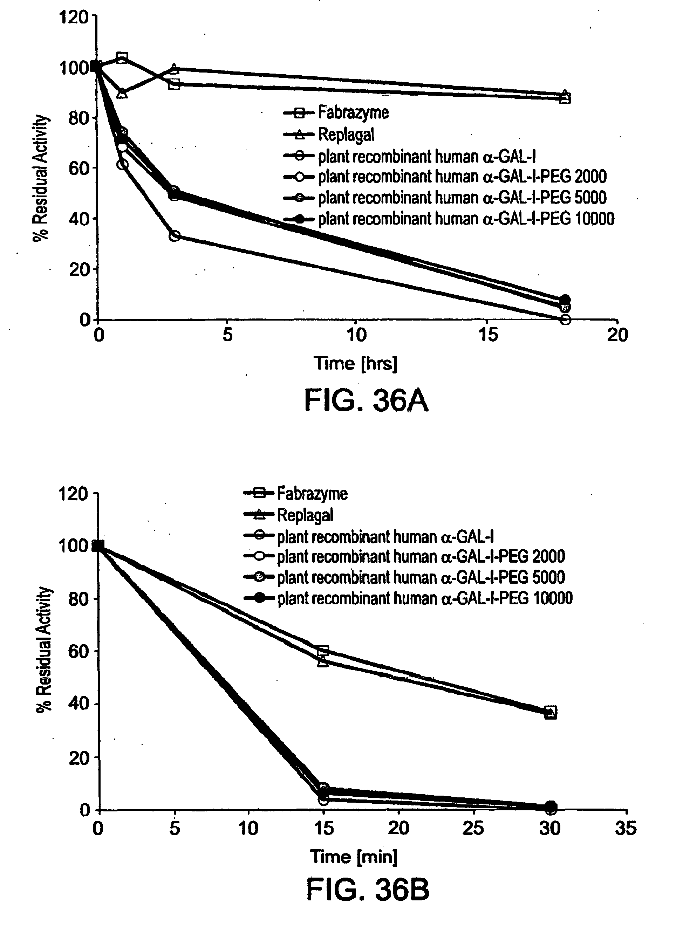

FIGs. 36A and 36B are graphs showing the activity of Fabrazyme^{®} mammalian recombinant human α-GAL (Fabrazyme), Replagal^{®} mammalian recombinant human α-GAL (Replagal), plant recombinant human α-GAL-I and plant recombinant human α-GAL-I which was reacted with methoxy-capped NHS-PEG having a molecular weight of 2 KDa (α-Gal-I-PEG 2000), 5 KDa (α-Gal-I-PEG 5000) or 10 KDa (α-Gal-I-PEG 10000), as a function of incubation time under simulated lysosomal conditions (citrate phosphate buffer, pH 4.6, 37°C) (FIG. 36A) or in human plasma at 37°C (FIG. 36B);

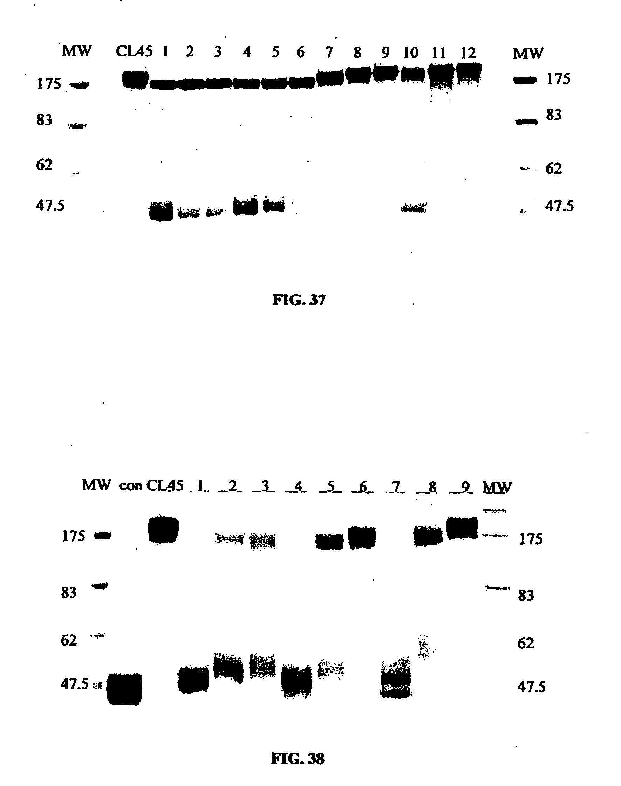

FIG. 37 presents a scan of an SDS-PAGE gel showing plant recombinant α-GAL-I which was reacted with bis-NHS-PEG₂ (lanes 1-3), bis-NHS-PEG₄ (lanes 4-6), bis-NHS-PEG₆₈ (lanes 7-9), bis-NHS-PEG₁₅₀ (lanes 10-12) and bis-NHS-PEG₄₅ (CL45), at a molar ratio of 50:1 (lanes 1, 4, 7 and 10), 100:1 (lanes 2, 5, 8 and 11) and 200:1 (lanes 3, 6, 9 and 12) bis-NHS-PEG:α-GAL, as well as molecular weight markers (MW);
FIG. 38 presents a scan of an SDS-PAGE gel showing plant recombinant α-GAL-I which was reacted with bis-COOH-PEG₁₂ (lanes 1-3), bis-COOH-PEG₂₈ (lanes 4-6), bis-COOH-PEG₄₅ (lanes 7-9), and bis-NHS-PEG₄₅ (CL45), at a molar ratio of 50:1 (lanes 1, 4 and 7), 100:1 (lanes 2, 5 and 8) and 200:1 (lanes 3, 6 and 9) bis-NHS-PEG:α-GAL, as well as molecular weight markers (MW), and non-crosslinked plant recombinant α-GAL-I as a control (con);

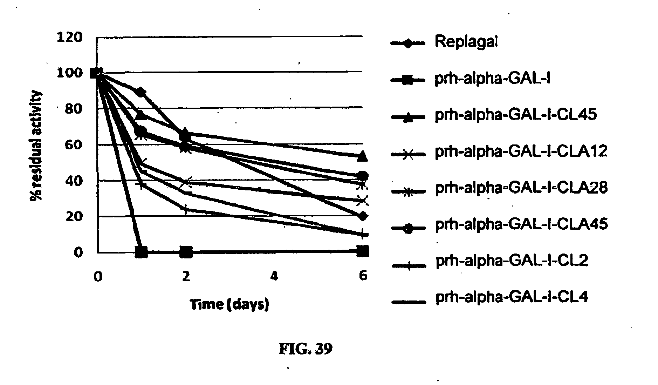

FIG. 39 is a graph showing the activity of Replagal^{®} α-GAL, plant recombinant human α-GAL-I (prh-a-GAL-I), and plant recombinant human **-**GAL-I cross-linked with bis-NHS-PEG₄₅ (prh-α-GAL-I-CLA5), bis-NHS-PEG₄ (prh-α-GAL-I-CL4), bis-NHS-PEG₂ (prh-α-GAL-I-CL2), bis-COOH-PEG₄₅ (prh-α-GAL-I-CLA45) bis-COOH-PEG₂₈ (prh-α-GAL-I-CLA28) or bis-COOH-PEG₁₂ (prh-α-GAL-I-CLA12) as a function of incubation time under simulated lysosomal conditions (citrate phosphate buffer, pH 4.6, 37 °C);

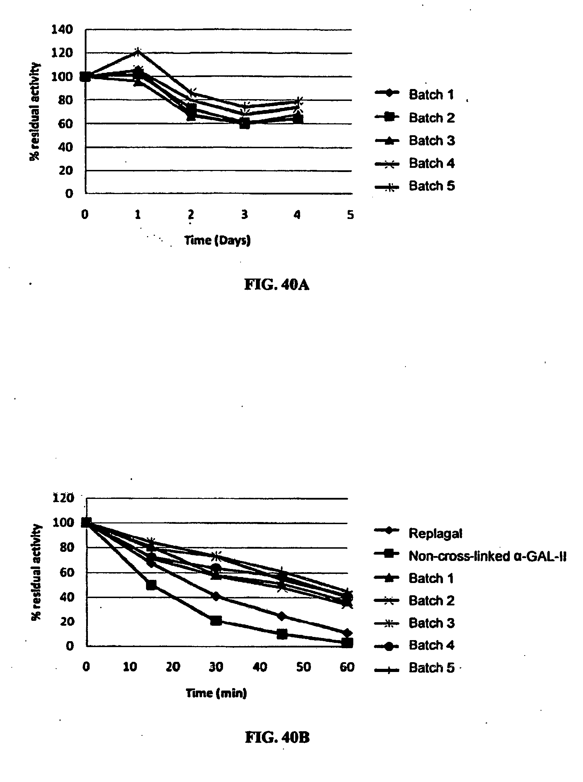

FIGs. 40A and 40B are graphs showing the activity of plant recombinant human α-GAL-II cross-linked by bis-NHS-PEG₄₅ as a function of incubation time under simulated lysosomal conditions (citrate phosphate buffer, pH 4.6, 37 °C) (FIG. 40A) or in human plasma at 37 °C (FIG. 40B) (FIG. 40B shows the activity of Replagal^{®} mammalian recombinant α-GAL and non-cross-linked plant recombinant human α-GAL-II for comparison);

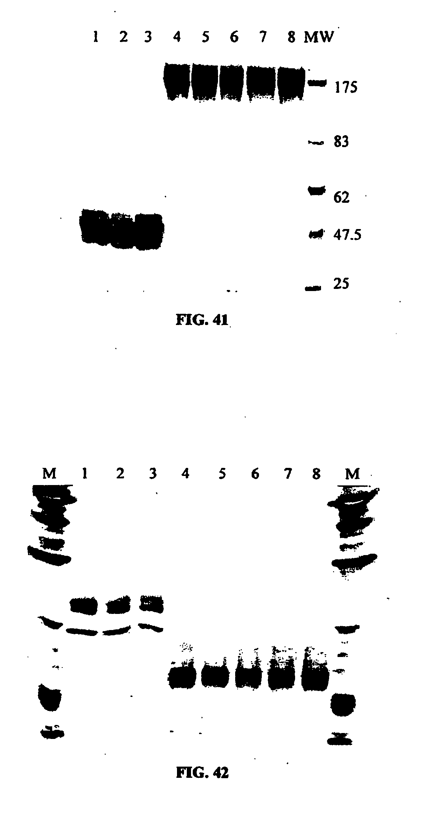

FIG. 41 presents a scan of an SDS-PAGE gel showing plant recombinant α-GAL-II from 3 different batches (lanes 1-3) and plant recombinant α-GAL-II which was reacted with bis-NHS-PEG₄₅ from 5 different batches (lanes 4-8), as well as molecular weight markers (MW);
FIG. 42 presents a scan of an isoelectric focusing gel showing plant recombinant α-GAL-II from 3 different batches (lanes 1-3) and plant recombinant α-GAL-II which was reacted with bis-NHS-PEG₄₅ from 5 different batches (lanes 4-8), as well as pH markers (M);

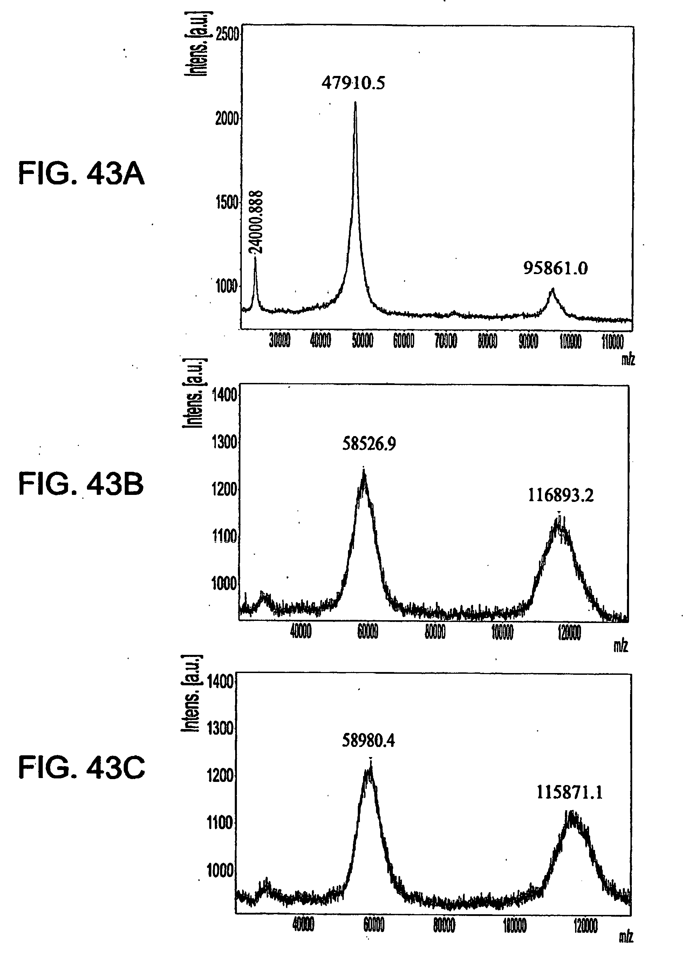

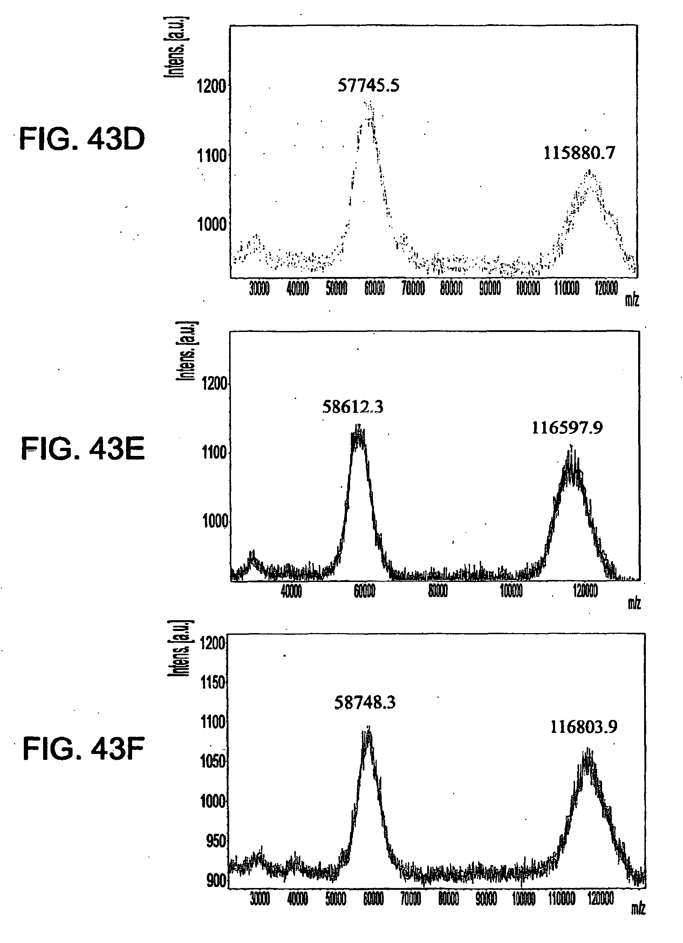

FIGs. 43A-43F are MALDI-TOF mass spectroscopy spectra of plant recombinant human α-GAL-II (FIG. 43A), and plant human α-GAL-II cross-linked by bis-NHS-PEG₄₅ from 5 different batches (FIGs. 43B-43F, respectively) (x-axis indicates m/z values, and m/z values (in Da units) of peaks are shown); and

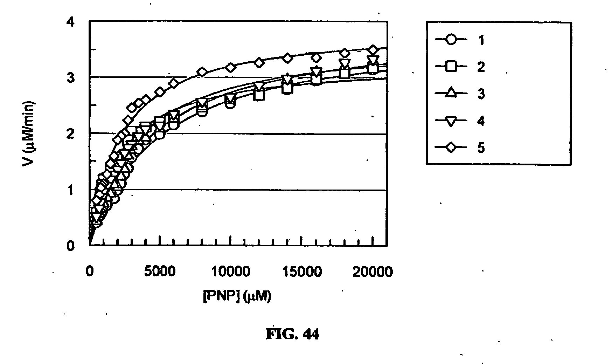

FIG. 44 is a graph showing the catalytic velocity (V) of α-GAL activity exhibited by plant human α-GAL-II cross-linked by bis-NHS-PEG₄₅ from 5 different batches, as a function of substrate (*p*-nitrophenyl-α-D-galactopyranoside) concentration.

### DESCRIPTION OF SPECIFIC EMBODIMENTS OF THE INVENTION

The present invention, in some embodiments thereof, relates to novel multimeric protein structures and, more particularly, but not exclusively, to multimeric protein structures of α-galactosidase and to uses thereof in treating Fabry disease.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Deficiencies of a lysosomal protein (e.g., defects in a lysosomal protein or absence of a lysosomal protein) can cause considerable harm to the health of a subject (a lysosomal storage disease). Enzyme replacement therapy (ERT), in which the deficient protein is administered to a patient, has been used in attempts to treat lysosomal storage diseases. However, administration of the deficient protein does not necessarily result in a considerable and/or persistent increase in the activity of the protein *in vivo.*

Fabry disease is an example of an X-linked recessive (inherited) lysosomal storage disease which can cause a wide range of systemic symptoms. A deficiency of the lysosomal enzyme α-galactosidase A due to mutation causes a glycolipid known as globotriaosylceramide (also known as Gb₃ or ceramide trihexoside) to accumulate within the blood vessels, other tissues, and organs. This accumulation leads to an impairment of their proper function. Two enzyme replacement therapies (ERTs) are available to functionally compensate for α-galactosidase deficiency. Agalsidase alpha (Replagal®, Shire) and agalsidase beta (Fabrazyme®, Genzyme) are both recombinant forms of the human α-galactosidase A enzyme. These enzymes are difficult to manufacture and as such are expensive. Recently, contamination at Genzyme's Allston, MA, plant caused a worldwide shortage of agalsidase beta, and supplies were rationed to patients at one-third the recommended dose.

As shown herein, α-galactosidases exert their maximal activity at low pH levels characteristic of lysosomes, while their activity at higher pH levels is compromised. Thus, for example, α-galactosidase used in ERT would have little ability to hydrolyze terminal galactosylated glycolipids in the serum of Fabry patients.

Moreover, as further shown herein, even under lysosomal conditions, the activity of α-galactosidases is gradually compromised, although at a slower rate than at higher pH levels.

Motivated by a need to solve the compromised activity of α-galactosidases, the present inventors have searched for stabilized forms of α-galactosidase (α-GAL). More specifically, the present inventors have envisioned that a stabilized form of α-galactosidase would exhibit longer lasting activity in general, including longer lasting activity in serum. The present inventors have thus designed and successfully prepared and practiced stabilized forms of native α-galactosidase and have indeed shown that such stabilized forms exhibit an improved performance, in terms of enhanced activity and/or a longer lasting activity under both lysosomal conditions and in a serum environment, which allows for an enhanced activity of the protein *in vivo.*

The present inventors have demonstrated a formation of stabilized forms of α-galactosidase which exhibit an improved performance by means of cross-linking native α-galactosidase, via formation of new covalent linkage between α-galactosidase monomers.

Referring now to the drawings, Figures 1 and 4 show the decline of enzyme activity under lysosomal conditions for plant recombinant human α-GAL I (prh-α-GAL I) and Fabrazyme^{®} and Replagal^{®} α-GAL. Figures 2 and 3 show the decline of enzyme activity under simulated physiological conditions or in human plasma, for the same α-GAL varieties. Figures 2 and 4 show that galactose decreases the rate of the decline in α-GAL activity.

Figure 5 shows exemplary PEG (polyethylene glycol) cross-linking agents, according to optional embodiments of the invention. Figure 6 depicts a cross-linked α-GAL dimer according to optional embodiments of the invention.

Figures 7-10 and 37 show that prh-α-GAL-I reacted with exemplary cross-linking agents comprising N-hydroxysuccinimide moieties. Figure 38 shows that prh-α-GAL-I reacted with exemplary cross-linking agents comprising carboxyl groups, following *in situ* activation with N-hydroxysuccinimide. Figures 7, 37 and 38 show that the reaction with the cross-linking agent resulted in α-GAL appearing primarily in a dimeric form rather than a monomeric form under denaturing conditions, indicating that the quaternary structure of the α-GAL was maintained by covalent cross-linking. Figure 11 shows that the cross-linked α-GAL retained its enzymatic activity.

Figures 12A-12C and 39 show that the cross-linked prh α-GAL-I exhibits a longer lasting activity than non-cross-linked α-GAL under simulated lysosomal conditions. The increase in stability is stronger for PEG₂₈ and PEG₄₅ linkers than for shorter PEG linkers. Figure 13 shows that the cross-linked prh-α-GAL-I exhibits a longer lasting activity than non-cross-linked α-GAL in plasma *in vivo.* Figures 14A-21 show that cross-linked prh-α-GAL-I exhibits an enhanced activity *in vivo* in the spleen, liver, heart and kidneys. The enhancement of α-GAL activity is stronger for PEG₄₅ linkers than for shorter PEG linkers. Figures 15A, 15B and 19 show that although cross-linked prh-α-GAL-I exhibits an enhanced activity *in vivo,* the enhanced activity is not as concentrated in the liver as is Replagal^{®} α-GAL activity.

The above results indicate that cross-linking plant recombinant human α-GAL-I results in a dimer with improved stability, which allows for a more effective increase of α-GAL activity when administered *in vivo.*

Similarly, Figures 22-28D show that cross-linking mammalian recombinant human α-GAL results in a covalently-linked dimer (Figures 22-24B), which exhibits normal enzymatic activity (Figure 25), as well as longer lasting activity under both lysosomal conditions and in plama (Figures 26A-26B), and enhanced activity *in vivo* in the spleen, liver, heart and kidneys (Figures 27A-28D).

Similarly, Figures 29A-33L show that cross-linking plant recombinant human α-GAL II results in a covalently-linked dimer (Figures 29-30), which exhibits longer lasting activity under both lysosomal conditions and in plama (Figures 31A-31B), and enhanced activity *in vivo* in plasma and in the spleen, liver, heart and kidneys (Figures 32A-33L). As shown in FIGs. 33A-33L, cross-linking with a PEG₄₅ linker was particularly effective at enhancing *in vivo* activity.

These results indicate that the advantageous effects of cross-linking are applicable to a variety of α-GAL proteins.

Figures 34A-34C show that cross-linking α-GAL enhances parameters of α-GAL enzymatic catalysis, broadens the pH range for α-GAL activity, and allows for α-GAL activity at a pH of about 7 or more.

Figures 35-36B show that PEGylation without cross-linking has no significant effect on α-GAL activity, indicating that the advantageous effects of cross-linking are specifically due to cross-linking, rather than to an effect of PEGylation.

Figures 40-44 show that cross-linking of α-GAL according to embodiments of the invention allows for good reproducibility of the stability (Figures 40A-40B), degree of covalent cross-linking (Figures 41-43F) and enzymatic properties (Figure 44) of the cross-linked α-GAL.

The results presented herein show that covalently cross-linked, multimeric protein structures of α-galactosidase are characterized by a higher stability and enhanced activity under physiologically relevant conditions, as compared to the native forms of α-galactosidase.

Thus, the covalently-linked multimeric protein structure may exhibit an activity which is higher than an activity of a native form of α-galactosidase, as a result of the activity of the native form decaying more rapidly over time than the activity of the cross-linked multimeric protein structure, which is stabilized by the covalent cross-linking.

The covalently cross-linked multimeric protein structure may exhibit an activity which is higher than an activity of a native form of α-galactosidase, also due to a higher initial activity (e.g., due to different parameters of activity), i.e., independently of any decay of activity over time.

Hence, according to an aspect of some embodiments of the present invention there is provided a multimeric protein structure comprising at least two α-galactosidase monomers being covalently linked to one another via a linking moiety. According to some embodiments, the multimeric protein structure features a stability higher than that of native α-galactosidase and/or an initial activity higher than that of native α-galactosidase, as described in detail below.

Herein, the term "monomer," with respect to α-galactosidase refers to an individual polypeptide of α-galactosidase. The polypeptide may include non-peptidic substituents (e.g., one or more saccharide moieties).

Herein, the term "native" with respect to α-galactosidase encompasses proteins comprising an amino acid sequence substantially identical (i.e., at least 95 % homology, optionally at least 99 % homology, and optionally 100 %) to an amino acid sequence of a naturally occurring α-galactosidase protein. A native α-galactosidase may be a protein isolated from a natural source, or a recombinantly produced protein (e.g., derived from mammalian cells, plant cells, yeast cells, bacterial cells, insect cells).

The term "native", when used in reference to a quaternary structure of α-galactosidase (e.g., an α-galactosidase dimer), further comprises a quaternary structure substantially identical to that of a naturally occurring protein.

Herein, the phrase "naturally occurring protein" refers to a protein in a form which occurs in nature (e.g., in an organism), with respect to the protein's amino acid sequence, as well as the protein's quaternary structure if the protein is in a multimeric form.

Post-translational modifications (e.g., glycosylation) of naturally occurring α-galactosidase proteins (e.g., in an organism which expresses the naturally occurring α-galactosidase protein) may be present, absent or modified in the native form of α-galactosidase referred to herein. A native form of α-galactosidase (e.g., a recombinantly produced α-galactosidase) may optionally comprise different post-translational modifications than those of the naturally occurring α-galactosidase, provided that the native form of the α-galactosidase retains a substantially similar amino acid sequence and structure as the naturally occurring α-galactosidase, as described hereinabove.

Herein, the native form of a protein may refer to a monomeric structure (e.g., an α-galactosidase monomer) and/or a multimeric structure (e.g., an α-galactosidase dimer). For example, a dimeric protein can be described as a native form of α-galactosidase, and a monomeric polypeptide in a dimeric protein can be described as a native form of the α-galactosidase monomer.

Optionally, the multimeric protein structure described herein is a dimeric structure, as is the native form of α-galactosidase.

Alternatively, the multimeric protein structure comprises more than two α-galactosidase monomers. For example, the multimeric protein structure may be a tetramer, a hexamer, or an octamer comprised of α-galactosidase monomers.

The multimeric protein structures described herein comprise covalent bonds which link the α-galactosidase monomers therein, and which are absent from the native form of the α-galactosidase.

Optionally, the linking moiety which links the α-galactosidase monomers is a moiety which is not present in a native form of α-galactosidase (e.g., a synthetic linking moiety).

Thus, for example, the linking moiety is optionally a moiety which is covalently attached to a side chain, an N-terminus or a C-terminus, or a moiety related to post-translational modifications (e.g., a saccharide moiety) of an α-galactosidase monomer, as well as to a side chain, an N-terminus or a C-terminus, or a moiety related to post-translational modifications (e.g., a saccharide moiety) of another α-galactosidase monomer. Exemplary such linking moieties are described in detail hereinunder.

Alternatively, the linking moiety forms a part of the α-galactosidase monomers being linked (e.g., a part of a side chain, N-terminus or C-terminus, or a moiety related to post-translational modifications (e.g., a saccharide moiety) of an α-galactosidase monomer, as well as of a side chain, an N-terminus or a C-terminus, or a moiety related to post-translational modifications (e.g., a saccharide moiety) of another α-galactosidase monomer).

Thus, for example, the linking moiety can be a covalent bond (e.g., an amide bond) between a functional group of a side chain, N-terminus, C-terminus or moiety related to post-translational modifications of a monomer (e.g., an amine), and a complementary functional group of a side chain, N-terminus, C-terminus or moiety related to post-translational modifications of another monomer (e.g., carboxyl), wherein such a covalent bond is absent from the native form of the α-galactosidase. Other covalent bonds, such as, for example, an ester bond (between a hydroxy group and a carboxyl); a thioester bond; an ether bond (between two hydroxy groups); a thioether bond; an anhydride bond (between two carboxyls); a thioamide bond; a carbamate or thiocarbamate bond, are also contemplated.

Optionally, the linking moiety is devoid of a disulfide bond. However, a linking moiety which includes a disulfide bond at a position which does not form a link between monomers (e.g., cleavage of the disulfide bond does not cleave the link between the monomers) is within the scope of this embodiment of the invention. A potential advantage of linking moiety devoid of a disulfide bond is that it is not susceptible to cleavage by mildly reducing conditions, as are disulfide bonds.

Optionally, the linking moiety is a non-peptidic moiety (e.g., the linking moiety does not consist of an amide bond, an amino acid, a dipeptide, a tripeptide, an oligopeptide or a polypeptide).

Alternatively, the linking moiety may be, or may comprise, a peptidic moiety (e.g., an amino acid, a dipeptide, a tripeptide, an oligopeptide or a polypeptide).

Optionally, the linking moiety is not merely a linear extension of any of the α-galactosidase monomers attached thereto (i.e., the N-terminus and C-terminus of the peptidic moiety is not attached directly to the C-terminus or N-terminus of any of the α-galactosidase monomers).

Alternatively, the linking moiety is formed by direct covalent attachment of an N-terminus of an α-galactosidase monomer with a C-terminus of another α-galactosidase monomer, so as to produce a fused polypeptide. Such a polypeptide will not be a native form of α-galactosidase, although it may comprise two α-galactosidase monomers essentially in their native form.

However, the covalent linking of α-galactosidase monomers described herein is preferably in a form other than direct linkage of an N-terminus to a C-terminus.

The linking moiety is also referred to herein as a cross-linking moiety. The linking of α-galactosidase monomers by a linking moiety is referred to herein as "cross-linking".

The cross-linking moiety can be a covalent bond, a chemical atom or group (e.g., a C(=O)-O- group, -O-, -S-, NR-, -N=N-, -NH-C(=O)-NH-, and the like) or a bridging moiety (composed of a chain of chemical groups).

A bridging moiety can be, for example, a polymeric or oligomeric group.

The bridging moiety is a multifunctional moiety (e.g., biradical, triradical, etc.) that is attached to side chains, moieties related to post-translational modifications (e.g., saccharide moieties) and/or termini (i.e., N-termini, C-termini) of two or more of the monomers.

As exemplified herein in the Examples section, relatively short linking moieties (e.g., PEG₂, PEG₄, PEG₅) may be less effective than longer linking moieties (e.g., PEG₂₈, PEG₄₅) at cross-linking between different α-galactosidase monomers.

Hence, according to some embodiments, the linking moiety is not a covalent bond, a chemical atom or group, but is rather a bridging moiety.

Hence, according to some embodiments, the linking moiety is at least 10 atoms long, optionally at least 20 atoms long, optionally at least 30 atoms long, optionally at least 50 atoms long, optionally at least 100 atoms long, and optionally at least 200 atoms long.

Herein, the length of a linking moiety (when expressed as a number of atoms) refers to length of the backbone of the linking moiety, i.e., the number atoms forming a linear chain between residues of each of two monomers linked via the linking moiety.

Optionally, the linking moiety is below a certain size, so as to avoid an unnecessarily excessive part of the linking moiety in the formed cross-linked protein, which may interfere with the function of the protein.

Hence, according to some embodiments, each linking moiety is characterized by a molecular weight of less than 20 KDa, optionally less than 10 KDa, optionally less than 5 KDa, and optionally less than 3 KDa.

In order to facilitate cross-linking, the linking moiety is optionally substantially flexible, wherein the bonds in the backbone of the linking moiety are mostly rotationally free, for example, single bonds which are not coupled to a double bond (e.g., unlike an amide bond) and wherein rotation is not sterically hindered. Optionally, at least 70 %, optionally at least 80 %, and optionally at least 90 % (e.g., 100 %) of the bonds in the backbone of the linking moiety are rotationally free.

In some embodiments, the linking moiety comprises a poly(alkylene glycol) chain.

The phrase "poly(alkylene glycol)", as used herein, encompasses a family of polyether polymers which share the following general formula: -O-[(CH₂)ₘ-O-]ₙ-, wherein m represents the number of methylene groups present in each alkylene glycol unit, and n represents the number of repeating units, and therefore represents the size or length of the polymer. For example, when m = 2, the polymer is referred to as a polyethylene glycol, and when m = 3, the polymer is referred to as a polypropylene glycol.

In some embodiments, m is an integer greater than 1 (e.g., m = 2, 3, 4, etc.).

Optionally, m varies among the units of the poly(alkylene glycol) chain. For example, a poly(alkylene glycol) chain may comprise both ethylene glycol (m=2) and propylene glycol (m=3) units linked together.

The poly(alkylene glycol) optionally comprises at least two functional groups (e.g., as described herein), each functional group forming a covalent bond with one of the α-galactosidase monomers. The functional groups are optionally terminal groups of the poly(alkylene glycol), such that the entire length of the poly(alkylene glycol) lies between the two functional groups.

The phrase "poly(alkylene glycol)" also encompasses analogs thereof, in which the oxygen atom is replaced by another heteroatom such as, for example, S, -NH- and the like. This term further encompasses derivatives of the above, in which one or more of the methylene groups composing the polymer are substituted. Exemplary substituents on the methylene groups include, but are not limited to, alkyl, cycloalkyl, alkenyl, alkynyl, alkoxy, hydroxy, oxo, thiol and thioalkoxy, and the like.

The phrase "alkylene glycol unit", as used herein, encompasses a -(CH₂)ₘ-O-group or an analog thereof, as described hereinabove, which forms the backbone chain of the poly(alkylene glycol), wherein the (CH₂)ₘ (or analog thereof) is bound to a heteroatom belonging to another alkylene glycol unit or to an α-galactosidase monomer moiety (in cases of a terminal unit), and the O (or heteroatom analog thereof) is bound to the (CH₂)ₘ (or analog thereof) of another alkylene glycol unit, or to a functional group which forms a bond with an α-galactosidase monomer.

An alkylene glycol unit may be branched, such that it is linked to 3 or more neighboring alkylene glycol units, wherein each of the 3 or more neighboring alkylene glycol units are part of a poly(alkylene glycol) chain. Such a branched alkylene glycol unit is linked via the heteroatom thereof to one neighboring alkylene glycol unit, and heteroatoms of the remaining neighboring alkylene glycol units are each linked to a carbon atom of the branched alkylene glycol unit. In addition, a heteroatom (e.g., nitrogen) may bind more than one carbon atom of an alkylene glycol unit of which it is part, thereby forming a branched alkylene glycol unit (e.g., [(-CH₂)ₘ]₂N- and the like).

In exemplary embodiments, at least 50 % of alkylene glycol units are identical, e.g., they comprise the same heteroatoms and the same m values as one another. Optionally, at least 70 %, optionally at least 90 %, and optionally 100 % of the alkylene glycol units are identical. In exemplary embodiments, the heteroatoms bound to the identical alkylene glycol units are oxygen atoms. In further exemplary embodiments, m is 2 for the identical units.

In one embodiment, the linker is a single, straight chain linker, preferably being polyethylene glycol (PEG).

As used herein, the term "poly(ethylene glycol)" describes a poly(alkylene glycol), as defined hereinabove, wherein at least 50 %, at least 70 %, at least 90 %, and preferably 100 %, of the alkylene glycol units are -CH₂CH₂-O-. Similarly, the phrase "ethylene glycol units" is defined herein as units of -CH₂CH₂O-.

According to optional embodiments, the linking moiety comprises a poly(ethylene glycol) or analog thereof, having a general formula:

-X₁-(CR₁R₂-CR₃R₄-Y)ₙ-X₂-

wherein each of X₁ and X₂ is a functional group (e.g., as described herein) that forms a covalent bond with at least one α-galactosidase monomer;
Y is O, S or NR₅ (optionally O);
n is an integer, optionally from 1 to 200 (optionally from 5 to 150, and optionally from 40 to 70), although higher values of n are also contemplated; and
each of R₁, R₂, R₃, R₄, and R₅ is independently selected from the group consisting of hydrogen, alkyl, cycloalkyl, alkenyl, alkynyl, alkoxy, hydroxy, oxo, thiol and thioalkoxy.

In some embodiments, n is at least 5, optionally at least 8, optionally at least 15, optionally at least 25, and optionally at least 40.

In some embodiments, n is no more than 200, optionally no more than 150, and optionally no more than 70.

The poly(ethylene glycol) or analog thereof may optionally comprise a copolymer, for example, wherein the CR₁R₂-CR₃R₄-Y units in the above formula are not all identical to one another.

In some embodiments, at least 50 % of CR₁R₂-CR₃R₄-Y units are identical. Optionally, at least 70 %, optionally at least 90 %, and optionally 100 % of the CR₁R₂-CR₃R₄-Y units are identical.

Optionally, the linking moiety is branched, for example, such that for one or more CR₁R₂-CR₃R₄-Y units in the above formula, at least of one of R₁, R₂, R₃, R₄, and Rs is -(CR₁R₂-CR₃R₄-Y)ₚ-X₃-, wherein R₁-R₄ and Y are as defined hereinabove, p is an integer as defined herein for n (e.g., from 1 to 200), and X₃ is as defined herein for X₁ and X₂.

The functional groups may optionally form a bond such as, but not limited to, an amide bond, an amide bond, an ester bond, and/or an ether bond.

For example, the functional group may optionally comprise a carbonyl group which forms an amide bond with a nitrogen atom in a polypeptide (e.g., in a lysine residue or N-terminus), or an ester bond with an oxygen atom in a polypeptide (e.g., in a serine, threonine or tyrosine residue).

Alternatively or additionally, the functional group may optionally comprise a heteroatom (e.g., N, S, O) which forms an amide bond, ester bond or thioester bond with a carbonyl group in a polypeptide (e.g., in a glutamate or aspartate residue or in a C-terminus).

Alternative or additionally, the functional group may comprise an alkyl or aryl group attached to a polypeptide (e.g., to a heteroatom in the polypeptide).

Alternatively or additionally, the functional group may optionally comprise a nitrogen atom which forms an amine bond with an alkyl group in an α-galactosidase monomer, or an α-galactosidase monomermay optionally comprise a nitrogen atom which forms an amine bond with an alkyl group in the functional group. Such an amine bond may be formed by reductive amination (e.g., as described hereinbelow).

In some embodiments, at least one of the functional groups forms an amide bond with a polypeptide (e.g., with a lysine residue therein).

The functional groups may be identical to one another or different.

In some embodiments, at least one of the functional groups is attached to one functionality of a polypeptide (e.g., an amine group of a lysine residue or N-terminus), and at least one of the functional groups is attached to a different functionality of a polypeptide (e.g., a thiol group of a cysteine residue).

According to optional embodiments, the multimeric protein structure described herein exhibits a high stability in human plasma conditions and/or in lysosomal conditions.

As used herein, the phrase "human plasma conditions" refers to human plasma as a medium, at a temperature of 37 °C.

As used herein, the phrase "lysosomal conditions" refers to an aqueous solution having a pH of 4.6 as a medium (e.g., a citrate phosphate buffer described herein), at a temperature of 37 °C.

Enhanced stability under lysosomal conditions is advantageous because the lysosome is a target for replacement therapy for α-galactosidase, as lysosomes are the normal location for α-galactosidase activity in a body, and lysosomal conditions (e.g., acidic pH) represent optimal conditions for activity of α-galactosidase.

Without being bound by any particular theory, it is believed that enhanced stability in serum-like conditions (e.g., the human plasma conditions described herein) is also advantageous because stable α-galactosidase in the blood can act on metabolites (e.g., Gb₃) present in the blood due to efflux from cells. A serum-active multimeric protein structure could optionally be efficient in removing and preventing glycosphinglipids deposited within blood vessel walls which promote inflammation [Bodary et al., TCM 17(4):129-133]. For example, in Fabry disease, the major pathogenesis results from the accumulation of Gb₃ in the vascular endothelium, leading to vascular occlusion of small vessels, ischemia and infarction of these vessels and ischemia and infarction of the kidney, heart and brain [Desnick et al., 2003, Annals of Internal Medicine, 138(4):338-346]. Additionally, enhanced stability in serum can negate the need for lysosomal trafficking. ERT can thereby become much more accessible, as robust cost-effective host systems e.g., plants, can be employed.

According to optional embodiments, the high stability of the multimeric protein structure in human plasma conditions is such that the multimeric protein structure exhibits, upon being subjected to human plasma conditions for one hour, an α-galactosidase activity which is at least 10 % higher, optionally 20 % higher, optionally 50 % higher, and optionally 100 % higher, than an α-galactosidase activity of native α-galactosidase upon subjecting the native α-galactosidase to the human plasma conditions for one hour.

Alternatively or additionally, the high stability of the multimeric protein structure in human plasma conditions is such that an α-galactosidase activity of the multimeric protein structure decreases more slowly in human plasma conditions than a corresponding activity of the native α-galactosidase. Optionally, the multimeric protein structure exhibits an activity which decreases upon subjecting the protein structure to human plasma conditions for one hour by a percentage which is at least 10 % less, optionally 20 % less, optionally 50 % less, and optionally 80 % less, than the percentage by which a corresponding activity of the native α-galactosidase decreases upon subjecting the native α-galactosidase to human plasma conditions for one hour.

It is to be understood that herein, a decrease which is "10 % less" than a decrease of 50 % refers to a decrease of 45 % (45 being 10% less than 50), and not to a decrease of 40 % (50 % - 10%).

Alternatively or additionally, the high stability of the multimeric protein structure in human plasma conditions is such that an α-galactosidase activity of the multimeric protein structure remains substantially unchanged upon subjecting the multimeric protein structure to human plasma conditions for one hour, and optionally for 2, 4 or even 6 hours.

As used herein, the phrase "substantially unchanged" refers to a level (e.g., of activity) which remains in a range of from 50 % to 150 % of the initial level, and optionally a level which remains at least 60 %, optionally at least 70 %, optionally at least 80 %, and optionally at least 90 % of the initial level.

Optionally, the high stability of the multimeric protein structure in lysosomal conditions is such that the multimeric protein structure exhibits, upon being subjected to lysosomal conditions for a predetermined time period (e.g., one day, two days, 3 days, one week), an α-galactosidase activity which is at least 10 % higher, optionally 20 % higher, optionally 50 % higher, and optionally 100 % higher, than an activity of native α-galactosidase upon subjecting the native α-galactosidase to the lysosomal conditions for the same predetermined time period.

Alternatively or additionally, the high stability of the multimeric protein structure in lysosomal conditions is such that an α-galactosidase activity of the multimeric protein structure decreases more slowly in lysosomal conditions than a corresponding activity of the native α-galactosidase. Optionally, the multimeric protein structure exhibits an activity which decreases upon subjecting the protein structure to lysosomal conditions for a predetermined time period (e.g., one day, 2 days, 3 days, one week), by a percentage which is at least 10 % less, optionally 20 % less, optionally 50 % less, and optionally 80 % less, than the percentage by which a corresponding activity of the native α-galactosidase decreases upon subjecting the native α-galactosidase to lysosomal conditions for the same time period.

Alternatively or additionally, the high stability of the multimeric protein structure in lysosomal conditions is such that an α-galactosidase activity of the multimeric protein structure remains substantially unchanged upon subjecting the multimeric protein structure to lysosomal conditions for one day, for 2 days, for 3 days, for one week, for two weeks, and/or for one month.

As exemplified in the Examples section herein, in addition to exhibiting more stability over time, the multimeric protein structure may exhibit parameters of α-galactosidase activity which are different than those of the native α-galactosidase.

Hence, according to optional embodiments, the multimeric protein structure is characterized as exhibiting, independently of any decay of activity over time, an α-galactosidase activity which is higher than an α-galactosidase activity of a native form of the protein. Optionally, the activity is 10 % higher, and optionally 20 % higher, than the corresponding activity of the native form.

In order to characterize such an activity, the activity is preferably determined immediately (e.g., within 1 hour, within 15 minutes) upon subjecting the native α-galactosidase or multimeric protein structure to conditions (e.g., as described herein) in which the activity decreases substantially, so that the measured activity will reflect the activity *per se*, and not a degree of stability.

Optionally, the multimeric protein structure is characterized as exhibiting an α-galactosidase activity in lysosomal conditions which is higher than a corresponding activity of native α-galactosidase.

Alternatively or additionally, the multimeric protein structure is characterized as exhibiting an α-galactosidase activity in simulated physiological conditions at a neutral pH which is higher than a corresponding activity of native α-galactosidase. The simulated physiological conditions comprise an aqueous solution (e.g., phosphate buffer saline) at a temperature of 37°C. The pH is optionally 7. Alternatively, the pH is 7.4.

The α-galactosidase activity described herein is a biological activity which is characteristic of α-galactosidase (e.g., a catalytic activity characteristic of α-galactosidase, such as hydrolysis of a terminal α-galactosyl moiety of a substrate).

In some embodiments, a catalytic activity of α-galactosidase is characterized by a rate of catalysis at saturation (i.e., a *Vₘₐₓ* value).

Alternatively, the α-galactosidase activity is a therapeutic activity (e.g., an enzymatic activity having a therapeutic effect), such as a therapeutic activity in the context of Fabry disease. Optionally, the therapeutic activity is determined in experimental animals (e.g., Fabry mice), and optionally in human Fabry patients.

Techniques for determining an activity of α-galactosidase will be known to a skilled person. Typically, the α-galactosidase (i.e., the native form or a multimeric protein structure described herein) is contacted with a compound recognized in the art as a substrate of α-galactosidase, and the degree of activity is then determined quantitatively. Compounds which allow for particularly convenient detection of α-galactosidase activity are known in the art and are commercially available.

In some embodiments, α-galactosidase activity is determined by assaying hydrolysis of 4-methylumbelliferyl-α-D-galactopyranoside (e.g., as described in the Examples section herein).

In some embodiments, α-galactosidase activity is determined by assaying hydrolysis of p-nitrophenyl-α-D-galactopyranoside (e.g., as described in the Examples section herein).

When comparing an activity of a multimeric protein structure described herein with an activity of native α-galactosidase, the native α-galactosidase preferably comprises α-galactosidase monomers substantially identical (e.g., with respect to amino acid sequence and glycosylation pattern) to the α-galactosidase monomers of the multimeric structure.

According to some embodiments, the multimeric protein structure is characterized by a circulating half-life in a physiological system (e.g., blood, serum and/or plasma of a human or laboratory animal) which is higher (e.g., at least 20 %, at least 50 % higher, at least 100 % higher, at least 400 % higher, at least 900 % higher) than a circulating half-life of native α-galactosidase.

An increased circulating half-life may optionally be associated with a higher *in vitro* stability (e.g, as described herein), a higher *in vivo* stability (e.g, resistance to metabolism) and/or with other factors (e.g., reduced renal clearance).

Circulating half-lives can be determined by taking samples (e.g., blood samples, tissue samples) from physiological systems (e.g., humans, laboratory animals) at various intervals, and determining a level of α-galactosidase in the sample, using techniques known in the art.

Optionally, the half-life is calculated as a terminal half-life (e.g., as described in the Examples section), wherein half-life is the time required for a concentration (e.g., a blood concentration) to decrease by 50 % after pseudo-equilibrium of distribution has been reached. The terminal half-life may be calculated from a terminal linear portion of a time vs. log concentration, by linear regression of time vs. log concentration (see, for example, Toutain & Bousquet-Melou [J Vet Pharmacol Ther 2004, 27:427-39]). Thus, the terminal half-life is a measure of the decrease in drug plasma concentration due to drug elimination and not of decreases due to other reasons, and is not necessarily the time necessary for the amount of the administered drug to fall by one half.

Determining a level of α-galactosidase (e.g., the multimeric protein structure or the native α-galactosidase) may comprise detecting the physical presence of α-galactosidase (e.g., via an antibody against α-galactosidase) and/or detecting a level of an α-galactosidase activity (e.g., as described herein).

According to some embodiments, the multimeric protein structure is characterized by an α-galactosidase activity in an organ (e.g., spleen, heart, kidney, brain, liver) upon administration (e.g., intravenous administration) of the protein structure to a vertebrate (e.g., a human, a mouse), for example, a vertebrate with an α-galactosidase deficiency (e.g., a human Fabry disease patient, a Fabry mouse). Optionally, the α-galactosidase activity in the organ is higher than an α-galactosidase activity of native α-galactosidase in the organ, upon an equivalent administration to a vertebrate.

The activity in an organ may be a function of uptake of the α-galactosidase and/or retention of α-galactosidase activity following uptake.

Optionally, α-galactosidase activity in the organ is determined 2 hours after administration, and optionally 24 hours, optionally 3 days, optionally 7 days, and optionally 14 days, after administration.

As increased activity of α-galactosidase in a liver may in some cases be associated with a lower activity in other parts of a body, and hence, with a reduced biological effect of the α-galactosidase.

Hence, in some embodiments, the multimeric protein structure is characterized by an enhanced α-galactosidase activity in an organ other than a liver. Exemplary organs include the spleen, heart and kidneys.

In some embodiments, the multimeric protein structure is characterized by an enhanced α-galactosidase activity in an organ after administration (as described herein) which is at least 20 % higher, optionally at least 50 % higher, optionally at least 100 % higher, and optionally at least 300 % higher, than the activity of native α-galactosidase after an equivalent administration. As noted hereinabove, the present inventors have devised and successfully prepared and practiced stabilized forms of α-galactosidase by means of multimeric structures of cross-linked α-galactosidase monomers.

Optionally, the α-galactosidase is a human α-galactosidase (e.g., a recombinant human α-galactosidase), for example, in order to facilitate optimal biocompatibility for administration to human subjects. Human α-galactosidase is commercially available, for example, as Replagal^{®} (agalsidase alpha, Shire) and Fabrazyme^{e} (agalsidase beta, Genzyme).

Herein, "human α-galactosidase" refers to an α-galactosidase comprising an amino acid sequence substantially identical (e.g., as described hereinabove) to an amino acid sequence of an α-galactosidase protein which naturally occurs in humans.

In some embodiments, the α-galactosidase is a plant recombinant α-galactosidase. Exemplary α-galactosidases include plant recombinant human α-galactosidases.

Examples of α-GAL include, without limitation, α-GAL having an amino acid sequence selected from the group consisting of SEQ ID NO: 1, SEQ ID NO: 2, and SEQ ID NO: 3. Optionally, the α-GAL has an amino acid sequence selected from the group consisting of SEQ ID NO: 2 and SEQ ID NO: 3.

As used herein, "α-galactosidase" refers to any protein which exhibits an enzymatic activity (e.g., hydrolysis) towards galactose moieties in Gb₃ (e.g., α-galactosidase A). Optionally, "α-galactosidase" refers to E.C. 3.2.1.22.

The α-galactosidase of embodiments of the invention can be purified (e.g., from plants or animal tissue) or generated by recombinant DNA technology.

As described herein, activity of α-galactosidase in serum may be highly advantageous, for example, for reducing Gb₃ levels in serum.

Hence, in some embodiments, the α-galactosidase is an alkaline α-galactosidase.

As used herein the phrase "alkaline α-galactosidase" refers to α-GAL characterized by an ability to hydrolyse terminal-linked α-galactose moieties from galactose-containing oligosaccharides under neutral to basic pH conditions (e.g., about pH 7-7.5), particularly at a normal serum pH (e.g., about 7.35-7.45).

It will be appreciated that an alkaline α-GAL of some embodiments of the invention may be active under neutral to basic pH conditions but may still display activity under acidic pH conditions (i.e., about 4.6).

In a specific embodiment the enzyme is active under acidic to basic pH conditions (i.e., about pH 4.2-7.5).

In yet another specific embodiment the enzyme is active under pH of about 6.5-7.5.

Specific examples of alkaline α-galactosidases which can be used in accordance with the present teachings are provided in US Patent Application 20070036883, WO03/097791, and in PCT/IL2010/000956.

Thus, alkaline α-galactosidase can be a member of the plant family selected from the group consisting of Cucurbitaceae, Lamiaceae, Piperaceae, Solanaceae, Leguminosae, Cruciferae and Gramineae families.

According to a specific embodiment, the alkaline α-galactosidase is from melon.

P.-R. Gaudreault and J. A. Webb have described in several publications, (such as "Alkaline alpha-galactosidase in leaves of Cucurbita pepo", Plant Sci. Lett. 24, 281-288, 1982, "Partial purification and properties of an alkaline alpha-galactosidase from mature leaves of Cucurbita pepo", Plant Physiol., 71, 662-668, 1983, and "Alkaline alpha-galactosidase activity and galactose metabolism in the family Cucurbitaceae", Plant Science, 45, 71-75, 1986), a novel α-galactosidase purified from young leaves of Cucurbita pepo, that has an optimal activity at alkaline conditions (pH 7.5). In addition to the alkaline α-galactosidase, they also reported three acid forms of the enzyme, and distinct substrate preferences were found for the acid and alkaline forms

α-Galactosidase activity at alkaline pH has been observed in other cucurbit tissue, such as cucumber fruit pedicels, young squash fruit and young melon fruit ("Melons: Biochemical and Physiological Control of Sugar Accumulation", In: Encyclopedia of Agricultural Science, vol. 3, pp. 25-37, Arntzen, C. J., et al., eds. Academic Press, New York, 1994).

Bachmann et al. ("Metabolism of the raffinose family oligosaccharides in leaves of Ajuga reptens L.", Plant Physiology 105:1335-1345, 1994) that Ajuga reptens plants (common bugle), a stachyose translocator from the unrelated Lamiaceae family also contains an alkaline α-galactosidase. This enzyme was partially characterized and found to have high affinity to stachyose. Also, leaves of the Peperomia camptotricha L. plant, from the family Piperaceae, show α-galactosidase activity at alkaline pH, suggesting that they also contain an alkaline α-galactosidase enzyme (Madore, M., "Catabolism of raffinose family oligosaccharides by vegetative sink tissues", In: Carbon Partitioning and Source-Sink Interactions in Plants, Madore, M. and Lucas, W. J. (eds.) pp. 204-214, 1995, American Society of Plant Physiologists, Maryland). Similarly, Gao and Schaffer (Plant Physiol. 1999; 119:979=88) have reported an α-galactosidase activity with alkaline pH optimum in crude extracts of tissues from a variety of species including members of the Cucurbit and Coleus (Lamiaceae) families.

Specific examples of plant alkaline α-galactosidase sequences are provided in SEQ ID NOs: 4, 5 and 13 (*Cucumis melo*), 6 *(T. tetragonioides),* 7 and 12 *(Cucumis sativus),* 8 and 9 *(Zea mays),* 10 *(Oruza sativa),* 11 *(Pisum sativum)* and 14 *(Coffea arabica).*

In some embodiments, the α-galactosidase is an acid α-galactosidase.

As used herein, "acid α-galactosidase" refers to α-galactosidase characterized by an ability to hydrolyse terminal-linked α-galactose moieties from galactose-containing oligosaccharides under acidic pH conditions (e.g., about pH 4.2 - 5), such as occur in a lysosome.

The α-galactosidase of embodiments of the invention can be of any human, animal or plant source, provided no excessively adverse immunological reaction is induced upon *in vivo* administration (e.g., plant to human).

To reduce immunological reaction, a non-human α-galactosidase preparation (e.g., of plant α-galactosidase) can be co-administered with a human α-galactosidase (i.e., acid human α-galactosidase).

Optionally, the multimeric protein structure further comprises at least one mannose-6-phosphate (M6P) moiety. The M6P moiety (or moieties) may be linked to one or more of the α-galactosidase monomers of the multimeric protein structure (e.g., via a linker).

Techniques and reagents for introducing M6P-containing moieties to a biomolecule (e.g., a polypeptide) are described in WO 2009/024977.

As exemplified in the Examples section herein, a multimeric protein structure described herein may be conveniently prepared by reacting α-galactosidase with a cross-linking agent.

Hence, according to another aspect of embodiments of the invention, there is provided a process of preparing a multimeric protein structure described herein. The process comprises reacting α-galactosidase, so as to introduce at least one linking moiety which covalently links at least two α-galactosidase monomers.

Optionally, the linking moiety is a bond (e.g., an amide bond, a disulfide bond) which links one α-galactosidase monomer to another α-galactosidase monomer. Optionally, the bond is introduced by using suitable conditions and/or reagents. For example, reagents which are suitable for forming an amide bond from a carboxylic acid group and an amine group are known in the art.

Optionally, the linking moiety is a moiety which is not derived from a part of the α-galactosidase. For example, the linking moiety may be an oligomer, a polymer, a residue of a small molecule (e.g., an amino acid).

In some embodiments, the linking moiety is introduced by reacting the α-galactosidase with a cross-linking agent which comprises the linking moiety (e.g., as described herein) and at least two reactive groups.

Optionally, the α-galactosidase is reacted under conditions in which the native α-galactosidase is in a dimeric form.

In some embodiments, the cross-linking agent is reacted with the α-galactosidase at a molar ratio in a range of from 5:1 to 500:1 (cross-linking agent: α-galactosidase monomer), optionally in a range of from 50:1 to 400:1, and optionally in a range of from 75:1 to 300:1 (e.g., about 100:1, about 200:1).

The process optionally further comprises purifying the cross-linked protein, for example, removing excess cross-linking agent. Common purification methods may be used, such as dialysis and/or ultra-filtration using appropriate cut-off membranes and/or additional chromatographic steps, including size exclusion chromatography, ion exchange chromatography, affinity chromatography, hydrophobic interaction chromatography, and the like.

The reactive group is selected suitable for undergoing a chemical reaction that leads to a bond formation with a complementary functionality in the α-galactosidase monomer. Optionally, each reactive group is capable of forming a covalent bond between the linking moiety described herein and at least one polypeptide (e.g., so as to form a functional group bound to the polypeptide, as described herein).

The reactive groups of a cross-linking agent may be identical to one another or different.

As used herein, the phrase "reactive group" describes a chemical group that is capable of undergoing a chemical reaction that typically leads to a bond formation. The bond, according to the present embodiments, is preferably a covalent bond (e.g., for each of the reactive groups). Chemical reactions that lead to a bond formation include, for example, nucleophilic and electrophilic substitutions, nucleophilic and electrophilic addition reactions, alkylations, addition-elimination reactions, cycloaddition reactions, rearrangement reactions and any other known organic reactions that involve a functional group, as well as combinations thereof.

The reactive group may optionally comprise a non-reactive portion (e.g., an alkyl) which may serve, for example, to attach a reactive portion of the reactive group to a linking moiety (e.g., poly(alkylene glycol) or analog thereof) described herein.

The reactive group is preferably selected so as to enable its conjugation to α-galactosidase. Exemplary reactive groups include, but are not limited to, carboxylate (e.g., -CO₂H), thiol (-SH), amine (-NH₂), halo, azide (-N₃), isocyanate (-NCO), isothiocyanate (-N=C=S), hydroxy (-OH), carbonyl (e.g., aldehyde), maleimide, sulfate, phosphate, sulfonyl (e.g. mesyl, tosyl), etc. as well as activated groups, such as *N-*hydroxysuccinimide (NHS) (e.g. NHS esters), sulfo-N-hydroxysuccinimide, anhydride, acyl halide (-C(=O)-halogen) etc.

In some embodiments, the reactive group comprises a leaving group, such as a leaving group susceptible to nucleophilic substitution (e.g., halo, sulfate, phosphate, carboxylate, N-hydroxysuccinimide).

Optionally, the reactive group may be in an activated form thereof.

As used herein, the phrase "activated form" describes a derivative of a chemical group (e.g., a reactive group) which is more reactive than the chemical group, and which is thus readily capable of undergoing a chemical reaction that leads to a bond formation. The activated form may comprise a particularly suitable leaving group, thereby facilitating substitution reactions. For example, a -C(=O)-NHS group (N-hydroxysuccinimide ester, or -C(=O)-O-succinimide) is a well-known activated form of -C(=O)OH, as NHS (N-hydroxysuccinimide) can be reacted with a -C(=O)OH to form -C(=O)-NHS, which readily reacts to form products characteristic of reactions involving -C(=O)OH groups, such as amides and esters.

The reactive group can be attached to the rest of the linking moiety (e.g., a poly(alkylene glycol) or analog thereof) via different groups, atoms or bonds. These may include an ether bond [e.g., -O-alkyl-], an ester bond [e.g., -O-C(=O)-alkyl-], a carbamate [e.g., O-C(=O)-NH-alkyl-], etc. Thus, a variety of terminal groups can be employed.

The following are non-limiting examples of the different groups that may constitute a reactive group as described herein: -CH₂CO₂H, -CH₂CH₂CO₂H, -CH₂CH₂SH, -CH₂CH₂NH₂, -CH₂CH₂N₃, -CH₂CH₂NCO, -CH₂-C(=O)-NHS, -CH₂CH₂-C(=O)-NHS, -C(=O)-CH₂-C(=O)-NHS, -CH₂CH₂-NHC(=O)CH₂CH₂-maleimide, etc.

The number of methylene groups in each of the above reactive groups is merely exemplary, and may be varied.

The reactive group may also comprise the heteroatom at the end of a poly(alkylene glycol) chain (e.g., -OH).

In exemplary embodiments of the present invention, the reactive group comprises a carboxylate (e.g., an activated carboxylate such as an N-hydroxysuccinimide ester).

Optionally, the reactive group reacts with an amine group in the α-galactosidase (e.g., in a lysine residue and/or an N-tcrminus) to form an amide bond.

In some embodiments, the reaction of the reactive group comprises reductive amination, wherein an amine group reacts with an aldehyde group to form an imine, and the imine is reduced (e.g., by addition of a reducing agent, such as sodium cyanoborohydride) to form an amine bond. The reactive group may be an amine group which reacts with an aldehyde group of the α-galactosidase (e.g., on a saccharide moiety attached to the polypeptide of the protein), or the reactive group may be an aldehyde group which reacts with an amine group of the α-galactosidase (e.g., on a lysine residue). Optionally, a saccharide moiety of α-galactosidase is oxidized by an oxidizing agent to form an aldehyde group, prior to reaction of the reactive group with the α-galactosidase. For example, reaction of a saccharide with sodium periodate may be used to produce a pair of aldehyde groups in a saccharide moiety.

In some embodiments, at least one of the reactive groups is selected so as to react with one functionality of an α-galactosidase monomer (e.g., an amine group of a lysine residue or N-terminus), and at least one of the reactive groups is selected so as to react with a different functionality of an α-galactosidase monomer (e.g., a thiol group of a cysteine residue).

Optionally, one or more polypeptides described herein are reacted with a glycosylation reagent for introducing one or more M6P moieties, in order to obtain an M6P-containing multimeric protein structure (e.g., as described herein). Suitable M6P-containing glycosylation reagents and their use are described, for example, in WO 2009/024977.

As used herein, the terms "amine" and "amino" refer to either a -NR'R" group, wherein R' and R" are selected from the group consisting of hydrogen, alkyl, cycloalkyl, heteroalicyclic (bonded through a ring carbon), aryl and heteroaryl (bonded through a ring carbon). R' and R" are bound via a carbon atom thereof. Optionally, R' and R" are selected from the group consisting of hydrogen and alkyl comprising 1 to 4 carbon atoms. Optionally, R' and R" are hydrogen.

As used herein throughout, the term "alkyl" refers to a saturated aliphatic hydrocarbon including straight chain and branched chain groups. Preferably, the alkyl group has 1 to 20 carbon atoms. Whenever a numerical range; e.g., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. When substituted, the substituent group can be, for example, cycloalkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "cycloalkyl" group refers to an all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be, for example, alkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

An "alkenyl" group refers to an alkyl group which consists of at least two carbon atoms and at least one carbon-carbon double bond.

An "alkynyl" group refers to an alkyl group which consists of at least two carbon atoms and at least one carbon-carbon triple bond.

An "aryl" group refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroaryl" group refers to a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. Representative examples are piperidine, piperazine, tetrahydrofuran, tetrahydropyran, morpholine and the like.

A "hydroxy" group refers to an -OH group.

An "azide" group refers to a -N=N⁺=N⁻ group.

An "alkoxy" group refers to both an -O-alkyl and an -O-cycloalkyl group, as defined herein.

An "aryloxy" group refers to both an -O-aryl and an -O-heteroaryl group, as defined herein.

An "ether" refers to both an alkoxy and an aryloxy group, wherein the group is linked to an alkyl, alkenyl, alkynyl, cycloalkyl, aryl, heteroaryl or heteroalicyclic group.

An ether bond describes a -0- bond.

A "thiohydroxy" or "thiol" group refers to a -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "thioether" refers to both a thioalkoxy and a thioaryloxy group, wherein the group is linked to an alkyl, alkenyl, alkynyl, cycloalkyl, aryl, heteroaryl or heteroalicyclic group.

A thioether bond describes a -S- bond.

A "disulfide" group refers to both a -S-thioalkoxy and a -S-thioaryloxy group.

A disulfide bond describes a -S-S- bond.

A "carbonyl" group refers to a -C(=O)-R' group, where R' is defined as hereinabove.

A "thiocarbonyl" group refers to a -C(=S)-R' group, where R' is as defined herein.

A "carboxyl" refers to both "C-carboxy" and O-carboxy".

A "C-carboxy" group refers to a -C(=O)-O-R' groups, where R' is as defined herein.

An "O-carboxy" group refers to an R'C(=O)-O- group, where R' is as defined herein.

An "oxo" group refers to a =O group.

A "carboxylate" or "carboxyl" encompasses both C-carboxy and O-carboxy groups, as defined herein.

A "carboxylic acid" group refers to a C-carboxy group in which R' is hydrogen.

A "thiocarboxy" or "thiocarboxylate" group refers to both -C(=S)-O-R' and -O-C(=S)R' groups.

An "ester" refers to a C-carboxy group wherein R' is not hydrogen.

An ester bond refers to a -O-C(=O)- bond.

A thioester bond refers to a -0-C(=S)- bond or to a -S-C(=O) bond.

A "halo" group refers to fluorine, chlorine, bromine or iodine.

A "sulfinyl" group refers to an -S(=O)-R' group, where R' is as defined herein.

A "sulfonyl" group refers to an -S(=O)₂-R' group, where R' is as defined herein.

A "sulfonate" group refers to an -S(=O)₂-O-R' group, where R' is as defined herein.

A "sulfate" group refers to an -O-S(=O)₂-O-R' group, where R' is as defined as herein.

A "sulfonamide" or "sulfonamido" group encompasses both S-sulfonamido and N-sulfonamido groups, as defined herein.

An "S-sulfonamido" group refers to a -S(=O)₂-NR'R" group, with each of R' and R" as defined herein.

An "N-sulfonamido" group refers to an R'S(=O)₂-NR" group, where each of R' and R" is as defined herein.

An "O-carbamyl" group refers to an -OC(=O)-NR'R" group, where each of R' and R" is as defined herein.

An "N-carbamyl" group refers to an R'OC(=O)-NR"- group, where each of R' and R" is as defined herein.

A "carbamyl" or "carbamate" group encompasses O-carbamyl and N-carbamyl groups.

A carbamate bond describes a -O-C(=O)-NR'- bond, where R' is as described herein.

An "O-thiocarbamyl" group refers to an -OC(=S)-NR'R" group, where each of R' and R" is as defined herein.

An "N-thiocarbamyl" group refers to an R'OC(=S)NR"- group, where each of R' and R" is as defined herein.

A "thiocarbamyl" or "thiocarbamate" group encompasses O-thiocarbamyl and N-thiocarbamyl groups.

A thiocarbamate bond describes a -O-C(=S)-NR'- bond, where R' is as described herein.

A "C-amido" group refers to a -C(=O)-NR'R" group, where each of R' and R" is as defined herein.

An "N-amido" group refers to an R'C(=O)-NR"- group, where each of R' and R" is as defined herein.

An "amide" group encompasses both C-amido and N-amido groups.

An amide bond describes a -NR'-C(=O)- bond, where R' is as defined herein.

An amine bond describes a bond between a nitrogen atom in an amine group (as defined herein) and an R' group in the amine group.

A thioamide bond describes a -NR'-C(=S)- bond, where R' is as defined herein.

A "urea" group refers to an -N(R')-C(=O)-NR"R"' group, where each of R' and R" is as defined herein, and R'" is defined as R' and R" are defined herein.

A "nitro" group refers to an -NO₂ group.

A "cyano" group refers to a -C≡N group.

The term "phosphonyl" or "phosphonate" describes a -P(=O)(OR')(OR") , group, with R' and R" as defined hereinabove.

The term "phosphate" describes an -O-P(=O)(OR')(OR") group, with each of R' and R" as defined hereinabove.

A "phosphoric acid" is a phosphate group is which each of R is hydrogen.

The term "phosphinyl" describes a -PR'R" group, with each of R' and R" as defined hereinabove.

The term "thiourea" describes a -N(R')-C(=S)-NR"- group, with each of R' and R" as defined hereinabove.

As described herein, multimeric protein structures described herein may exhibit improved stability and stronger and/or longer lasting α-galactosidase activity at therapeutically important sites *in vivo.* Such multimeric protein structures are therefore highly beneficial for use in various medical applications in which α-galactosidase activity is desirable, including therapeutic and research applications.

Hence, according to some embodiments, the multimeric protein structure described herein is for use as a medicament, for example, a medicament for treating Fabry disease.

According to another aspect of embodiments of the invention, there is provided a method of treating Fabry disease, the method comprising administering to a subject in need thereof a therapeutically effective amount of a multimeric protein structure described herein.

According to another aspect of embodiments of the invention, there is provided a pharmaceutical composition that comprises a multimeric protein structure as described herein and a pharmaceutically acceptable carrier.

As used herein a "pharmaceutical composition" refers to a preparation of one or more of the multimeric protein structures described herein, with other chemical components such as pharmaceutically acceptable and suitable carriers and excipients. The purpose of a pharmaceutical composition is to facilitate administration of a compound to an organism.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

The pharmaceutical composition optionally comprises an additional ingredient which further stabilizes the α-galactosidase of the multimeric protein structure. Optionally, the additional ingredient is galactose.

Alternatively, a galactose derivative (e.g., a galactose-containing glycoside) may be used instead of galactose. Optionally, a non-reducing galactose derivative is used.

Techniques for formulation and administration of drugs may be found in "Remington's Pharmaceutical Sciences" Mack Publishing Co., Easton, PA, latest edition.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more pharmaceutically acceptable carriers comprising excipients and auxiliaries, which facilitate processing of the multimeric protein structure into preparations which can be used pharmaceutically. Proper formulation is dependent upon the route of administration chosen.

For injection or infusion, the multimeric protein structures of embodiments of the invention may be formulated in aqueous solutions, preferably in physiologically compatible buffers such as Hank's solution, Ringer's solution, or physiological saline buffer with or without organic solvents such as propylene glycol, polyethylene glycol.

For transmucosal administration, penetrants are used in the formulation. Such penetrants are generally known in the art.

For oral administration, the multimeric protein structures of the invention can be formulated readily by combining the multimeric protein structures with pharmaceutically acceptable carriers well known in the art. Such carriers enable the multimeric protein structures described herein to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of doses of active multimeric protein structure.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the multimeric protein structures may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by inhalation, the multimeric protein structures for use according to embodiments of the present invention are conveniently delivered in the form of an aerosol spray presentation (which typically includes powdered, liquified and/or gaseous carriers) from a pressurized pack or a nebulizer, with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of the multimeric protein structures and a suitable powder base such as, but not limited to, lactose or starch.

The multimeric protein structures described herein may be formulated for parenteral administration, e.g., by bolus injection or continuous infusion. Formulations for injection or infusion may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the multimeric protein structure preparation in water-soluble form. Additionally, suspensions of the multimeric protein structures may be prepared as appropriate oily injection suspensions and emulsions (e.g., water-in-oil, oil-in-water or water-in-oil in oil emulsions). Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents, which increase the solubility of the multimeric protein structures to allow for the preparation of highly concentrated solutions.

Alternatively, the multimeric protein structures may be in powder form for constitution with a suitable vehicle, e.g., sterile, pyrogen-free water, before use.

The multimeric protein structure of embodiments of the present invention may also be formulated in rectal compositions such as suppositories or retention enemas, using, e.g., conventional suppository bases such as cocoa butter or other glycerides.

The pharmaceutical compositions herein described may also comprise suitable solid of gel phase carriers or excipients. Examples of such carriers or excipients include, but are not limited to, calcium carbonate, calcium phosphate, various sugars, starches, cellulose derivatives, gelatin and polymers such as polyethylene glycols.

Pharmaceutical compositions suitable for use in the context of the present invention include compositions wherein the active ingredients are contained in an amount effective to achieve the intended purpose. More specifically, a therapeutically effective amount means an amount of multimeric protein structures effective to prevent, alleviate or ameliorate symptoms of disease or prolong the survival of the subject being treated.

For any multimeric protein structures used in the methods of the invention, the therapeutically effective amount or dose can be estimated initially from activity assays in animals. For example, a dose can be formulated in animal models to achieve a circulating concentration range that includes the IC₅₀ as determined by activity assays (e.g., the concentration of the test protein structures, which achieves a half-maximal increase in a biological activity of the multimeric protein structure). Such information can be used to more accurately determine useful doses in humans.

As is demonstrated in the Examples section that follows, a therapeutically effective amount for the multimeric protein structures of embodiments of the present invention may range between about 1 µg/kg body weight and about 500 mg/kg body weight.

Toxicity and therapeutic efficacy of the multimeric protein structures described herein can be determined by standard pharmaceutical procedures in experimental animals, e.g., by determining the EC₅₀, the IC₅₀ and the LD₅₀ (lethal dose causing death in 50 % of the tested animals) for a subject protein structure. The data obtained from these activity assays and animal studies can be used in formulating a range of dosage for use in human.

The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p.1).

Dosage amount and interval may be adjusted individually to provide plasma levels of the active moiety which are sufficient to maintain the desired effects, termed the minimal effective concentration (MEC). The MEC will vary for each preparation, but can be estimated from *in vitro* data; e.g., the concentration necessary to achieve the desired level of activity *in vitro.* Dosages necessary to achieve the MEC will depend on individual characteristics and route of administration. HPLC assays or bioassays can be used to determine plasma concentrations.

Dosage intervals can also be determined using the MEC value. Preparations should be administered using a regimen, which maintains plasma levels above the MEC for 10-90 % of the time, preferable between 30-90 % and most preferably 50-90 %.

Depending on the severity and responsiveness of the condition to be treated, dosing can also be a single administration of a slow release composition described hereinabove, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a multimeric protein structure of embodiments of the invention formulated in a compatible pharmaceutical carrier may also be prepared, placed in an appropriate container, and labeled for treatment of an indicated condition or diagnosis, as is detailed herein.

Thus, according to an embodiment of the present invention, depending on the selected multimeric protein structures, the pharmaceutical composition described herein is packaged in a packaging material and identified in print, in or on the packaging material, for use in the treatment of a condition in which the activity of the multimeric protein structure is beneficial, as described hereinabove.

As used herein the term "about" refers to ± 10 %

The terms "comprises", "comprising", "includes", "including", "having" and their conjugates mean "including but not limited to".

The term "consisting of means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

### MATERIALS AND METHODS

### Materials:

bis N-hydroxysuccinimide-poly(ethylene glycol) (bis-NHS-PEG) was obtained from Iris Biotech GmbH in PEG₈ and 2000 Dalton (PEG₄₅) PEG forms, and from Pierce in PEG₅ form, and dissolved in dimethyl sulfoxide (DMSO) at a concentration of 25 mg/mL;
Citric acid was obtained from Sigma;
Coomassie Blue G250 was obtained from Bio-Rad;
Dimethyl sulfoxide was obtained from Sigma;
D-(+)-galactose was obtained from Sigma;
Human plasma (K3 EDTA) was obtained from Bioreclamation Inc.;
4-Methylumbelliferone was obtained from Sigma;
4-Methylumbelliferyl-α-D-galactopyranoside was obtained from Sigma;
N-dodecanoyl-nitrobenzoxadiazole-ceramide trihexoside (Gb₃-NBD) was obtained from Matreya;
2-((*N*-morpholino)ethanesulfonic acid was obtained from Merck;
Phosphate buffered saline was obtained from Sigma;
*p*-Nitrophenyl-α-D-galactopyranoside was obtained from Sigma;
Primuline was obtained from Sigma; Primuline spray reagent was prepared by dissolving 12.5 mg primuline in 200 ml acetone:water (8:2 volume ratio);
Pyridine was obtained from Sigma;
Sinapinic acid was obtained from Sigma;
Sodium carbonate was obtained from Sigma;
Sodium phosphate was obtained from Sigma;
Sodium taurocholate was obtained from Sigma;
Trifluoroacetic acid was obtained from Sigma.

### Plant recombinant human α-GAL-I:

Plant recombinant human α-GAL (prh-α-GAL) having SEQ ID NO: 1, referred to herein as plant recombinant human α-GAL-I (prh-α-GAL-I), was prepared as described in International Patent Application PCT/IL2008/000576 (published as WO 2008/132743).

Transgenic plant material was generated using *Nicotiana Benthamiana* plants infiltrated with genetic construct containing the expression cassette for α-GAL-A, for expressing the human α-GAL-A protein. This was performed in a growth chamber under controlled conditions. This was followed by harvest of plant material and extraction of soluble proteins from the plant cells. prh-α-GAL-A was then purified by a purification process involving standard methods for protein purification followed by a chemical modification step to manufacture the cross-linked protein. The current prh-α-GAL-A was extracted from plant material using homogenizers. The plant debris was removed by centrifugation and the protein was further purified using ammonium sulfate precipitation and acidification steps. The supernatant was filtered and loaded onto a hydrophobic column, followed by desalting and loading onto a cation exchange column. The pool of the cation exchange column was concentrated.

### Plant recombinant Human α-GAL-II:

Plant recombinant human α-GAL comprising a mixture of α-GAL having SEQ ID NO: 2 and α-GAL having SEQ ID NO: 3 (without the N-terminal amino acids EF present in SEQ ID NO: 1), referred to herein as prh-α-GAL-II, was prepared by a process similar to that described above for prh-α-GAL-I, using a different genetic construct.

cDNA encoding the human α-galactosidase protein (EC 3.2.1-22 GenBank: X05790) was optimized and synthesized by GENEART AG (Regensburg, Germany). The codon usage without the leader peptide (endoplasmic reticulum target signal peptide) was adapted to the codon bias of *Nicotiana tobaccum* genes. During the optimization process the following cis-acting sequence motifs were avoided: internal TATA-boxes, chi-sites and ribosomal entry sites, AT-rich or GC-rich sequence stretches, RNA instability elements ("killer motifs"), repeat sequences and RNA secondary structures, splice donor (cryptic) and acceptor sites, branch points. In addition, regions of very high (>80 %) or very low (<30 %) GC content has been avoided.

The nucleotide sequence of the native human α-galactosidase leader peptide (endoplasmic reticulum target signal peptide) of full length human α-galactosidase protein (GenBank: X05790) was replaced with a nucleotide sequence encoding the 33 amino acid endoplasmic reticulum targeting signal peptide (leader peptide) of the Arabidopsis ABPI protein. This signal peptide provides efficient targeting of α-galactosidase to the secretory pathway and is cleaved from the polypeptide, by signal peptidase, once the protein has been translocated into the endoplasmic reticulum. A nucleotide sequence encoding the endoplasmic reticulum retention signal SEKDEL was added to the cDNA sequence at the 3' terminus, allowing retrieval of the expressed protein from the Golgi apparatus, effectively maintaining the protein in the endoplasmic reticulum.

The protein of interest was expressed from a strong subgenomic viral promoter of the coat protein. The system relies on transient amplification (by agroinfection) of viral vectors delivered to a plant by *Agrobacterium*. In agroinfection, a plant functional promoter and the cDNA encoding a viral replicon are transferred as T-DNA from *Agrobacterium* into plant cells. The T-DNA is transcribed *in-planta* by the plant promoter to generate biologically active viral RNA that initiates self replication.

For the transient expression a 3 vector recombination system based on the system previously developed as described [Gleba et al., Vaccine 2005, 23:2042-2048]. One of the vectors was inserted with α-galactosidase cDNA and the two other vectors containing genes for construction of the whole viral replicon (RdRp and Integrase), thus generating the biologically active viral RNA that can initiate self replication

*N. Benthamiana* plants were germinated and grown in commercial mix soil (Givaat Ada, IL) supplemented with granular slow release fertilizer (Scott Marysville, OH) under a long day (16 hours light / 8 hours dark) light regime at 24-25 °C.

Agrobacteria were transformed with the pICH20866-alpha-GAL based replicon vector system using electroporation (2500 V, 5 milliseconds) [den Dulk-Ra and Hooykaas, Methods Mol Biol 1995, 55:63-72]. Plants were infiltrated with Agrobacteria containing the 3 ICON plasmids by vacuum infiltration with standard methods known in the art. Briefly, *N. benthamiana* plants, 5-6 week old, were infiltrated by immersing all aerial plant organs into a bacterial suspension and were placed in a vacuum chamber. A minus (-) 0.8 bar vacuum was applied for 1 minute, followed by a quick return to atmospheric pressure. Plants were returned to the greenhouse for additional 5-7 days under the same growth conditions.

Samples of *Nicotiana benthamiana* leaves were harvested 5 days post infiltration and extracted in Laemmli buffer for SDS-PAGE, or in activity assay buffer (20 mM citric acid, 30 mM sodium phosphate, 0.1 % bovine serum albumin and 0.67 % ethanol, pH 4.6.) for assay of catalytic activity of the plant expressed protein.

Human α-galactosidase protein from plant extracts was purified by a two-step ammonium sulfate differential precipitation ("salting out": 1^{st} step 0.57 M; 2^{nd} step 2.27 M), followed by hydrophobic interaction chromatography (Phenyl 650 M resin) and cation exchange chromatography.

Two sequences (i.e., SEQ ID NO: 2 and SEQ ID NO: 3), which differ in the presence or absence of an N-terminal glycine, were obtained due to different leader sequence processing.

### 4-Methylumbelliferyl-α-D-galactopyranoside assay of α-GAL activity:

α-GAL activity was measured using 4-methylumbelliferyl-α-D-galactopyranoside as a hydrolysis substrate. The assay was performed in citrate-phosphate buffer (20 mM citric acid, 30 mM sodium phosphate, pH 4.6). 10 µL of sample containing the tested α-GAL was incubated with 40 µL assay buffer containing 5 mM 4-methylumbelliferyl-α-D-galactopyranoside. The reaction mixture was incubated at 37 °C for 60 minutes. 10 µL of the reaction mixture were transferred into a black 96-well plate (Greiner), 90 µL of stop solution (2 M sodium carbonate) was added, and fluorescence was measured at an excitation wavelength of 365 nm and an emission wavelength of 450 nm. Fluorescence was translated to product concentration, and further to activity, using a calibration curve of 4-methylumbelliferone, the reaction product.

### N-Dodecanoyl-nitrobenzoxadiazole-ceramide trihexoside (Gb₃-NBD) assay of α-GAL activity

The fluorescently labeled substrate *N*-dodecanoyl-nitrobenzoxadiazole-ceramide trihexoside (Gb₃-NBD) is less lipophilic than Gb₃, facilitating its use in *in-vitro* enzymatic reactions.

10 µL of 0.1 µg/µL Gb₃-NBD (in water with 10 % ethanol), and 5 µL of 0.2 mg/mL α-GAL were added to 85 µL of citrate-phosphate buffer at a pH of 4.6. The final α-GAL concentration was 10 µg/mL. The background or non-catalyzed reaction, without α-GAL, was composed of 90 µL of citrate-phosphate buffer at a pH of 4.6 with 10 µL of 0.1 µg/µL Gb₃-NBD (in water with 10 % ethanol). The reaction mixtures were incubated for 60 minutes at 37 °C. Following the incubation, 50 µL methanol was added to the reaction mixture, and the solutions were vortexed for 1 minute. 100 µL chloroform was then added, and the solutions were further vortexed for 1 minute. Water and organic solvents were removed under vacuum using a Speed Vac system. The residues were dissolved in 80 µL of chloroform:methanol (1:1). 30 µL of each sample was loaded on HPTLC (high performance thin layer chromatography) Silica Gel 60 plates (Merck) using a Linomat V system (CAMAG). HPTLC plates were developed using a chloroform:methanol:H₂O solution at a ratio of 100:42:6 as a solvent system. Plates were then allowed to dry and the substrate and product spots were visualized by irradiation under UV light at a wavelength of 365 nm.

### p-Nitrophenyl-α-D-galactopyranoside (p-NP-G) assay of α-GAL activity:

p-Nitrophenyl-α-D-galactopyranoside was used as a hydrolysis substrate for α-GAL activity assays. The assay buffer contained 20 mM citric acid, 30 mM sodium phosphate, 0.1 % BSA (bovine serum albumin) and 0.67 % ethanol at pH 4.6. The assay was performed in 96 well ELISA plates (Greiner). 50 µL of sample were incubated with 150 µL assay buffer and 30 µL substrate was added to obtain a final concentration of 8 mM *p*-nitrophenyl-α-D-galactopyranoside. The reaction mixture was incubated at 37 °C for 90 minutes. After 90 minutes, 100 µL of 1.98 M sodium carbonate was added to each well in order to terminate the reaction. The amount of reaction product was determined by measuring absorbance at 405 nm.

### Measurement of α-GAL stability in vitro:

The stability of α-GAL from various sources was determined by adding α-GAL to one of the following conditions:
1) simulated lysosomal conditions: citrate-phosphate buffer (20 mM citric acid, 30 mM sodium phosphate), pH 4.6, 37°C;
2) simulated physiological conditions: phosphate buffered saline (PBS), pH 7.4, 37 °C;
3) human plasma at 37 °C.

The α-GAL was added at a concentration of 1 µg/mL, as determined by the activity of α-GAL in the solution, and the solution was incubated at 37 °C. Samples of each solution were withdrawn at predetermined time points and the α-GAL activity was measured as described hereinabove. The value of the enzymatic activity immediately after addition of the tested α-GAL to each environment was defined as 100 %, and further activity results at the tested time points were calculated as a percentage of that initial activity.

### Pharmacokinetics of α-GAL:

Individual Fabry (α-Gal-A -/0) mice were placed in an illuminated plexiglass restraining device, and the enzyme was injected into the tail vein. Blood samples were obtained at the indicated times after injection by either tail bleed or retro-orbital eye bleed, using heparinized microhematocrit tubes. Plasma was diluted in 4-methylumbelliferyl-α-D-galactopyranoside activity buffer. A 4-methylumbelliferyl-α-D-galactopyranoside assay was performed as described above.

Terminal elimination half-life (T_{1/2}) was calculated based on plasma activity results. The terminal half-life (elimination half-life) is the time required for the plasma concentration to decrease by 50 % after pseudo-equilibrium of distribution has been reached. The terminal half-life was calculated from the terminal (log-linear) portion of the curve, by linear regression of time vs. log concentration [Toutain & Bousquet-Melou, J Vet Pharmacol Ther 2004, 27:427-39].

### Bio-distribution of α-GAL:

Fabry (α-Gal-A -/0) mice were injected intravenously (in the tail vein) with α-GAL at a dose of 2 mg/Kg. Tissues (livers, kidneys, hearts, and spleens) were harvested 2 hours, 24 hours, 3 days, 7 days, 14 days or 28 days post-injection of the enzyme. α-GAL levels in normal control mice and in saline-administered (untreated) Fabry mice were compared with the levels in Fabry mice that received exogenous α-GAL. To determine the α-GAL activity in tissues, thawed tissue samples were placed in 2 mL polypropylene tubes containing lysis buffer (28 mM citric acid, 44 mM dibasic sodium phosphate, 0.5 % sodium taurocholate, pH 4.4) as described in Oshima et al. [PNAS 1997, 94:2540-2544]. The samples were homogenized by use of a Tissuelyzer (Retsch MM400) for 10 minutes. Debris was pelleted by centrifugation at 4 °C, and the resulting supernatants were assayed for α-GAL activity by a 4-methylumbelliferyl-α-D-galactopyranoside assay, as described above. The same samples were also subjected to Western blot analysis.

### In vivo Gb₃ assay:

The end point efficacy of injected α-GAL was measured by assay of Gb₃ levels of the animal tissues, in order to determine whether Gb₃ levels were decreased by α-GAL activity.

To measure Gb₃ hydrolysis, neutral glycosphingolipids were extracted from target organs (e.g., liver, kidney, heart and spleen). 100 mg tissue samples were homogenized in 1 mL of 2:1 (v/v) chloroform:methanol and centrifuged for 20 minutes at 13,500 rpm. 62 µL of water was added to 1 mL homogenate to yield a solution of 20:10:2 chloroform:methanol:water. 10 µL pyridine was added to the homogenate to give a final pyridine concentration of 1 %. The sample was agitated for 24 hours at 48 °C. Solvents and water were removed under vacuum using a SpeedVac system. The sample was resuspended in 2.5 mL methanol and 250 µL of 1 M KOH in methanol was then added. The sample was then shaken for 2 hours at 37 °C. The saponification reaction was stopped by the addition of 10 µL of acetic acid. 2.5 mL chloroform was then added to the sample, followed by the addition of 2.5 mL cold water. The sample was vigorously shaken for 5 minutes and was allowed to rest for 5 minutes to allow phase separation. The upper phase, composed of methanol and water, was discarded, and the lower phase, composed of chloroform and methanol, was evaporated under vacuum (SpeedVac), and the residue was resuspended in 300 µL of 1:1 (v/v) chloroform:methanol for analysis of the glycosphingolipids by HPTLC.

Qualitative and semiquantitative analyses of tissue glycolipids were performed by high performance thin layer chromatography (HPTLC) (CAMAG, Switzerland). HPTLC analysis was performed on HPTLC silica gel 60 glass coated plates (Merck). Samples were loaded on the plates using a Linomat 5 system (CAMAG, Switzerland). Plates were developed using chloroform-methanol-water (60:35:4) as the solvent system. Neutral glycosphingolipids were detected with primuline spray reagent. Gb₃ was identified using porcine red blood cell Gb₃ (Matreya) as a standard, and quantified using a calibration curve of N-heptadecanoyl ceramide trihexoside (Matreya), a semisynthetic standard. Plates were visualized and relevant spots were quantified using a TLC Scanner III (CAMAG, Switzerland) supported by winCATS software (CAMAG, Switzerland).

### SDS-PAGE:

SDS-PAGE was carried out under reduced conditions using a Bio-Rad Criterion^{™} system and in-house casted 12 % acrylamide gel. The gel was stained by Coomassie Blue G250 stain.

### IEF (isoelectric focusing):

IEF was carried out using an Invitrogen Novex® mini-cell and precasted IEF gels having a pH range of 3-7 (Invitrogen). The gel was stained by Coomassie Blue G250.

### Mass spectrometry (MALDI-TOF):

MALDI-TOF was performed using a Bruker Reflex IV MALDI-ToF Mass-spectrometer system (Bruker-Franzen Analytik GmbH, Germany) and a sinapinic acid/trifluoroacetic acid (TFA) (0.1 % TFA/acetonitrile (2:1, v/v)) saturated matrix solution.

### EXAMPLE I

### In vitro stability of recombinant α-GAL

The *in vitro* stability of recombinant α-GAL was measured in various conditions as described hereinabove in the Materials and Methods Section. Plant recombinant human α-GAL-I, as well as Fabrazyme^{®} and Replagal^{®} commercial recombinant human α-GAL, were tested.

As shown in Figure 1, all of the tested types of α-GAL exhibited a loss of activity under simulated lysosomal conditions.

In addition, as shown in Figure 2, all of the tested types of α-GAL exhibited a loss of activity under simulated physiological conditions. As further shown therein, the presence of 100 mg/mL galactose partially protected the activity of plant recombinant α-GAL-1 under such conditions.

Similarly, as shown in Figure 3, all of the tested types of α-GAL exhibited a loss of activity in human plasma at 37 °C.

As shown in Figure 4, the presence of 100 mg/mL galactose partially protected the activity of plant recombinant α-GAL-I under simulated lysosomal conditions.

Size exclusion chromatography (SEC) experiments at lysosomal and neutral pH levels demonstrated changes in the protein structure (data not shown), while SDS-PAGE and Western blot analyses did not exhibit any degradation of the primary amino acid sequence (data not shown).

These results indicate that α-GAL loses activity under lysosomal conditions and physiological conditions due to alteration of the α-GAL protein structure, and that galactose partially prevents this loss of activity.

### EXAMPLE II

### Cross-linking of plant recombinant human α-GAL-I with bis-N-hydroxysuccinimidepoly(ethylene glycol) (bis-NHS-PEG) agents

Plant recombinant human α-GAL-I (prh-α-GAL-I) was cross-linked at 50:1, 100:1 and 200:1 molar ratios with bis-N-hydroxysuccinimide-poly(ethylene glycol) (bis-NHS-PEG) of various molecular weights, namely bis-NHS-PEG₅, bis-NHS-PEG₈ or bis-NHS-PEG₄₅ (bis-NHS-PEG with 2,000 Dalton PEG), the structures of which are shown in Figure 5.

The bis-NHS-PEG may attach to the protein at two sites on a protein (e.g., lysine residues), thereby forming cross-linking, or at one site on a protein. These two forms of attachment are depicted in Figure 6.

100 µg α-GAL-I in 28.5 µL of 2-(N-morpholino)ethanesulfonic acid (MES) buffer (25 mM, pH 6) was added to 13.5 µL of phosphate buffer (100 mM, pH 8) containing 100 mg/ml galactose.

α-GAL-I was cross-linked with bis-NHS-PEG₅ at 1:50, 1:100, and 1:200 protein: reagent molar ratios, by adding bis-NHS-PEG₅ in 8 µL DMSO to the α-GAL-I solution (27.4 µg α-GAL-I solution for a 1:50 molar ratio, 54.8 µg α-GAL-I solution for a 1:100 molar ratio, and 109.7 µg α-GAL-I solution for a 1:200 molar ratio).

α-GAL-I was cross-linked with bis-NHS-PEG₄₅ at 1:50, 1:100, and 1:200 protein: reagent molar ratios, by adding bis-NHS-PEG₄₅ in 8 µL DMSO to the α-GAL-I solution (103 µg α-GAL-I solution for a 1:50 molar ratio, 206 µg α-GAL-I solution for a 1:100 molar ratio, and 412 µg α-GAL-I solution for a 1:200 molar ratio) α-GAL-1 was crosslinked with bis-NHS-PEG₈ at 1:50, 1:100, and 1:200 protein: reagent molar ratios, by adding bis-NHS-PEG₈ in 11.5 µL DMSO to the α-GAL-I solution (37 µg α-GAL-I for a 1:50 molar ratio, 73 µg α-GAL-I solution for a 1:100 molar ratio, and 146 µg α-GAL-I solution for a 1:200 molar ratio).

After adding the bis-NHS-PEG agent to the α-GAL-I, the reactions were pipetted and agitated on an orbital shaker for 2 hours at room temperature.

In all reactions the excess of bis-NHS-PEG cross-linking reagent was removed by dialysis against saline (50KDa cut off).

The yield of dimer increased with increasing protein concentration and DMSO concentration, reaching up to 30 %.

The reaction products were analyzed by SDS-PAGE (sodium dodecyl sulfate-polyacrylamide gel electrophoresis), IEF (isoelectric focusing), Western blot, and MALDI-TOF mass spectrometry, as described hereinabove.

As shown in Figure 7, the standard native prh-α-GAL-I was observed as a monomer (having a molecular weight of 48 KDa) following gel electrophoresis, whereas following reaction of prh-α-GAL-I with bis-NHS-PEG, prh-α-GAL-I appeared primarily in the form of a dimer (with some monomer present), indicating that the two monomers were covalently linked by cross-linking with bis-NHS-PEG.

As is further shown in Figure 7, a higher proportion of monomeric prh-α-GAL-I was observed with the shorter cross-linkers, bis-NHS-PEG₅ and bis-NHS-PEG₈, than with the longer cross-linker bis-NHS-PEG₄₅. The bis-NHS-PEG₄₅ yielded a high proportion of cross-linked protein. These results indicate that the shorter cross-linkers are less effective at covalently linking monomers.

As is further shown in Figure 7, for each of the tested cross-linkers, the molecular weight of the monomeric portion of prh-α-GAL-I increased following reaction with the cross-linker. The increase in molecular weight was greater when a higher ratio of cross-linker to protein was used (e.g., 200:1), and when the molecular weight of the cross-linker was greater (e.g., bis-NHS-PEG₄₅). These results indicate that protein monomers which were not dimerized by cross-linking, were covalently attached to the bis-NHS-PEG cross-linker, i.e., the proteins were PEGylated.

The above results indicate that the use of higher molar excess of cross-linker to protein yields higher levels of α-GAL modification, including both cross-linking to form a dimer and PEGylation of the proteins. However, a molar ratio of 100:1 provided a high level of cross-linking, especially in the reactions using the bis-NHS-PEG₄₅ reagent, such that a molar ratio of 200:1 provided only a marginal addition to the cross-linking efficiency.

As shown in Figure 8, reacting prh-α-GAL-1 with bis-NHS-PEG reduced the isoelectric point (pI) of prh-α-GAL-I, thereby confirming that the bis-NHS-PEG is covalently attached to the prh-α-GAL-I. Attachment of bis-NHS-PEG to prh-α-GAL-I converts basic amine groups in lysine residues to neutral amide groups, thereby reducing the pI. The reduction in pI was more pronounced when a greater molar excess (e.g., 200:1) of bis-NHS-PEG was used, confirming the above results obtained with SDS-PAGE.

As is further shown in Figure 8, the pI is reduced more by bis-NHS-PEG₅ and bis-NHS-PEG₈ than by bis-NHS-PEG₄₅.

This result indicates that bis-NHS-PEG₅ and bis-NHS-PEG₈ are more likely than bis-NHS-PEG₄₅ to result in PEGylation in which only one terminus of the cross-linker is attached to α-GAL. A cross-linker attached to α-GAL at only one terminus is more effective at reducing the pI because such a cross-linker comprises an acidic carboxylic (-CO₂H) group at the non-attached terminus, in addition to converting a lysine amine group to an amide group at the attached terminus.

As shown in Figure 9, reacting prh-α-GAL-I with bis-NHS-PEG₄₅ cross-linker increased the molecular weight of the prh-α-GAL-I dimer from 97 KDa to 113 KDa, as determined by MALDI-TOF mass spectrometry. The increase in molecular weight indicates an addition of approximately 8 molecules of bis-NHS-PEG₄₅ to the prh-α-GAL-I dimer.

As shown in Figure 10, reacting prh-α-GAL-I with a bis-NHS-PEG₈ cross-linker increased the molecular weight of the prh-α-GAL-I dimer from 97 KDa to 104 KDa, as determined by MALDI-TOF mass spectrometry. The increase in molecular weight indicates an addition of approximately 10 molecules of bis-NHS-PEG₈ to the prh-α-GAL-I dimer.

### EXAMPLE III

### Activity of cross-linked plant recombinant human α-GAL-I

In order to determine whether the Cross-linked plant recombinant α-GAL-I (prh-α-GAL-I) described in Example II retained enzymatic activity, the cross-linked prh-α-GAL-I was assayed for its enzymatic activity using the 4-methylumbelliferyl-α-D-galactopyranoside assay described hereinabove.

As shown in Table 1 below, prh-α-GAL-I which was reacted with bis-NHS-PEG₅, bis-NHS-PEG₈ or bis-NHS-PEG₄₅ at molar excesses of 50:1, 100:1 and 200:1 bis-NHS-PEG reagent in all cases exhibited a level of enzymatic activity similar to that of native prh-α-GAL-I. As shown therein, both moderate decreases and moderate increases in activity were observed in some cases, which may be a result of formulation effects. These results indicate that the cross-linking did not reduce the activity of prh-α-GAL-I.

**Table 1: Activity results of cross-linked plant recombinant human α-GAL I**

| **Sample** | **Reagent** | **Molar excess** | **Activity mg/mL** |
|---|---|---|---|
| **standard** | - | - | 2 |
| **1** | Bis-NHS-PEG₅ | 50:1 | 2.25 |
| **2** | Bis-NHS-PEG₅ | 100:1 | 1.30 |
| **3** | Bis-NHS-PEG₅ | 200:1 | 1.24 |
| **4** | Bis-NHS-PEG₄₅ | 50:1 | 2.82 |
| **5** | Bis-NHS-PEG₄₅ | 100:1 | 2.76 |
| **6** | Bis-NHS-PEG₄₅ | 200:1 | 3.48 |
| **7** | Bis-NHS-PEG₈ | 50:1 | 2.18 |
| **8** | Bis-NHS-PEG₈ | 100:1 | 2.43 |
| **9** | Bis-NHS-PEG₈ | 200:1 | 1.82 |

The activity of the bis-NHS-PEG₄₅ cross-linked prh-α-GAL-I was further verified using the *N*-dodecanoyl-NBD-ceramide trihexoside assay described, hereinabove, which assays the activity of α-GAL towards its natural substrate, ceramide trihexoside (Gb₃). Replagal^{®} mammalian recombinant human α-GAL was assayed for comparison.

As shown in Figure 11, following incubation of the cross-linked plant recombinant human α-GAL-I with the fluorescent substrate, almost all substrate was converted to the product, *N*-dodecanoyl-nitrobenzoxadiazole-lactosyl ceramide, similarly to the reaction catalyzed by the mammalian recombinant α-GAL (Replagal^{®}). This result confirms that the cross-linking did not impair the enzymatic hydrolytic efficiency of the prh-α-GAL-I, using a close analog of the natural substrate.

### EXAMPLE IV

### In vitro stability of cross-linked plant recombinant human α-GAL-I

The *in vitro* stability of the cross-linked plant recombinant human α-GAL-I (prh-α-GAL-I), obtained as described in Example II, was measured in various conditions as described hereinabove in the Materials and Methods Section. The stability of Fabrazyme^{®} and Replagal^{®} commercial recombinant human α-GALs was measured for comparison.

As shown in Figures 12A-12C, the stability of plant recombinant human α-GAL-I under simulated lysosomal conditions was enhanced by cross-linking with bis-NHS-PEG₅ (Figure 12A), bis-NHS-PEG₈ (Figure 12B) and bis-NHS-PEG₄₅ (Figure 12C). As further shown therein, the stability of the cross-linked prh-α-GAL-I over the course of one week compared favorably to the stability of the commercial recombinant human α-GAL. After a small decrease in residual activity during the first 24 hours, the cross-linked prh-α-GAL-I maintained activity, even after 10 days. The initial decrease in activity, observed during the first 24 hours, may reflect the portion of plant recombinant human α-GAL-I that did not undergo cross-linking.

As further shown in Figures 12A-12C, prh-α-GAL-I cross-linked by bis-NHS-PEG₄₅ exhibited the highest stability under simulated lysosomal conditions.

The stability of plant recombinant human α-GAL-I in human plasma at 37 °C was also enhanced by cross-linking with bis-NHS-PEG₄₅ (data not shown).

These results indicate that cross-linking α-GAL as described herein can increase the efficacy of α-GAL *in vivo* by increasing the stability of α-GAL in lysosomes, thereby allowing α-GAL to act for a longer period of time in the lysosomes, and by increasing the stability of α-GAL in the blood, thereby increasing the circulatory half-life of α-GAL.

### EXAMPLE V

### In vivo pharmacokinetics and bio-distribution of cross-linked plant recombinant human α-GAL:I

The pharmacokinetics and bio-distribution of plant recombinant human α-GAL-I (prh-α-GAL-I) cross-linked with bis-NHS-PEG₄₅ or bis-NHS-PEG₈ as described in Example II was determined in Fabry mice injected with 2 mg/Kg of α-GAL, as described hereinabove in the Materials and Methods Section. The pharmacokinetics and bio-distribution of non-cross-linked plant recombinant human α-GAL-I and of Replagal^{®} recombinant human α-GAL was determined for comparison. Blood samples were collected for pharmacokinetic analysis 1, 3, 5,10, 20, 30, 40, 60 and 120 minutes post-injection. For each type of α-GAL, the treatment group consisted of six mice.

As shown in Table 2 below, cross-linking prh-α-GAL-I with bis-NHS-PEG₈ and with bis-NHS-PEG₄₅ increased the circulatory terminal half-life of plant recombinant human α-GAL-I, with the latter exhibiting a more pronounced effect.

**Table 2: Circulatory terminal half lives of recombinant α-GAL**

| **α-GAL sample** | **t_{1/2} (minutes)** |
|---|---|
| Replagal^{®} mammalian recombinant human α-GAL | 8.1 |
| Plant recombinant human α-GAL-I | 4.8 |
| Plant recombinant human α-GAL I cross-linked with bis-NHS-PEG₈ | 6.2 |
| Plant recombinant human α-GAL-I cross-linked with bis-NHS-PEG₄₅ | 90 |

As shown in Figure 13 and in Table 2, the terminal half-life of prh α-GAL-1 cross-linked by bis-NHS-PEG₄₅ was considerably greater than the terminal half-life of Replagal^{®} α-GAL.

As further shown in Figure 13, the activity of plant recombinant human α-GAL-I cross-linked with bis-NHS-PEG₄₅ at 20 minutes was about 40 % of the activity at 1 minute. Furthermore, the cross-linked prh-α-GAL-I exhibited an active plasma presence even 4 hours post-injection.

These results indicate that the cross-linked prh-α-GAL-I remains active *in vivo* for a relative long time, which can allow the enzyme to reach additional tissues and organs.

As shown in Figures 14A and 14B, the levels of plant recombinant α-GAL-I cross-linked with bis-NHS-PEG₈ and bis-NHS-PEG₄₅ in the spleens of Fabry mice 2 hours post-injection were considerably higher than those of non-cross-linked plant recombinant α-GAL-I as well those of Replagae mammalian recombinant α-GAL. As further shown therein, levels of prh-α-GAL-I cross-linked with bis-NHS-PEG₄₅ were higher than levels of prh-α-GAL-I cross-linked with bis-NHS-PEG₈. The Western blot analyses (Figure 14B) are consistent with the bio-distribution results obtained by assaying α-GAL enzymatic activity (Figure 14A).

As shown in Figure 15A and 15B, the levels of plant recombinant α-GAL-I cross-linked with bis-NHS-PEG₈ and bis-NHS-PEG₄₅ in the livers of Fabry mice 2 hours post-injection were considerably higher than those of non-cross-linked plant recombinant α-GAL-I, but lower than levels of Replagal^{®} mammalian recombinant α-GAL in the liver. As further shown therein, levels of prh-α-GAL-I cross-linked with bis-NHS-PEG₄₅ were slightly higher than levels of prh-α-GAL-I cross-linked with bis-NHS-PEG₈. The Western blot analyses (Figure 15B) are consistent with the bio-distribution results obtained by assaying α-GAL enzymatic activity (Figure 15A).

Lower levels of α-GAL in the liver may be therapeutically advantageous, as about 95 % of the recovered enzyme in enzyme replacement therapy is typically found in the liver, and hence high levels of recombinant α-GAL in the liver indicate lower levels of exogenous α-GAL in target organs, such as heart and kidneys.

As shown in Figure 16, the levels of plant recombinant α-GAL-I cross-linked with bis-NHS-PEG₈ and bis-NHS-PEG₄₅ in the hearts of Fabry mice 2 hours post-injection were higher than those of non-cross-linked plant recombinant α-GAL-I. As further shown therein, levels of prh-α-GAL-I cross-linked with bis-NHS-PEG₄₅ were higher than levels of prh-α-GAL-I cross-linked with bis-NHS-PEG₈, as well as levels of Replagal^{®} mammalian recombinant α-GAL.

As shown in Figure 17, the levels of plant recombinant α-GAL-I cross-linked with bis-NHS-PEG₈ and bis-NHS-PEG₄₅ in the kidneys of Fabry mice 2 hours post-injection were higher than those of non-cross-linked plant recombinant α-GALI. As further shown therein, levels of prh-α-GAL-I cross-linked with bis-NHS-PEG₄₅ were higher than levels of prh-α-GAL-I cross-linked with bis-NHS-PEG₈, as well as levels of Replagal® mammalian recombinant α-GAL.

Similarly, as shown in Figures 18-21, the levels of plant recombinant α-GAL-I cross-linked with bis-NHS-PEG₄₅ were higher than the levels of non-cross-linked plant recombinant α-GAL-I in the spleen (Figure 18), liver (Figure 19), heart (Figure 20) and kidneys (Figure 21) of Fabry mice, for up to 7 days post-injection. As further shown therein, the levels of plant recombinant α-GAL-I cross-linked with bis-NHS-PEG₄₅ were higher than the levels of Replagal^{®} mammalian recombinant α-GAL in the spleen, heart and kidneys.

These results indicate that α-GAL cross-linked with bis-NHS-PEG, particularly bis-NHS-PEG₄₅, exhibits enhanced uptake into organs, including the kidney and heart, which are major target organs in the treatment of Fabry disorder. These results are consistent with the increased circulatory half-life and enhanced stability of cross-linked α-GAL.

### EXAMPLE VI

### Cross-linking of mammalian recombinant human α-GAL with bis-N-hydroxysuccinimide poly(ethylene glycol) (bis-NHS-PEG)

In order to confirm the advantageous effects of cross-linking described hereinabove, Replagal^{®} mammalian recombinant human α-GAL, which is produced from human fibrosarcoma line HT-1080, was cross-linked.

333 µL of phosphate buffer (100 mM, pH 8) with 100 mg/ml D-(+)-galactose was added to 3.8 mg bis-NHS-PEG₄₅ in 151 µL of DMSO solution (25 mg/ml) and 1.8 mg of Replagal^{®} recombinant human α-GAL in 130 µL citrate buffer (25 mM, pH 6). Replagal^{®} α-GAL concentration was determined by an activity assay. The reaction mixture was agitated using an orbital shaker for 2 hours at room temperature. The excess of bis-NHS-PEG₄₅ cross-linking reagent was removed by dialysis against saline using a Vivaspin 6 concentrator with a cut-off of 50 KDa. The α-GAL activity of the cross-linked Replagal^{®} α-GAL indicated that the α-GAL concentration was 3 mg/mL.

The reaction products were analyzed by SDS-PAGE (sodium dodecyl sulfate-polyacrylamide gel electrophoresis), IEF (isoelectric focusing), and MALDI-TOF mass spectrometry, as described hereinabove.

As shown in Figure 22, the standard native Replagal^{®} α-GAL was observed as a monomer following gel electrophoresis, whereas following reaction of Replagal^{®} α-GAL with bis-NHS-PEG₄₅, the α-GAL appeared in the form of a dimer, indicating that the two monomers were covalently linked by cross-linking with bis-NHS-PEG.

As shown in Figure 23, reacting Replagal^{®} α-GAL with bis-NHS-PEG₄₅ reduced the isoelectric point (pI) of the α-GAL, thereby confirming that the bis-NHS-PEG is covalently attached to the α-GAL.

As shown in Figure 24, reacting Replagal^{®} α-GAL with bis-NHS-PEG₄₅ cross-linker increased the molecular weight of the Replagal^{®} α-GAL dimer from 103.0 KDa to 121.3 KDa, as determined by MALDI-TOF mass spectrometry. The increase in molecular weight indicates an addition of approximately 9-10 molecules of bis-NHS-PEG₄₅ to the α-GAL dimer, which is similar to results described hereinabove for prh-α-GAL-I.

### EXAMPLE VII

### Activity of cross-linked mammalian recombinant human α-GAL

In order to determine whether the cross-linking of mammalian recombinant α-GAL described in Example VI affected enzymatic activity, the cross-linked α-GAL was assayed for its enzymatic activity using a *p*-nitrophenyl-α-D-galactopyranoside (*p*NP-G) assay, according to the procedures described hereinabove.

As shown in Figure 25 and in Table 3 below, mammalian recombinant human α-GAL which was cross-linked with bis-NHS-PEG₄₅ exhibited parameters of enzymatic activity which are very similar to those of native mammalian recombinant human α-GAL. These results indicate that the cross-linking did not significantly affect the activity or the catalytic machinery and mechanism of the mammalian recombinant human α-GAL.

**Table 3: Activity results of cross-linked mammalian recombinant human α-GAL**

| **Sample** | ***K*_{M} (µM)** | ***V*ₘₐₓ (µM/minute)** | ***k*_{cat} (second⁻¹)** | ***k*_{cat}/*K*_{M} (second⁻¹*µM⁻¹)** |
|---|---|---|---|---|
| Replagal^{®} α-GAL | 3212±98 | 4.20±0.05 | 67.2±1 | 0.0209±0.001 |
| Cross-linked Replagal^{®} α-GAL | 3419±162 | 4.43±0.07 | 70.9±1 | 0.0210±0.001 |

### EXAMPLE VIII

### In vitro stability of cross-linked mammalian recombinant human α-GAL

The *in vitro* stability of the cross-linked Replagal^{®} mammalian recombinant human α-GAL, obtained as described in Example VI, was measured in various conditions as described hereinabove in the Materials and Methods Section. The stability of non-cross-linked Replagal^{®} α-GAL was measured for comparison, in order to assess the effect of cross-linking.

As shown in Figures 26A and 26B, the stability of mammalian recombinant human α-GAL under both simulated lysosomal conditions (Figure 26A) and in human plasma (Figure 26B) was considerably enhanced by cross-linking with bis-NHS-PEG₄₅. The cross-linked mammalian recombinant human α-GAL exhibited higher stability under simulated lysosomal conditions than in plasma.

These results indicate that the cross-linking of α-GAL as described herein can stabilize recombinant α-GAL from multiple sources and expression platforms.

### EXAMPLE IX

### In vivo pharmacokinetics and bio-distribution of cross-linked mammalian recombinant human α-GAL

The pharmacokinetics and bio-distribution of the cross-linked mammalian recombinant human α-GAL described in Example VI was determined by measuring α-GAL activity in the spleen, liver, heart and kidneys of Fabry mice 2 hours, 7, 14, and 28 days post-injection, as well as Gb₃ levels in these organs, as described hereinabove in the Materials and Methods section. The bio-distribution of non-cross-linked Replagal^{®} mammalian recombinant human α-GAL was determined for comparison.

As shown in Figures 27A-27D, the levels of cross-linked mammalian recombinant α-GAL in the spleens (Figure 27A), liver (Figure 27B), heart (Figure 27C) and kidneys (Figure 27D) of Fabry mice were considerably higher than those of non-cross-linked mammalian recombinant α-GAL.

As shown in Figures 28A-28D, cross-linked mammalian recombinant α-GAL decreased Gb₃ levels in the heart (Figure 28A), kidney (Figure 28B), liver (Figure 28C) and spleen (Figure 28D) of Fabry mice, over the course of 28 days post-injection. Cross-linked mammalian recombinant α-GAL decreased Gb₃ levels to a greater extent than did non-cross-linked recombinant α-GAL in the kidney (Figure 28B) and spleen (Figure 28D) of Fabry mice, and to about the same extent as non-cross-linked mammalian recombinant α-GAL in the heart (Figure 28A) and liver (Figure 28C).

These results indicate that cross-linking with bis-NHS-PEG results in considerably enhanced uptake of recombinant α-GAL from a variety of sources and expression platforms into organs, including the kidney and heart, which are major target organs in the treatment of Fabry disorder. These results further indicate that cross-linking with bis-NHS-PEG results in a more substantial decrease of Gb₃ levels in organs.

### EXAMPLE X

### Cross-linking of plant recombinant human α-GAL-II with bis N-hydroxysuccinimide poly(ethylene glycol) (bis-NHS-PEG)

Plant recombinant human α-GAL-II (prh-α-GAL-II), which lacks the amino acids EF present in the N-terminus of prh-α-GAL-I, was cross-linked with bis-NHS-PEG₄₅, bis-NHS-PEG₂₁, or bis-NHS-PEG₆₈ at a 200:1 molar ratio of bis-NHS-PEG to α-GAL, according to the protocol described in Example II.

The prh-α-GAL-II retained its biological activity following cross-linking with bis-NHS-PEG (data not shown).

The reaction products were analyzed by SDS-PAGE (sodium dodecyl sulfate-polyacrylamide gel electrophoresis) and MALDI-TOF mass spectrometry, as described hereinabove.

As shown in Figures 29A-29B, the standard native prh-α-GAL-II was observed as a monomer following gel electrophoresis, whereas following reaction of prh-α-GAL-II with bis-NHS-PEG₄₅ or bis-NHS-PEG₂₁ (Figure 29A), or with bis-NHS-PEG₆₈ (Figure 29B), prh-α-GAL-II appeared primarily in the form of a dimer (with some monomer present), indicating that the two monomers were covalently linked by cross-linking with a bis-NHS-PEG cross-linking agent.

As is further shown in Figures 29A-29B, for each of the tested cross-linkers, the molecular weight of the monomeric portion of prh-α-GAL-II increased following reaction with the cross-linker. The increase in molecular weight was greater for bis-NHS-PEG₄₅ than for bis-NHS-PEG₂₁ (Figure 29A), and was greatest for bis-NHS-PEG₆₈ (compare Figure 29A with Figure 29B). These results indicate that the monomers which were not dimerized by cross-linking, were covalently attached to the bis-NHS-PEG cross-linker, i.e., the proteins were PEGylated.

As shown in Figures 30A-30C, reacting prh-α-GAL-II with bis-NHS-PEG₂₁ cross-linker increased the molecular weight of the prh-α-GAL-II dimer from 95 KDa (Figure 30A) to 109 KDa (Figure 30B), while reacting prh-α-GAL-11 with bis-NHS-PEG₄₅ cross-linker increased the molecular weight of the prh-α-GAL-II dimer to 114 KDa (Figure 30C), as determined by MALDI-TOF mass spectrometry. The increase in molecular weight indicates an addition of approximately 13 molecules of bis-NHS-PEG₂₁, or approximately 9 molecules of bis-NHS-PEG₄₅, to the prh-α-GAL-II dimer.

### EXAMPLE XI

### In vitro stability of cross-linked plant recombinant human α-GAL-II

The *in vitro* stability of the cross-linked plant recombinant human α-GAL-II (prh-α-GAL-II) obtained as described in Example X was measured under various conditions as described hereinabove in the Materials and Methods Section. The stability of Replagal^{®} commercial recombinant human α-GAL was measured for comparison.

As shown in Figures 31A-31D, the stability of plant recombinant human α-GAL-II was enhanced by cross-linking with either bis-NHS-PEG₆₈ (Figures 31B and 31D), bis-NHS-PEG₄₅ (Figures 31A-31D) or bis-NHS-PEG₂₁ (Figures 31A and 31C), under both simulated lysosomal conditions (Figures 31A and 31B) and in human plasma (Figures 31C and 31D). The different cross-linkers enhanced the stability of prh-α-GAL-II to comparable extents. As further shown therein, the stability of the cross-linked prh-α-GAL-II was greater than the stability of the Replagal^{®} recombinant human α-GAL. The cross-linked prh-α-GAL-II exhibited higher stability under simulated lysosomal conditions as well as under plasma conditions.

As further shown in Figures 31A-31D, non-cross-linked prh-α-GAL-II is considerably more stable than non-cross-linked prh-α-GAL-I (see Figures 1 and 3 for comparison), under both simulated lysosomal conditions (Figures 1 and 31A-31B) and in human plasma (Figures 3 and 31C-31D), although prh-α-GAL-II still exhibits some instability.

These results indicate that the cross-linking of α-GAL as described herein can stabilize different types of α-GALs.

### EXAMPLE XII

### In vivo pharmacokinetics and bio-distribution of cross-linked plant recombinant human α-GAL-II

The pharmacokinetics and bio-distribution of the PEG₄₅-cross-linked and PEG₂₁-cross-linked plant recombinant human α-GAL-II (prh-α-GAL-II), described in Example X, was determined by measuring α-GAL activity in plasma and organs as described hereinabove in the Materials and Methods section. The pharmacokinetics and bio-distribution of non-cross-linked Replagal^{®} mammalian recombinant human α-GAL were determined for comparison.

Blood samples were collected for pharmacokinetic analyses at 1, 5, 10, 30, 60, 120,240,480 and 1440 minutes after injection of Fabry mice with 1 mg/Kg of α-GAL.

Bio-distribution of α-GAL was determined by harvesting the liver, kidneys, heart and spleen of Fabry mice 2 hours, 7 days, 14 days and 28 days post-injection with 2 mg/Kg α-GAL.

was shown in Figures 32A and 32B and in Table 4, cross-linking of prh-α-GAL-II with bis-NHS-PEG₄₅ considerably increased the circulatory terminal half-life of prh-α-GAL-II, yielding a circulatory half-life considerably greater than that of mammalian recombinant α-GAL or of non-cross-linked prh-α-GAL-II.

**Table 4: Circulatory terminal half-life of recombinant α-GAL**

| **Test item** | **t_{1/2}(min)** |
|---|---|
| Replagal^{®} α-GAL | 13.3 |
| plant recombinant alpha-GAL-II | 4.8 |
| plant recombinant alpha-GAL-11 cross-linked with bis-NHS-PEG₄₅ | 581.6 |

As shown in Figures 33A-33L, cross-linking of prh-α-GAL-II with bis-NHS-PEG₄₅ increased the uptake of prh-α-GAL-II in heart (Figure 33A), kidney (Figure 33B), liver (Figure 33C) and spleen (Figure 33D) of Fabry mice, although to a lesser degree in the liver.

As shown in Figures 33E-33L, cross-linking of prh-α-GAL-II with bis-NHS-PEG₂₁ also increased the uptake of prh-α-GAL-II in heart (Figures 33E and 33I), kidney (Figures 33F and 33J), liver (Figures 33G and 33K) and spleen (Figures 33H and 33L) of Fabry mice, although such an increase was not always evident after only 2 hours.

As further shown therein, the levels of cross-linked prh-α-GAL-II were greater than the levels of mammalian recombinant α-GAL in the heart (Figures 33A, 33E and 33I), kidney (Figures 33B, 33F and 33J), and spleen (Figures 33D, 33H and 33L) of Fabry mice, and lower than the levels of mammalian recombinant α-GAL in the liver (Figures 33C, 33G and 33K).

These results indicate that cross-linked prh-α-GAL-II exhibits considerably enhanced activity of α-GAL in the plasma and in various organs, particularly in organs other than the liver.

### EXAMPLE XIII

### Effect of pH on activity of plant recombinant human α-GAL

The pH of the environment has a significant effect on the stability and kinetics of lysosomal enzymes such α-GAL. The pH may affect binding of substrate to the enzyme. The pH can also affect the protonation or deprotonation of catalytic groups, such as carboxyl or amino groups, which are part of the enzyme's active site, and thus affect the kinetic behavior of the enzyme. The stability of the tertiary or quaternary structure of enzymes is also pH-dependent, and affects the velocity of the enzymatic reaction, especially at extremely acidic or alkaline pH values.

The activity of PEG₄₅-cross-linked and non-cross-linked plant recombinant human α-GAL-II was determined at various pH values using a *p*NP-G substrate, in order to examine the pH-dependence of α-GAL activity, and the effect of cross-linking thereon. The measurements were performed in solutions of 20 mM citrate and 30 mM sodium phosphate.

The kinetic parameters characterizing α-GAL activity at various pH values are summarized in Table 5 below, and in Figures 34A-34C.

As shown in Figures 34A-34C, cross-linking of the α-GAL-II increased the Vₘₐₓ (Figure 34A) and *k*_{cat} (Figure 34C) parameters, and did not have a significant effect on the *K*_{M} parameter (Figure 34B).

**Table 5: Activity results of non-cross-linked plant recombinant human α-GAL-II (prh-α-GAL-II) and PEG₄₅-cross-linked plant recombinant human α-GAL II (prh-α-GAL-II-CL45) at various pH values**

| pH | Sample | *K*_{M} (µM) | *V*ₘₐₓ (µM/minute) | *k*_{cat} (second⁻¹) | *k*_{cat} /*K*_{M} (second⁻¹* µM⁻¹) |
|---|---|---|---|---|---|
| 2.8 | prh-α-GAL-II | 15216 | 0.57 | 9.04 | 0.0006 |
| | prh-α-GAL-II-CL45 | 13618 | 0.90 | 14.37 | 0.0011 |
| 3.2 | prh-α-GAL-II | 11476 | 0.55 | 8.85 | 0.0008 |
| | prh-α-GAL-II-CLA5 | 8489 | 1.34 | 21.44 | 0.0025 |
| 3.6 | prh-α-GAL-II | 11147 | 1.76 | 28.16 | 0.0025 |
| | prh-α-GAL-II-CL45 | 4699 | 2.23 | 35.68 | 0.0076 |
| 4.04 | prh-α-GAL-II | 5709 | 1.98 | 31.68 | 0.0055 |
| | prh-α-GAL-II-CL45 | 3207 | 2.74 | 43.76 | 0.0136 |
| 4.4 | prh-α-GAL-II | 4596 | 2.40 | 38.40 | 0.0084 |
| | prh-α-GAL-II-CLA5 | 3122 | 3.22 | 51.57 | 0.0165 |
| 4.8 | prh-α-GAL-II | 4531 | 2.32 | 37.12 | 0.0082 |
| | prh-α-GAL-II-CL45 | 3345 | 2.95 | 47.23 | 0.0141 |
| 5.29 | prh-α-GAL-II | 6793 | 2.06 | 32.99 | 0.0049 |
| | prh-α-GAL-II-CL45 | 3973 | 2.78 | 44.48 | 0.0112 |
| 5.66 | prh-α-GAL-II | 10396 | 1.75 | 28.05 | 0.0027 |
| | prh-α-GAL-II-CL45 | 4883 | 2.70 | 43.20 | 0.0088 |
| 6.09 | prh-α-GAL-II | 11357 | 1.44 | 23.04 | 0.0020 |
| | prh-α-GAL-II-CL45 | 8336 | 1.54 | 24.59 | 0.0030 |
| 6.4 | prh-α-GAL-II | 21046 | 1.32 | 21.12 | 0.0010 |
| | prh-α-GAL-II-CL45 | 16844 | 1.46 | 23.36 | 0.0014 |
| 6.76 | prh-α-GAL-II | 25188 | 1.12 | 17.92 | 0.0007 |
| | prh-α-GAL-II-CL45 | 18313 | 1.14 | 18.24 | 0.0010 |
| 7.36 | prh-α-GAL-II | _ | _ | _ | _ |
| | prh-α-GAL-II-CL45 | 32692 | 0.52 | 8.37 | 0.0003 |

The enhancement of the *V*ₘₐₓ and *k*_{cat} parameters indicates an increase in catalytic activity. This increase is particularly significant at pH values of at least about 7, where the catalytic activity of non-cross-linked α-GAL-II is negligible.

*K*_{M} is a kinetic parameter associated with enzyme/substrate affinity. The absence of a significant effect of cross-linking on *K*_{M} values indicates that the cross-linking has no significant effect on α-GAL affinity to the *p*NP-G substrate.

### EXAMPLE XIV

### Effect of PEGylation on stability of α-GAL

The effect of PEGylation *per se* on α-GAL stability was ascertained, in order to determine whether the stabilizing effect of PEG cross-linkers is due to the properties of PEG or due to the cross-linking.

Plant recombinant human α-GAL-I was reacted with N-hydroxysuccinimide (NHS)-activated methoxy-capped PEGs with different molecular weights (2, 5, and 10 KDa). Such PEG reagents have a single NHS group, and consequently PEGylate the protein without forming cross-linking. The reaction products were analyzed by SDS-PAGE.

As shown in Figure 35, the methoxy-capped PEGylating agents PEGylated the α-GAL (visible as an increase in molecular weight of the α-GAL), but did not substantially generate α-GAL dimers, indicating that the α-GAL was not cross-linked.

As shown in Figures 36A and 36B, PEGylating plant recombinant human α-GAL-1 without forming cross-linking did not substantially increase the stability of the plant recombinant α-GAL, under either simulated lysosomal conditions (Figure 36A) or in human plasma (Figure 36B).

These results indicate that the stabilizing effect of the cross-linking described hereinabove is not a result of PEGylation *per se.*

### EXAMPLE XV

### Effect of PEG chain length on activity of cross-linked α-GAL

In order to assess the effect of the chain length of PEG cross-linkers on α-GAL activity, plant recombinant human α-GAL-I was cross-linked with bis-NHS-PEG₂, bis-NHS-PEG₄, bis-NHS-PEG₆₈ and bis-NHS-PEG₁₅₀ agents, using essentially the same procedures as described in Example II (PEG₆₈ and PEG₁₅₀ are approximate chain lengths). The α-GAL-I was cross-linked at 50:1, 100:1 and 200:1 bis-NHS-PEG:α-GAL molar ratios. The reaction products were analyzed by SDS-PAGE, as described hereinabove. α-GAL-I cross-linked with bis-NHS-PEG₄₅ as described in Example II was also analyzed for comparison.

As shown in Figure 37, SDS-PAGE analysis showed that all of the bis-NHS-PEG agents cross-linked the α-GAL so as to result in a covalently cross-linked dimer, and that cross-linking was more efficient when a 200:1 molar ratio was used.

The enzymatic activity of the cross-linked α-GAL-I was then determined as described in Example III. The results are summarized in Table 6 below.

**Table 6: Activity results of cross-linked plant recombinant human α-GAL-I**

| Reagent | Molar ratio (reagent: α-GAL-I) | Expected α-GAL activity [mg/mL] | Measured α-GAL activity [mg/mL] |
|---|---|---|---|
| 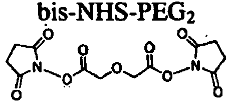 | 50:1 | 2 | 1.159 |
| | 100:1 | 2 | 1.001 |
| | 200:1 | 2 | 0.970 |
| bis-NHS-PEG₄ | 50:1 | 2 | 1.399 |
| | 100:1 | 2 | 1.333 |
| | 200:1 | 2 | 1.048 |
| bis-NHS-PEG₆₈ | 50:1 | 2 | 1.822 |
| | 100:1 | 2 | 2.252 |
| | 200:1 | 2 | 2.425 |
| bis-NHS-PEG₁₅₀ | 50:1 | 2 | 1.804 |
| | 100:1 | 2 | 2.031 |
| | 200:1 | 2 | 1.825 |

As shown in Table 6, cross-linking with PEG₂ and PEG₄ moderately reduced α-GAL activity (by approximately 30-50 %), whereas cross-linking with longer PEG chains did not significantly affect α-GAL activity.

These results indicate that cross-linking with PEG chains longer than PEG₄ is advantageous in terms of preserving activity of the cross-linked α-GAL.

### EXAMPLE XVI

### Cross-linking of α-GAL using bis-COOH-PEG agents

As an alternative to the above-described cross-linking of α-GAL using pre-prepared (e.g., commercially available) bis-NHS-PEG agents, α-GAL was cross-linked with bis-COOH-PEG agents by activating the carboxyl (i.e., COOH) groups in situ shortly before the cross-linking reaction was effected.

Bis-COOH-PEG₁₂, bis-COOH-PEG₂₈ and bis-COOH-PEG₄₅ were each activated by being reacted with 1.1 molar equivalents per carboxyl group (i.e., 2.2 molar equivalents per bis-COOH-PEG) of both NHS (N-hydroxysuccinimide) and EDC (1-ethyl-3-(3-dimethylaminopropyl) carbodiimide). The reaction mixture was then shaken in DMSO for 30 minutes at room temperature. The activated bis-COOH-PEG, which is essentially bis-NHS-PEG, was then reacted with plant recombinant human α-GAL-I at molar ratios of 50:1, 100:1 and 200:1, as described in Example II. The reaction products were analyzed by SDS-PAGE, as described hereinabove. α-GAL-I cross-linked with bis-NHS-PEG₄₅ as described in Example II was also analyzed for comparison.

As shown in Figure 38, SDS-PAGE analysis showed that all of the bis-COOH-PEG agents cross-linked the α-GAL to some extent, but that cross-linking was more efficient when a 200:1 molar ratio was used.

The enzymatic activity of the cross-linked α-GAL-1 was then determined as described in Example III. The results are summarized in Table 7 below.

**Table 7: Activity results of cross-linked plant recombinant human α-GAL-1**

| Reagent | Molar ratio (reagent: α-GAL-I) | Expected α-GAL activity [mg/mL] | Measured α-GAL activity [mg/mL] |
|---|---|---|---|
| Bis-HOOC-PEG₁₂ | 50:1 | 1.5 | 1.236 |
| | 100:1 | 1.5 | 1.304 |
| | 200:1 | 1.5 | 1.404 |
| Bis-HOOC-PEG₂₈ | 50:1 | 1.5 | 1.326 |
| | 100:1 | 1.5 | 1.371 |
| | 200:1 | 1.5 | 1.460 |
| Bis-HOOC-PEG₄₅ | 50:1 | 1.5 | 1.349 |
| | 100:1 | 1.5 | 1.541 |
| | 200:1 | 1.5 | 1.628 |

As shown in Table 7, cross-linking with each of the tested bis-COOH-PEG agents resulted in α-GAL with about the expected activity.

These results indicate that cross-linking bis-COOH-PEG agents does not reduce α-GAL activity in comparison to cross-linking with bis-NHS-PEG agents.

These results further confirm the above-described findings that cross-linking with PEG chains longer than PEG₄ does not significantly reduce the activity of the cross-linked α-GAL.

### EXAMPLE XVII

### Effect of length and type of cross-linking agent on in vitro stability of cross-linked plant recombinant human α-GAL-I

In order to further characterize the effect of chain length on cross-linked α-GAL stability, and to compare the stability of α-GAL cross-linked with bis-COOH-PEG agents (e.g., as described in Example XVI) with that of α-GAL cross-linked with bis-NHS-PEG agents, the *in vitro* stability of plant recombinant human α-GAL-I (prh-α-GAL-I) cross-linked with bis-NHS-PEG₂, bis-NHS-PEG₄, bis-COOH-PEG₁₂, bis-COOH₂₈, and bis-COOH-PEG₄₅, obtained as described in Examples XV and XVI, was measured in various conditions as described hereinabove in the Materials and Methods Section, and compared with the stability of prh-α-GAL-I cross-linked with bis-NHS-PEG₄₅ as described in Example II. The stability of Replagal^{®} commercial recombinant human α-GAL and non-crosslinked prh-GAL-I was measured for comparison.

As shown in Figure 39, the stability of plant recombinant human α-GAL-I under simulated lysosomal conditions was enhanced by cross-linking with each of the bis-NHS-PEG and bis-COOH-PEG agents.

As further shown therein, the stability of the cross-linked prh-α-GAL-I was correlated with the length of the cross-linking PEG chain, with bis-NHS-PEG₄₅ and bis-COOH-PEG₄₅ providing the most stability, and bis-NHS-PEG₂ providing the least stability. However, cross-linking with bis-COOH-PEG₄₅ provided only marginally more stability than did cross-linking with bis-COOH-PEG₄₅, suggesting that above a certain length, the stability is not affected by PEG chain length.

As further shown in Figure 39, cross-linking with bis-NHS-PEG₄₅ provided slightly more stability than did cross-linking with bis-COOH-PEG₄₅ This may be a result of incomplete activation of the bis-COOH-PEG agent. However the difference in stability was slight.

In addition, cross-linking with each of the bis-NHS-PEG and bis-COOH-PEG agents enhanced the stability of the plant recombinant human α-GAL-I in human plasma at 37°C (data not shown).

These results provide further evidence that cross-linking α-GAL as described herein can increase the efficacy of α-GAL *in vivo* by increasing the stability of α-GAL in lysosomes, and in the blood, and that PEG chains of about 28-45 units in length are more effective at stabilizing α-GAL by cross-linking than are shorter PEG chains.

### EXAMPLE XVIII

### Kinetic parameters of cross-linked plant recombinant human α-GAL-II

The kinetic parameters of cross-linked plant recombinant human α-GAL-II, obtained as described in Example X, as well as of non-cross-linked plant recombinant human α-GAL-II, were determined using a *p*NP-G substrate and Michaelis-Menten analysis; in order to examine the effect of cross-linking thereon. The measurements were performed in a solution of 20mM citrate, 30 mM sodium phosphate, 0.1 % bovine serum albumin and 0.67 % ethanol, at a pH of 4.6. The kinetic parameters were calculated using protein content values based on an activity assay.

As shown in Table 8 below, cross-linking of α-GAL-II resulted in improved kinetic properties, as compared with non-cross-linked α-GAL-II. The Michaelis constant (*K*_{M}) was reduced, indicating higher affinity of the enzyme to the substrate. Furthermore, the *k*_{cat}/*K*_{M}, which signifies the overall catalytic efficiency of the enzyme with this substrate under the described conditions, was enhanced for the cross-linked species.

**Table 8: Michaelis-Menten parameters of non-cross-linked plant recombinant human α-GAL-II (prh-α-GAL-II) and plant recombinant human α-GAL II cross-linked with bis-NHS-PEG₂₁ (prh-α-GAL-II-CL21), bis-NHS-PEG₄₅ (prh-α-GAL-II-CL45) or bis-NHS-PEG₆₈ (prh-α-GAL-II-CL68)**

| Sample | Kₘ (µM) | *V*ₘₐₓ (µM/min) | *k*_{cat} (sec⁻¹) | *k*_{cat} /Kₘ (sec⁻¹ (µM⁻¹) |
|---|---|---|---|---|
| prh-α-GAL-II | 4801 | 4.59 | 73.49 | 0.015 |
| prh-α-GAL-II-CL21 | 2661 | 4.85 | 77.55 | 0.029 |
| prh-α-GAL-CL45 | 2583 | 4.87 | 77.87 | 0.030 |
| prh-α-GAL-II-CL68 | 2556 | 4.12 | 65.97 | 0.026 |

### EXAMPLE XIX

### Reproducibility of cross-linking of plant recombinant human α-GAL-II

The batch-to-batch reproducibility of cross-linking was assessed after preparing 5 batches of plant recombinant human α-GAL-II (prh-α-GAL-II) cross-linked with bis-NHS-PEG₄₅ at a 200:1 ratio, using procedures similar to those described in Example II.

In batches 1, 2, 4 and 5, 1 mg of prh-α-GAL-II was reacted with 3.98 mg bis-NHS-PEG.

In batch 3, 20.5 mg of prh-α-GAL-II was reacted with 80.7 mg bis-NHS-PEG.

The enzymatic activity of the cross-linked prh-α-GAL-II was determined as described in Example III. The results are summarized in Table 9 below.

**Table 9: Activity results of cross-linked plant recombinant human α-GAL-II from different batches**

| Batch no. | Expected α-GAL activity [mg/mL] | Measured α-GAL activity [mg/mL] |
|---|---|---|
| 1 | 1.25 | 1.38 |
| 2 | 1.25 | 1.23 |
| 3 | 1.43 | 1.4 |
| 4 | 1.25 | 0.85 |
| 5 | 1.25 | 1.11 |

As shown in Table 9, the measured activity was close to the expected activity in all 5 batches. In 4 of the 5 batches, the measured activity differed from the expected activity by about 10 % or less.

These results indicate that the obtained activity of the cross-linked prh-α-GAL-II is relatively predictable and reproducible.

The stability of the cross-linked prh-α-GAL-II under lysosomal conditions and in human plasma was determined as described hereinabove.

As shown in Figures 40A and 40B, the stability of the cross-linked prh-α-GAL-II exhibited good reproducibility under both simulated lysosomal conditions and in human plasma.

The cross-linking was also analyzed by SDS-PAGE analysis, IEF (isoelectric focusing) analysis, and MALDI-TOF mass spectrometry, as described hereinabove. Non-cross-linked prh-α-GAL-II was analyzed for comparison.

As shown in Figure 41, the cross-linked prh-α-GAL-II from the different batches exhibited the same degree of covalent dimerization under SDS-PAGE analysis.

As shown in Figure 42, the cross-linked prh-α-GAL-II from the different batches exhibited the same isoelectric points under IEF analysis.

As shown in Figures 43A-43F, the cross-linked prh-α-GAL-II from batches 1-5 (FIGs. 43B-43F, respectively) all exhibited an increase of approximately 20-21 KDa in the dimer form, as compared to the non-cross-linked prh-α-GAL-II (FIG. 43A). Such an increase corresponds to about 10 PEG molecules per α-GAL dimer. As further shown in FIGs. 43B-43F, the cross-linked prh-α-GAL-II from the different batches exhibited similar proportions of monomer vs. dimer.

These results further indicate that good reproducibility in cross-linking of α-GAL.

The kinetic parameters of the cross-linked prh-α-GAL-II were determined using a pNP-G substrate and Michaelis-Menten analysis, in order to examine the reproducibility of enzymatic activity. The measurements were performed in a solution of 20mM citrate, 30 mM sodium phosphate, 0.1 % bovine serum albumin and 0.67 % ethanol, at a pH of 4.6. The kinetic parameters were calculated using protein content values based on optical density at 280 nm.

As shown in Figure 44, the cross-linked prh-α-GAL-II from the different batches exhibited similar profiles of catalytic velocity vs. substrate concentration.

As shown in Table 10 below, the cross-linked prh-α-GAL-II from the different batches exhibited good reproducibility of the *V*ₘₐₓ and *k*_{cat} parameters. The Kₘ parameter varied more between batches, although this may be an artifact of the protein quantification.

The above results indicate good reproducibility in the enzymatic properties of cross-linked α-GAL.

**Table 10: Michaelis-Menten parameters of plant recombinant human α-GAL II cross-linked with bis-NHS-PEG₄₅ in different batches**

| Batch no. | Kₘ (µM) | Vₘₐₓ (µM/min) | *k*_{cat} (sec⁻¹) | **k**_{cat} /Kₘ (sec⁻¹ µM^{- 1}) |
|---|---|---|---|---|
| 1 | 4939 | 3.87 | 61.92 | 0.0125 |
| 2 | 2215 | 3.30 | 52.86 | 0.0239 |
| 3 | 4470 | 3.95 | 63.12 | 0.0141 |
| 4 | 3285 | 3.72 | 59.53 | 0.018 |
| 5 | 2243 | 3.91 | 62.60 | 0.028 |

Although the invention has been described in conjunction with specific embodiments thereof, it is evident that many alternatives, modifications and variations will be apparent to those skilled in the art.

Citation or identification of any reference in this application shall not be construed as an admission that such reference is available as prior art to the present invention. To the extent that section headings are used, they should not be construed as necessarily limiting.

## Claims

1. A multimeric protein structure comprising at least two α-galactosidase monomers being covalently linked to one another via a linking moiety, the multimeric protein structure featuring a characteristic selected from the group consisting of:
(a) an α-galactosidase activity upon subjecting the multimeric protein structure to human plasma conditions for one hour, which is at least 10 % higher than an activity of native α-galactosidase upon subjecting said native α-galactosidase to said human plasma conditions for one hour;
(b) an α-galactosidase activity which decreases upon subjecting the multimeric protein structure to human plasma conditions for one hour by a percentage which is at least 10 % less than the percentage by which an activity of said native α-galactosidase decreases upon subjecting said native α-galactosidase to said human plasma conditions for one hour;
(c) an α-galactosidase activity which remains substantially unchanged upon subjecting the multimeric protein structure to human plasma conditions for one hour;
(d) an α-galactosidase activity, upon subjecting the multimeric protein structure to lysosomal conditions for one week, which is at least 10 % higher than an activity of native α-galactosidase upon subjecting said native α-galactosidase to said lysosomal conditions for one week;
(e) an α-galactosidase activity which decreases upon subjecting the multimeric protein structure to lysosomal conditions for one day by a percentage which is at least 10 % less than the percentage by which an activity of said native α-galactosidase decreases upon subjecting said native α-galactosidase to said lysosomal conditions for one day;
(f) an α-galactosidase activity which remains substantially unchanged upon subjecting the multimeric protein structure to lysosomal conditions for one day;
(g) an α-galactosidase activity, immediately upon subjecting the multimeric protein structure to lysosomal conditions, which is at least 10 % higher than an activity of native α-galactosidase immediately upon subjecting said native form of said protein to said lysosomal conditions;
(h) an α-galactosidase activity, immediately upon subjecting the multimeric protein structure to an aqueous solution having a pH of 7 and a temperature of 37 °C, which is at least 10 % higher than an activity of native α-galactosidase immediately upon subjecting said native α-galactosidase to said aqueous solution having a pH of 7 and a temperature of 37 °C; and
(i) a circulating half-life in a physiological system which is higher by at least 20 % than said circulating half-life of said native α-galactosidase.

2. A multimeric protein structure comprising at least two α-galactosidase monomers being covalently linked to one another via a linking moiety, wherein said linking moiety is not present in native α-galactosidase.

3. The multimeric protein structure of claim 2, featuring a characteristic selected from the group consisting of:
(a) an α-galactosidase activity, upon subjecting the multimeric protein structure to human plasma conditions for one hour, which is at least 10 % higher than an activity of native α-galactosidase upon subjecting said native α-galactosidase to said human plasma conditions for one hour;
(b) an α-galactosidase activity which decreases upon subjecting the multimeric protein structure to human plasma conditions for one hour by a percentage which is at least 10 % less than the percentage by which an activity of said native α-galactosidase decreases upon subjecting said native α-galactosidase to said human plasma conditions for one hour;
(c) an α-galactosidase activity which remains substantially unchanged upon subjecting the multimeric protein structure to human plasma conditions for one hour;
(d) an α-galactosidase activity, upon subjecting the multimeric protein structure to lysosomal conditions for one week, which is at least 10 % higher than an activity of native α-galactosidase upon subjecting said native α-galactosidase to said lysosomal conditions for one week;
(e) an α-galactosidase activity which decreases upon subjecting the multimeric protein structure to lysosomal conditions for one day by a percentage which is at least 10 % less than the percentage by which an activity of said native α-galactosidase decreases upon subjecting said native α-galactosidase to said lysosomal conditions for one day;
(f) an α-galactosidase activity which remains substantially unchanged upon subjecting the multimeric protein structure to lysosomal conditions for one day;
(g) an α-galactosidase activity, immediately upon subjecting the multimeric protein structure to lysosomal conditions, which is at least 10 % higher than an activity of native α-galactosidase immediately upon subjecting said native α-galactosidase to said lysosomal conditions;
(h) an α-galactosidase activity, immediately upon subjecting the multimeric protein structure to an aqueous solution having a pH of 7 and a temperature of 37 °C, which is at least 10 % higher than an activity of native α-galactosidase immediately upon subjecting said native α-galactosidase to said aqueous solution having a pH of 7 and a temperature of 37 °C; and
(i) a circulating half-life in a physiological system which is higher than a circulating half-life of said native α-galactosidase.

4. The multimeric protein structure of claim 3, wherein said circulating half-life of the muftimeric protein structure which is higher than a circulating half-life of said native α-galactosidase, is higher by at least 20 % than said circulating half-life of said native α-galactosidase.

5. The multimeric protein structure of any of claims 1, 3 and 4, wherein said α-galactosidase activity of said multimeric protein structure which remains substantially unchanged upon subjecting the multimeric protein structure to lysosomal conditions for one day, further remains substantially unchanged upon subjecting the multimeric protein structure to lysosomal conditions for one week.

6. The multimeric protein structure of any of claims 1 to 5, **characterized by** an α-galactosidase activity in an organ upon administration of said multimeric protein structure to a vertebrate, said organ being selected from the group consisting of a spleen, a heart and a kidney.

7. The multimeric protein structure of any of claims 1 to 6, comprising two α-galactosidase monomers, the protein structure being a dimeric protein structure.

8. The multimeric protein structure of any of claims 1 to 7, wherein said α-galactosidase is a α-galactosidase generated by recombinant DNA-technology.

9. The multimeric protein structure of any of claims 1 to 7, wherein said α-galactosidase has an amino acids sequence selected from the group consisting of SEQ ID NO:1, SEQ ID NO:2 and SEQ ID NO:3.

10. The multimeric protein structure of of any of claims 1 to 9, wherein said linking moiety is at least 20 atoms long.

11. The multimeric protein structure of any of claims 1 to 9, wherein said linking moiety comprises a poly(alkylene glycol).

12. The multimeric protein structure of any of claims 1 to 9, wherein said linking moiety has a general formula:
-X₁-(CR₁R₂-CR₃R₄-Y)n-X₂-
wherein each of X₁ and X₂ is a functional group that forms a covalent bond with at least one α-galactosidase monomer;
Y is O, S or NR₅;
n is an integer from 1 to 200; and
each of R₁, R₂, R₃, R₄ and R₅ is independently selected from the group consisting of hydrogen, alkyl, cycloalkyl, alkenyl, alkynyl, alkoxy, hydroxy, oxo, thiol and thioalkoxy.

13. The multimeric protein structure of claim 12, wherein n is at least 25.

14. The multimeric protein structure of claim 12, comprising two α-galactosidase monomers, the protein structure being a dimeric protein structure, wherein said α-galactosidase has an amino acids sequence selected from the group consisting of SEQ ID NO:1, SEQ ID NO:2 and SEQ ID NO:3, each of said functional groups forms an amide bond with an α-galactosidase monomer, and n is an integer from 40 to 70.

15. The multimeric protein structure of claim 9, comprising two α-galactosidase monomers, the protein structure being a dimeric protein structure, wherein said linking moiety has the formula: 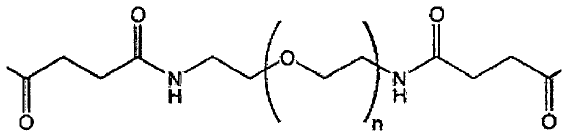 wherein a molecular weight of polyethylene glycol in said linking moiety is 2 kDa, and the terminal groups of said linking moiety each form an amide bond with an α-galactosidase monomer.

16. A process of preparing the multimeric protein structure of any of claims 1 to 15, the process comprising reacting α-galactosidase with a cross-linking agent which comprises said linking moiety and at least two reactive groups.

17. The process of claim 16, wherein a molar ratio of said cross-linking agent to monomers of said α-galactosidase is in a range of from 5:1 to 500:1.

## Patentansprüche

1. Multimere Proteinstruktur, die mindestens zwei α-Galaktosidase-Monomere umfasst, die kovalent über eine Verknüpfungseinheit miteinander verknüpft sind, wobei die multimere Proteinstruktur eine Eigenschaft aufweist, die ausgewählt ist aus der Gruppe bestehend aus:
(a) einer α-Galaktosidase-Aktivität, nach dem Aussetzen der multimeren Proteinstruktur gegenüber humanen Plasmabedingungen für eine Stunde, die mindestens 10% höher ist als eine Aktivität nativer α-Galaktosidase nach dem Aussetzen der nativen α-Galaktosidase gegenüber den humanen Plasmabedingungen für eine Stunde;
(b) einer α-Galaktosidase-Aktivität, die auf das Aussetzen der multimeren Proteinstruktur gegenüber humanen Plasmabedingungen für eine Stunde hin um einen Prozentsatz abnimmt, der mindestens 10% geringer ist als der Prozentsatz, um den eine Aktivität der nativen α-Galaktosidase, auf das Aussetzen der nativen α-Galaktosidase gegenüber den humanen Plasmabedingungen für eine Stunde hin, abnimmt;
(c) einer α-Galaktosidase-Aktivität, die nach dem Aussetzen der multimeren Proteinstruktur gegenüber humanen Plasmabedingungen für eine Stunde im Wesentlichen unverändert bleibt;
(d) einer α-Galaktosidase-Aktivität, nach dem Aussetzen der multimeren Proteinstruktur gegenüber lysosomalen Bedingungen für eine Woche, die mindestens 10% höher ist als eine Aktivität nativer α-Galaktosidase nach dem Aussetzen der nativen α-Galaktosidase gegenüber den lysosomalen Bedingungen für eine Woche;
(e) einer α-Galaktosidase-Aktivität, die auf das Aussetzen der multimeren Proteinstruktur gegenüber lysosomalen Bedingungen für einen Tag hin um einen Prozentsatz abnimmt, der mindestens 10% geringer ist als der Prozentsatz, um den eine Aktivität der nativen α-Galaktosidase, auf das Aussetzen der nativen α-Galaktosidase gegenüber den lysosomalen Bedingungen für einen Tag hin, abnimmt;
(f) einer α-Galaktosidase-Aktivität, die nach dem Aussetzen der multimeren Proteinstruktur gegenüber den lysosomalen Bedingungen für einen Tag im Wesentlichen unverändert bleibt;
(g) einer α-Galaktosidase-Aktivität, unmittelbar nach dem Aussetzen der multimeren Proteinstruktur gegenüber lysosomalen Bedingungen, die mindestens 10% höher ist als eine Aktivität nativer α-Galaktosidase unmittelbar nach dem Aussetzen der nativen Form des Proteins gegenüber den lysosomalen Bedingungen;
(h) einer α-Galaktosidase-Aktivität, unmittelbar nach dem Aussetzen der multimeren Proteinstruktur gegenüber einer wässrigen Lösung mit einem pH-Wert von 7 und einer Temperatur von 37°C, die mindestens 10% höher ist als eine Aktivität nativer α-Galaktosidase unmittelbar nach dem Aussetzen der nativen α-Galaktosidase gegenüber der wässrigen Lösung mit einem pH-Wert von 7 und einer Temperatur von 37°C; und
(i) einer Kreislauf-Halbwertszeit in einem physiologischen System, die mindestens 20% größer ist als die Kreislauf-Halbwertszeit der nativen α-Galaktosidase.

2. Multimere Proteinstruktur, die mindestens zwei α-Galaktosidase-Monomere umfasst, die kovalent über eine Verknüpfungseinheit miteinander verknüpft sind, wobei die Verknüpfungseinheit in nativer α-Galaktosidase nicht vorkommt.

3. Multimere Proteinstruktur nach Anspruch 2, die eine Eigenschaft aufweist, die ausgewählt ist aus der Gruppe bestehend aus:
(a) einer α-Galaktosidase-Aktivität, nach dem Aussetzen der multimeren Proteinstruktur gegenüber humanen Plasmabedingungen für eine Stunde, die mindestens 10% höher ist als eine Aktivität nativer α-Galaktosidase nach dem Aussetzen der nativen α-Galaktosidase gegenüber den humanen Plasmabedingungen für eine Stunde;
(b) einer α-Galaktosidase-Aktivität, die auf das Aussetzen der multimeren Proteinstruktur gegenüber humanen Plasmabedingungen für eine Stunde hin um einen Prozentsatz abnimmt, der mindestens 10% geringer ist als der Prozentsatz, um den eine Aktivität der nativen α-Galaktosidase, auf das Aussetzen der nativen α-Galaktosidase gegenüber den humanen Plasmabedingungen für eine Stunde hin, abnimmt;
(c) einer α-Galaktosidase-Aktivität, die nach dem Aussetzen der multimeren Proteinstruktur gegenüber humanen Plasmabedingungen für eine Stunde im Wesentlichen unverändert bleibt;
(d) einer α-Galaktosidase-Aktivität, nach dem Aussetzen der multimeren Proteinstruktur gegenüber lysosomalen Bedingungen für eine Woche, die mindestens 10% höher ist als eine Aktivität nativer α-Galaktosidase nach dem Aussetzen der nativen α-Galaktosidase gegenüber den lysosomalen Bedingungen für eine Woche;
(e) einer α-Galaktosidase-Aktivität, die auf das Aussetzen der multimeren Proteinstruktur gegenüber lysosomalen Bedingungen für einen Tag hin um einen Prozentsatz abnimmt, der mindestens 10% geringer ist als der Prozentsatz, um den eine Aktivität der nativen α-Galaktosidase, auf das Aussetzen der nativen α-Galaktosidase gegenüber den lysosomalen Bedingungen für einen Tag hin, abnimmt;
(f) einer α-Galaktosidase-Aktivität, die nach dem Aussetzen der multimeren Proteinstruktur gegenüber den lysosomalen Bedingungen für einen Tag im Wesentlichen unverändert bleibt;
(g) einer α-Galaktosidase-Aktivität, unmittelbar nach dem Aussetzen der multimeren Proteinstruktur gegenüber lysosomalen Bedingungen, die mindestens 10% höher ist als eine Aktivität nativer α-Galaktosidase unmittelbar nach dem Aussetzen der nativen α-Galaktosidase gegenüber den lysosomalen Bedingungen;
(h) einer α-Galaktosidase-Aktivität, unmittelbar nach dem Aussetzen der multimeren Proteinstruktur gegenüber einer wässrigen Lösung mit einem pH-Wert von 7 und einer Temperatur von 37°C, die mindestens 10% höher ist als eine Aktivität nativer α-Galaktosidase unmittelbar nach dem Aussetzen der nativen α-Galaktosidase gegenüber der wässrigen Lösung mit einem pH-Wert von 7 und einer Temperatur von 37°C; und
(i) einer Kreislauf-Halbwertszeit in einem physiologischen System, die größer ist als eine Kreislauf-Halbwertszeit der nativen α-Galaktosidase.

4. Multimere Proteinstruktur nach Anspruch 3, wobei die Kreislauf-Halbwertszeit der multimeren Proteinstruktur, die größer ist als eine Kreislauf-Halbwertszeit der nativen α-Galaktosidase, um mindestens 20% größer ist als die Kreislauf-Halbwertszeit der nativen α-Galaktosidase.

5. Multimere Proteinstruktur nach einem der Ansprüche 1, 3 und 4, wobei die α-Galaktosidase-Aktivität der multimeren Proteinstruktur, die nach dem Aussetzen der multimeren Proteinstruktur gegenüber lysosomalen Bedingungen für einen Tag im Wesentlichen unverändert bleibt, weiterhin nach dem Aussetzen der multimeren Proteinstruktur gegenüber den lysosomalen Bedingungen für eine Woche im Wesentlichen unverändert bleibt.

6. Multimere Proteinstruktur nach einem der Ansprüche 1 bis 5, **gekennzeichnet durch** eine α-Galaktosidase-Aktivität in einem Organ, nachdem die multimere Proteinstruktur an einen Vertebraten verabreicht wurde, wobei das Organ eine Milz, ein Herz oder eine Niere ist.

7. Multimere Proteinstruktur nach einem der Ansprüche 1 bis 6, die zwei α-Galaktosidase-Monomere umfasst, wobei die Proteinstruktur eine dimere Proteinstruktur ist.

8. Multimere Proteinstruktur nach einem der Ansprüche 1 bis 7, wobei die α-Galaktosidase eine α-Galaktosidase ist, die durch rekombinante DNA-Technology hergestellt ist.

9. Multimere Proteinstruktur nach einem der Ansprüche 1 bis 7, wobei die α-Galaktosidase eine Aminosäuresequenz, ausgewählt aus der Gruppe bestehend aus SEQ ID Nr:1, SEQ ID Nr:2 und SEQ ID Nr:3, hat.

10. Multimere Proteinstruktur nach einem der Ansprüche 1 bis 9, wobei die Verknüpfungseinheit mindestens 20 Atome lang ist.

11. Multimere Proteinstruktur nach einem der Ansprüche 1 bis 9, wobei die Verknüpfungseinheit ein Poly(Alkylen-Glycol) umfasst.

12. Multimere Proteinstruktur nach einem der Ansprüche 1 bis 9, wobei die Verknüpfungseinheit eine allgemeine Formel hat:
-X₁-(CR₁-R₂-CR₃R₄-Y)n-X₂-
wobei jeder von X₁ und X₂ ein funktioneller Rest ist, der eine kovalente Bindung mit mindestens einem α-Galaktosidase-Monomer bildet;
Y O, S oder NR₅ ist;
n eine ganze Zahl von 1 bis 200 ist; und
jeder von R₁, R₂, R₃, R₄ und R₅ unabhängig ausgewählt ist aus der Gruppe bestehend aus Wasserstoff, Alkyl, Cycloalkyl, Alkenyl, Alkynyl, Alkoxy, Hydroxy, Oxo, Thiol und Thioalkoxy.

13. Multimere Proteinstruktur nach Anspruch 12, wobei n mindestens 25 ist.

14. Multimere Proteinstruktur nach Anspruch 12, die zwei α-Galaktosidase-Monomere umfasst, wobei die Proteinstruktur eine dimere Proteinstruktur ist, die α-Galaktosidase eine Aminosäuresequenz ausgewählt aus der Gruppe bestehend aus SEQ ID Nr:1, SEQ ID Nr:2 und SEQ ID Nr:3 hat, jede funktionelle Gruppe eine Amidbindung mit einem α-Galaktosidase-Monomer bildet und n eine ganze Zahl zwischen 40 und 70 ist.

15. Multimere Proteinstruktur nach Anspruch 9, die zwei α-Galaktosidase-Monomere umfasst, wobei die Proteinstruktur eine dimere Proteinstruktur ist, wobei die Verknüpfungseinheit die Formel hat: 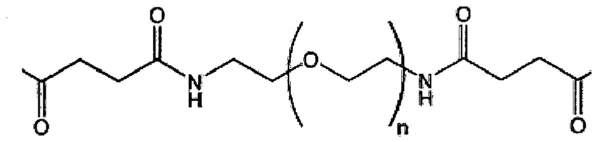 wobei ein Molekulargewicht des Polyethylenglycols in der Verknüpfungseinheit 2kDa ist und die terminalen Gruppen der Verknüpfungseinheit jeweils eine Amidbindung mit einem α-Galaktosidase-Monomer bilden.

16. Verfahren zur Herstellung einer multimeren Proteinstruktur nach einem der Ansprüche 1 bis 15, wobei das Verfahren die Umsetzung von α-Galaktosidase mit einem Verknüpfungsmittel umfasst, das die Verknüpfungseinheit und mindestens zwei reaktive Reste umfasst.

17. Verfahren nach Anspruch 16, wobei das molare Verhältnis des Verknüpfungsmittels zu Monomeren der α-Galaktosidase in einem Bereich von 5:1 bis 500:1 liegt.

## Revendications

1. Structure de protéine multimère comprenant au moins deux monomères d'α-galactosidase liés de façon covalente l'un à l'autre par l'intermédiaire d'un radical de liaison, la structure de protéine multimère présentant une caractéristique choisie dans le groupe constitué de :
(a) une activité α-galactosidase après soumission de la structure de protéine multimère à des conditions plasmatiques humaines pendant une heure, qui est au moins 10 % supérieure à une activité d'α-galactosidase native après soumission de ladite α-galactosidase native auxdites conditions plasmatiques humaines pendant une heure ;
(b) une activité α-galactosidase qui diminue après soumission de la structure de protéine multimère à des conditions plasmatiques humaines pendant une heure d'un pourcentage qui est au moins de 10 % inférieur au pourcentage duquel une activité de ladite α-galactosidase native diminue après soumission de ladite α-galactosidase native auxdites conditions plasmatiques humaines pendant une heure ;
(c) une activité α-galactosidase qui demeure substantiellement inchangée après soumission de la structure de protéine multimère à des conditions plasmatiques humaines pendant une heure ;
(d) une activité α-galactosidase, après soumission de la structure de protéine multimère à des conditions lysosomiales pendant une semaine, qui est au moins de 10 % supérieure à une activité d'α-galactosidase native après soumission de ladite α-galactosidase native auxdites conditions lysosomiales pendant une semaine ;
(e) une activité α-galactosidase qui diminue après soumission de la structure de protéine multimère à des conditions lysosomiales pendant un jour d'un pourcentage qui est au moins de 10 % inférieur au pourcentage duquel une activité de ladite α-galactosidase native diminue après soumission de ladite α-galactosidase native auxdites conditions lysosomiales pendant un jour ;
(f) une activité α-galactosidase qui demeure substantiellement inchangée après soumission de la structure de protéine multimère à des conditions lysosomiales pendant un jour ;
(g) une activité α-galactosidase, immédiatement après soumission de la structure de protéine multimère à des conditions lysosomiales, qui est au moins de 10 % supérieure à une activité d'α-galactosidase native immédiatement après soumission de ladite forme native de ladite protéine auxdites conditions lysosomiales ;
(h) une activité α-galactosidase, immédiatement après soumission de la structure de protéine multimère à une solution aqueuse ayant un pH de 7 et une température de 37 °C, qui est au moins de 10 % supérieure à une activité d'α-galactosidase native immédiatement après soumission de ladite α-galactosidase native à ladite solution aqueuse ayant un pH de 7 et une température de 37 °C ; et
(i) une demi-vie circulante dans un système physiologique qui est supérieure d'au moins 20 % à ladite demi-vie circulante de ladite α-galactosidase native.

2. Structure de protéine multimère comprenant au moins deux monomères d'α-galactosidase liés de façon covalente l'un à l'autre par l'intermédiaire d'un radical de liaison, où ledit radical de liaison n'est pas présent dans l'α-galactosidase native.

3. Structure de protéine multimère selon la revendication 2, présentant une caractéristique choisie dans le groupe constitué de :
(a) une activité α-galactosidase, après soumission de la structure de protéine multimère à des conditions plasmatiques humaines pendant une heure, qui est au moins de 10 % supérieure à une activité d'α-galactosidase native après soumission de ladite α-galactosidase native auxdites conditions plasmatiques humaines pendant une heure ;
(b) une activité α-galactosidase qui diminue après soumission de la structure de protéine multimère à des conditions plasmatiques humaines pendant une heure d'un pourcentage qui est au moins de 10 % inférieur au pourcentage duquel une activité de ladite α-galactosidase native diminue après soumission de ladite α-galactosidase native auxdites conditions plasmatiques humaines pendant une heure ;
(c) une activité α-galactosidase qui demeure substantiellement inchangée après soumission de la structure de protéine multimère à des conditions plasmatiques humaines pendant une heure ;
(d) une activité α-galactosidase, après soumission de la structure de protéine multimère à des conditions lysosomiales pendant une semaine, qui est au moins de 10 % supérieure à une activité d'α-galactosidase native après soumission de ladite α-galactosidase native auxdites conditions lysosomiales pendant une semaine ;
(e) une activité α-galactosidase qui diminue après soumission de la structure de protéine multimère à des conditions lysosomiales pendant un jour d'un pourcentage qui est au moins de 10 % inférieur au pourcentage duquel une activité de ladite α-galactosidase native diminue après soumission de ladite α-galactosidase native auxdites conditions lysosomiales pendant un jour ;
(f) une activité α-galactosidase qui demeure substantiellement inchangée après soumission de la structure de protéine multimère à des conditions lysosomiales pendant un jour ;
(g) une activité α-galactosidase, immédiatement après soumission de la structure de protéine multimère à des conditions lysosomiales, qui est au moins de 10 % supérieure à une activité d'α-galactosidase native immédiatement après soumission de ladite α-galactosidase native auxdites conditions lysosomiales ;
(h) une activité α-galactosidase, immédiatement après soumission de la structure de protéine multimère à une solution aqueuse ayant un pH de 7 et une température de 37 °C, qui est au moins de 10 % supérieure à une activité d'α-galactosidase native immédiatement après soumission de ladite α-galactosidase native à ladite solution aqueuse ayant un pH de 7 et une température de 37 °C ; et
(i) une demi-vie circulante dans un système physiologique qui est supérieure à une demi-vie circulante de ladite α-galactosidase native.

4. Structure de protéine multimère selon la revendication 3, où ladite demi-vie circulante de la structure de protéine multimère qui est supérieure à une demi-vie circulante de ladite α-galactosidase native, est supérieure d'au moins 20 % à ladite demi-vie circulante de ladite α-galactosidase native.

5. Structure de protéine multimère selon l'une quelconque des revendications 1, 3 et 4, où ladite activité α-galactosidase de ladite structure de protéine multimère qui demeure substantiellement inchangée après soumission de la structure de protéine multimère à des conditions lysosomiales pendant un jour, demeure en outre substantiellement inchangée après soumission de la structure de protéine multimère à des conditions lysosomiales pendant une semaine.

6. Structure de protéine multimère selon l'une quelconque des revendications 1 à 5, **caractérisée par** une activité α-galactosidase dans un organe après administration de ladite structure de protéine multimère à un vertébré, ledit organe étant choisi dans le groupe constitué d'une rate, d'un coeur et d'un rein.

7. Structure de protéine multimère selon l'une quelconque des revendications 1 à 6, comprenant deux monomères d'α-galactosidase, la structure de protéine étant une structure de protéine dimère.

8. Structure de protéine multimère selon l'une quelconque des revendications 1 à 7, où ladite α-galactosidase est une α-galactosidase générée par la technologie de l'ADN recombinant.

9. Structure de protéine multimère selon l'une quelconque des revendications 1 à 7, où ladite α-galactosidase a une séquence d'acides aminés choisie dans le groupe constitué de SEQ ID NO : 1, SEQ ID NO : 2 et SEQ ID NO : 3.

10. Structure de protéine multimère selon l'une quelconque des revendications 1 à 9, où ledit radical de liaison à une longueur d'au moins 20 atomes.

11. Structure de protéine multimère selon l'une quelconque des revendications 1 à 9, où ledit radical de liaison comprend un polyalkylène glycol.

12. Structure de protéine multimère selon l'une quelconque des revendications 1 à 9, où ledit radical de liaison a une formule générale :
-X₁-(CR₁R₂-CR₃R₄-Y)n-X2-
dans laquelle chacun de X₁ et X₂ représente un groupe fonctionnel qui forme une liaison covalente avec au moins un monomère d'α-galactosidase ;
Y représente O, S ou NR₅ ;
n est un nombre entier de 1 à 200 ; et
chacun de R₁, R₂, R₃, R₄ et R₅ est choisi indépendamment dans le groupe constitué d'un atome d'hydrogène, des groupes alkyle, cycloalkyle, alcényle, alcynyle, alcoxy, hydroxy, oxo, thiol et thioalcoxy.

13. Structure de protéine multimère selon la revendication 12, où n vaut au moins 25.

14. Structure de protéine multimère selon la revendication 12, comprenant deux monomères d'α-galactosidase, la structure de protéine étant une structure de protéine dimère, où ladite α-galactosidase a une séquence d'acides aminés choisie dans le groupe constitué de SEQ ID NO : 1, SEQ ID NO : 2 et SEQ ID NO : 3, chacun desdits groupes fonctionnels forme une liaison amide avec un monomère d'α-galactosidase, et n est un nombre entier de 40 à 70.

15. Structure de protéine multimère selon la revendication 9, comprenant deux monomères d'α-galactosidase, la structure de protéine étant une structure de protéine dimère, où ledit radical de liaison a la formule : 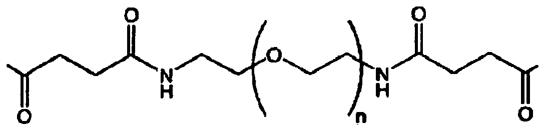 où une masse moléculaire de polyéthylène glycol dans ledit radical de liaison est de 2 kDa, et les groupes terminaux dudit radical de liaison forment chacun une liaison amide avec un monomère d'α-galactosidase.

16. Procédé de préparation de la structure de protéine multimère selon l'une quelconque des revendications 1 à 15, le procédé comprenant la réaction d'α-galactosidase avec un agent de réticulation qui comprend ledit radical de liaison et au moins deux groupes réactifs.

17. Procédé selon la revendication 16, dans lequel un rapport molaire dudit agent de réticulation sur les monomères de ladite α-galactosidase se situe dans une plage de 5/1 à 500/1.
